(19) Europäisches Patentamt
European Patent Office
Office européen des brevets

(11) EP 1 357 129 A2

(12) EUROPEAN PATENT APPLICATION
published in accordance with Art. 158(3) EPC

(43) Date of publication:
**29.10.2003 Bulletin 2003/44**

(21) Application number: **02711281.2**

(22) Date of filing: **01.02.2002**

(51) Int Cl.7: **C07K 14/47**, C07K 14/705, C12N 15/12, C12P 21/02, C07K 16/18, A01K 67/027, C12N 5/10, G01N 33/15, G01N 33/50, A61P 1/00

(86) International application number:
**PCT/JP02/00852**

(87) International publication number:
**WO 02/062944 (15.08.2002 Gazette 2002/33)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **02.02.2001 JP 2001026820**

(71) Applicant: **Takeda Chemical Industries, Ltd.**
**Osaka-shi, Osaka 541-0045 (JP)**

(72) Inventors:
- **OHTAKI, Tetsuya**
  **Tsukuba-shi, Ibaraki 305-0821 (JP)**
- **MASUDA, Yasushi, Tsukuba Sakura Danchi**
  **Tsukuba-shi, Ibaraki 305-0032 (JP)**
- **TAKATSU, Yoshihiro,**
  **Tsukuba Sakura Danchi 905-505**
  **Tsukuba-shi, Ibaraki 305-0032 (JP)**
- **WATANABE, Takuya**
  **Osaka-shi, Osaka 532-0033 (JP)**
- **TERAO, Yasuko, St. Hill**
  **Kobe-shi, Hyogo 658-0063 (JP)**
- **SHINTANI, Yasushi**
  **Osaka-shi, Osaka 532-0033 (JP)**
- **HINUMA, Syuji**
  **Tsukuba-shi, Ibaraki 305-0821 (JP)**

(74) Representative: **Rickard, Timothy Mark Adrian**
**Takeda Euro IP Department,**
**11-12 Charles II Street**
**London SW1Y 4QU (GB)**

# (54) NOVEL PHYSIOLOGICALLY ACTIVE PEPTIDE AND USE THEREOF

(57) The present invention is intended to provide therapeutic/prophylactic agents and diagnostic for digestive diseases, etc.

Specifically, the present invention provides a screening method/screening kit for a compound or its salt capable of promoting or inhibiting the activity of a novel peptide, etc., compounds or salts thereof obtained by the screening, pharmaceuticals comprising the compounds or salts thereof, etc. The peptide of the invention can be used for the diagnosis, treatment, prevention, etc. of digestive diseases, etc. and is useful as a reagent for screening a compound or its salt capable of promoting or inhibiting the activity of the protein of the invention.

EP 1 357 129 A2

SEQUENCE LISTING

<110> Takeda Chemical Industries, Ltd.

<120> Novel Phisiological Active Peptide and Its Use

<130> P01-0295PCT

<150> JP2001-026820
<151> 2001-02-02

<160> 71

<210> 1
<211> 393
<212> PRT
<213> Human

<400> 1

```
Met Glu Thr Thr Met Gly Phe Met Asp Asp Asn Ala Thr Asn Thr Ser
                5                   10                  15
Thr Ser Phe Leu Ser Val Leu Asn Pro His Gly Ala His Ala Thr Ser
            20                  25                  30
Phe Pro Phe Asn Phe Ser Tyr Ser Asp Tyr Asp Met Pro Leu Asp Glu
        35                  40                  45
Asp Glu Asp Val Thr Asn Ser Arg Thr Phe Phe Ala Ala Lys Ile Val
    50                  55                  60
Ile Gly Met Ala Leu Val Gly Ile Met Leu Val Cys Gly Ile Gly Asn
65                  70                  75                  80
```

Phe Ile Phe Ile Ala Ala Leu Val Arg Tyr Lys Lys Leu Arg Asn Leu
85 90 95
Thr Asn Leu Leu Ile Ala Asn Leu Ala Ile Ser Asp Phe Leu Val Ala
100 105 110
Ile Val Cys Cys Pro Phe Glu Met Asp Tyr Tyr Val Val Arg Gln Leu
115 120 125
Ser Trp Glu His Gly His Val Leu Cys Thr Ser Val Asn Tyr Leu Arg
130 135 140
Thr Val Ser Leu Tyr Val Ser Thr Asn Ala Leu Leu Ala Ile Ala Ile
145 150 155 160
Asp Arg Tyr Leu Ala Ile Val His Pro Leu Arg Pro Arg Met Lys Cys
165 170 175
Gln Thr Ala Thr Gly Leu Ile Ala Leu Val Trp Thr Val Ser Ile Leu
180 185 190
Ile Ala Ile Pro Ser Ala Tyr Phe Thr Thr Glu Thr Val Leu Val Ile
195 200 205
Val Lys Ser Gln Glu Lys Ile Phe Cys Gly Gln Ile Trp Pro Val Asp
210 215 220
Gln Gln Leu Tyr Tyr Lys Ser Tyr Phe Leu Phe Ile Phe Gly Ile Glu
225 230 235 240
Phe Val Gly Pro Val Val Thr Met Thr Leu Cys Tyr Ala Arg Ile Ser
245 250 255
Arg Glu Leu Trp Phe Lys Ala Val Pro Gly Phe Gln Thr Glu Gln Ile
260 265 270
Arg Lys Arg Leu Arg Cys Arg Arg Lys Thr Val Leu Val Leu Met Cys
275 280 285
Ile Leu Thr Ala Tyr Val Leu Cys Trp Ala Pro Phe Tyr Gly Phe Thr
290 295 300
Ile Val Arg Asp Phe Phe Pro Thr Val Phe Val Lys Glu Lys His Tyr

305 310 315 320

Leu Thr Ala Phe Tyr Ile Val Glu Cys Ile Ala Met Ser Asn Ser Met

325 330 335

Ile Asn Thr Leu Cys Phe Val Thr Val Lys Asn Asp Thr Val Lys Tyr

340 345 350

Phe Lys Lys Ile Met Leu Leu His Trp Lys Ala Ser Tyr Asn Gly Gly

355 360 365

Lys Ser Ser Ala Asp Leu Asp Leu Lys Thr Ile Gly Met Pro Ala Thr

370 375 380

Glu Glu Val Asp Cys Ile Arg Leu Lys

385 390

<210> 2

<211> 1179

<212> DNA

<213> Human

<400> 2

atggagacca ccatggggtt catggatgac aatgccacca acacttccac cagcttcctt 60

tctgtgctca accctcatgg agcccatgcc acttccttcc cattcaactt cagctacagc 120

gactatgata tgcctttgga tgaagatgag gatgtgacca attccaggac gttctttgct 180

gccaagattg tcattgggat ggccctggtg ggcatcatgc tggtctgcgg cattggaaac 240

ttcatcttta tcgctgccct ggtccgctac aagaaactgc gcaacctcac caacctgctc 300

atcgccaacc tggccatctc tgacttcctg gtggccattg tctgctgccc ctttgagatg 360

gactactatg tggtgcgcca gctctcctgg gagcacggcc acgtcctgtg cacctctgtc 420

aactacctgc gcactgtctc tctctatgtc tccaccaatg ccctgctggc catcgccatt 480

gacaggtatc tggctattgt ccatccgctg agaccacgga tgaagtgcca aacagccact 540

ggcctgattg ccttggtgtg gacggtgtcc atcctgatcg ccatcccttc cgcctacttc 600

accaccgaga cggtcctcgt cattgtcaag agccaggaaa agatcttctg cggccagatc 660

tggcctgtgg accagcagct ctactacaag tcctacttcc tctttatctt tggcatagaa 720
ttcgtgggcc ccgtggtcac catgaccctg tgctatgcca ggatctcccg ggagctctgg 780
ttcaaggcgg tccctggatt ccagacagag cagatccgca agaggctgcg ctgccgcagg 840
aagacggtcc tggtgctcat gtgcatcctc accgcctacg tgctatgctg ggcgcccttc 900
tacggcttca ccatcgtgcg cgacttcttc cccaccgtgt tcgtgaagga gaagcactac 960
ctcactgcct tctacatcgt cgagtgcatc gccatgagca acagcatgat caacactctg 1020
tgcttcgtga ccgtcaagaa cgacaccgtc aagtacttca aaaagatcat gttgctccac 1080
tggaaggctt cttacaatgg cggtaagtcc agtgcagacc tggacctcaa gacaattggg 1140
atgcctgcca ccgaagaggt ggactgcatc agactaaaa 1179

<210> 3
<211> 1179
<212> DNA
<213> Human

<400> 3
atggagacca ccatggggtt catggatgac aatgccacca acacttccac cagcttcctt 60
tctgtgctca accctcatgg agcccatgcc acttccttcc cattcaactt cagctacagc 120
gactatgata tgcctttgga tgaagatgag gatgtgacca attccaggac gttctttgct 180
gccaagattg tcattgggat ggccctggtg ggcatcatgc tggtctgcgg cattggaaac 240
ttcatcttta tcgctgccct ggtccgctac aagaaactgc gcaacctcac caacctgctc 300
atcgccaacc tggccatctc tgacttcctg gtggccattg tctgctgccc ctttgagatg 360
gactactatg tggtgcgcca gctctcctgg gagcacggcc acgtcctgtg cacctctgtc 420
aactacctgc gcactgtctc tctctatgtc tccaccaatg ccctgctggc catcgccatt 480
gacaggtatc tggctattgt ccatccgctg agaccacgga tgaagtgcca aacagccact 540
ggcctgattg ccttggtgtg gacggtgtcc atcctgatcg ccatcccttc cgcctacttc 600
accaccgaga cggtcctcgt cattgtcaag agccaggaaa agatcttctg cggccagatc 660
tggcctgtgg accagcagct ctactacaag tcctacttcc tctttatctt tggcatagaa 720
ttcgtgggcc ccgtggtcac catgaccctg tgctatgcca ggatctcccg ggagctctgg 780

ttcaaggcgg tccctggatt ccagacagag cagatccgca agaggctgcg ctgccgcagg 840
aagacggtcc tggtgctcat gtgcatcctc accgcctacg tgctatgctg ggcgcccttc 900
tacggcttca ccatcgtgcg cgacttcttc cccaccgtgt ttgtgaagga gaagcactac 960
ctcactgcct tctacatcgt cgagtgcatc gccatgagca acagcatgat caacactctg 1020
tgcttcgtga ccgtcaagaa cgacaccgtc aagtacttca aaaagatcat gttgctccac 1080
tggaaggctt cttacaatgg cggtaagtcc agtgcagacc tggacctcaa gacaattggg 1140
atgcctgcca ccgaagaggt ggactgcatc agactaaaa 1179

<210> 4
<211> 31
<212> DNA
<213> Artificial Sequence

<220>
<223>DNA primer, primer 1

<400> 4
gtcgacatgg agaccaccat ggggttcatg g 31

<210> 5
<211> 36
<212> DNA
<213> Artificial Sequence

<220>
<223> DNA primer, primer 2

<400> 5
actagtttat tttagtctga tgcagtccac ctcttc 36

<210> 6
<211> 1152
<212> DNA
<213> Human

<400> 6
atggcagccc agaatggaaa caccagtttc acacccaact ttaatccacc ccaagaccat 60
gcctcctccc tctcctttaa cttcagttat ggtgattatg acctccctat ggatgaggat 120
gaggacatga ccaagacccg gaccttcttc gcagccaaga tcgtcattgg cattgcactg 180
gcaggcatca tgctggtctg cggcatcggt aactttgtct ttatcgctgc cctcacccgc 240
tataagaagt tgcgcaacct caccaatctg ctcattgcca acctggccat ctccgacttc 300
ctggtggcca tcatctgctg ccccttcgag atggactact acgtggtacg gcagctctcc 360
tgggagcatg gccacgtgct ctgtgcctcc gtcaactacc tgcgcaccgt ctccctctac 420
gtctccacca atgccttgct ggccattgcc attgacagat atctcgccat cgttcacccc 480
ttgaaaccac ggatgaatta tcaaacggcc tccttcctga tcgccttggt ctggatggtg 540
tccattctca ttgccatccc atcggcttac tttgcaacag aaaccgtcct ctttattgtc 600
aagagccagg agaagatctt ctgtggccag atctggcctg tggatcagca gctctactac 660
aagtcctact tcctcttcat ctttggtgtc gagttcgtgg gccctgtggt caccatgacc 720
ctgtgctatg ccaggatctc ccgggagctc tggttcaagg cagtccctgg gttccagacg 780
gagcagattc gcaagcggct gcgctgccgc aggaagacgg tcctggtgct catgtgcatt 840
ctcacggcct atgtgctgtg ctgggcaccc ttctacggtt tcaccatcgt tcgtgacttc 900
ttccccactg tgttcgtgaa ggaaaagcac tacctcactg ccttctacgt ggtcgagtgc 960
atcgccatga gcaacagcat gatcaacacc gtgtgcttcg tgacggtcaa gaacaacacc 1020
atgaagtact tcaagaagat gatgctgctg cactggcgtc cctcccagcg ggggagcaag 1080
tccagtgctg accttgacct cagaaccaac ggggtgccca ccacagaaga agtggactgt 1140
atcaggctga ag 1152

<210> 7

<211> 26
<212> DNA
<213> Artificial Sequence

<220>
<223> DNA primer, hBv8-F1 primer

<400> 7
ctacttctgc tgctgccgct gctgtt 26

<210> 8
<211> 28
<212> DNA
<213> Artificial Sequence

<220>
<223> DNA primer, hBv8-R1 primer

<400> 8
ttggaaagtt gaggaagcaa gagcattt 28

<210> 9
<211> 359
<212> DNA
<213> Human

<400> 9
cacgccccgc gctggggacg ccgccgtgat caccggggct tgtgacaagg actcccaatg 60
tggtggaggc atgtgctgtg ctgtcagtat ctgggtcaag agcataagga tttgcacacc 120

tatgggcaaa ctgggagaca gctgccatcc actgactcgt aaagttccat tttttgggcg 180
gaggatgcat cacacttgcc catgtctgcc aggcttggcc tgtttacgga cttcatttaa 240
ccgatttatt tgtttagccc aaaagtaatc gctctggagt agaaaccaaa tgtgaatagc 300
cacatcttac ctgtaaagtc ttacttgtga ttgtgccaaa caaaaaatgt gccagaaag 359

<210> 10
<211> 27
<212> DNA
<213> Artificial Sequence

<220>
<223> DNA primer, hBv8-R2 primer

<400> 10
tgtctcccag tttgcccata ggtgtgc 27

<210> 11
<211> 184
<212> DNA
<213> Human

<400> 11
cccgagggcg ccatgaggag cctgtgctgc gccccactcc tgctcctctt gctgctgccg 60
ccgctgctgc tcacgccccg cgctggggac gccgccgtga tcaccggggc ttgtgacaag 120
gactcccaat gtggtggagg catgtgctgt gctgtcagta tctgggtcaa gagcataagg 180
attt 184

<210> 12
<211> 23

<212> DNA

<213> Artificial Sequence

<220>

<223> DNA primer, hBv8-WF primer

<400> 12

ccatgaggag cctgtgctgc gcc 23

<210> 13

<211> 23

<212> DNA

<213> Artificial Sequence

<220>

<223> DNA primer, hBv8-WR primer

<400> 13

ctattcacat ttggtttcta ctc 23

<210> 14

<211> 29

<212> DNA

<213> Artificial Sequence

<220>

<223> DNA primer, hBv8-CF primer

<400> 14

gtcgaccacc atgaggagcc tgtgctgcg 29

<210> 15
<211> 26
<212> DNA
<213> Artificial Sequence

<220>
<223> DNA primer, hBv8-SR primer

<400> 15
actagtcgat tacttttggg ctaaac 26

<210> 16
<211> 346
<212> DNA
<213> Human

<400> 16
gtcgaccacc atgaggagcc tgtgctgcgc cccactcctg ctcctcttgc tgctgccgcc 60
gctgctgctc acgccccgcg ctggggacgc cgccgtgatc accggggctt gtgacaagga 120
ctcccaatgt ggtggaggca tgtgctgtgc tgtcagtatc tgggtcaaga gcataaggat 180
ttgcacacct atgggcaaac tgggagacag ctgccatcca ctgactcgta aagttccatt 240
ttttgggcgg aggatgcatc acacttgccc atgtctgcca ggcttggcct gtttacggac 300
ttcatttaac cgatttattt gtttagccca aaagtaatcg actagt 346

<210> 17
<211> 108
<212> PRT

<213> Human

<400> 17

Met Arg Ser Leu Cys Cys Ala Pro Leu Leu Leu Leu Leu Leu Leu Pro
                5                   10                  15
Pro Leu Leu Leu Thr Pro Arg Ala Gly Asp Ala Ala Val Ile Thr Gly
            20                  25                  30
Ala Cys Asp Lys Asp Ser Gln Cys Gly Gly Gly Met Cys Cys Ala Val
        35                  40                  45
Ser Ile Trp Val Lys Ser Ile Arg Ile Cys Thr Pro Met Gly Lys Leu
    50                  55                  60
Gly Asp Ser Cys His Pro Leu Thr Arg Lys Val Pro Phe Phe Gly Arg
65                  70                  75                  80
Arg Met His His Thr Cys Pro Cys Leu Pro Gly Leu Ala Cys Leu Arg
                85                  90                  95
Thr Ser Phe Asn Arg Phe Ile Cys Leu Ala Gln Lys
            100                 105

<210> 18

<211> 324

<212> DNA

<213> Human

<400> 18

atgaggagcc tgtgctgcgc cccactcctg ctcctcttgc tgctgccgcc gctgctgctc 60

acgccccgcg ctggggacgc cgccgtgatc accggggctt gtgacaagga ctcccaatgt 120

ggtggaggca tgtgctgtgc tgtcagtatc tgggtcaaga gcataaggat ttgcacacct 180

atgggcaaac tgggagacag ctgccatcca ctgactcgta aagttccatt ttttggcggg 240

aggatgcatc acacttgccc atgtctgcca ggcttggcct gtttacggac ttcatttaac 300

cgatttattt gtttagccca aaag 324

<210> 19

<211> 81

<212> PRT

<213> Human

<400> 19

Ala Val Ile Thr Gly Ala Cys Asp Lys Asp Ser Gln Cys Gly Gly Gly
5 10 15

Met Cys Cys Ala Val Ser Ile Trp Val Lys Ser Ile Arg Ile Cys Thr
20 25 30

Pro Met Gly Lys Leu Gly Asp Ser Cys His Pro Leu Thr Arg Lys Val
35 40 45

Pro Phe Phe Gly Arg Arg Met His His Thr Cys Pro Cys Leu Pro Gly
50 55 60

Leu Ala Cys Leu Arg Thr Ser Phe Asn Arg Phe Ile Cys Leu Ala Gln
65 70 75 80

Lys

<210> 20

<211> 243

<212> DNA

<213> Human

<400> 20

gccgtgatca ccggggcttg tgacaaggac tcccaatgtg gtggaggcat gtgctgtgct 60

gtcagtatct gggtcaagag cataaggatt tgcacaccta tgggcaaact gggagacagc 120

tgccatccac tgactcgtaa agttccattt tttgggcgga ggatgcatca cacttgccca 180

tgtctgccag gcttggcctg tttacggact tcatttaacc gatttatttg tttagcccaa 240

aag 243

<210> 21

<211> 80

<212> PRT

<213> Dendroaspip polylepis

<400> 21

Ala Val Ile Thr Gly Ala Cys Glu Arg Asp Leu Gln Cys Gly Lys Gly
5 10 15

Thr Cys Cys Ala Val Ser Leu Trp Ile Lys Ser Val Arg Val Cys Thr
20 25 30

Pro Val Gly Thr Ser Gly Glu Asp Cys His Pro Ala Ser His Lys Ile
35 40 45

Pro Phe Ser Gly Gln Arg Met His His Thr Cys Pro Cys Ala Pro Asn
50 55 60

Leu Ala Cys Val Gln Thr Ser Pro Lys Lys Phe Lys Cys Leu Ser Lys
65 70 75 80

<210> 22

<211> 80

<212> PRT

<213> Human

<400> 22

Ala Val Ile Thr Gly Ala Cys Asp Lys Asp Ser Gln Cys Gly Gly Gly
5 10 15

Met Cys Cys Ala Val Ser Ile Trp Val Lys Ser Ile Arg Ile Cys Thr

20 25 30

Pro Met Gly Lys Leu Gly Asp Ser Cys His Pro Leu Thr Arg Lys Val

35 40 45

Pro Phe Phe Gly Arg Arg Met His His Thr Cys Pro Cys Leu Pro Gly

50 55 60

Leu Ala Cys Leu Arg Thr Ser Phe Asn Arg Phe Ile Cys Leu Ala Gln

65 70 75 80

<210> 23

<211> 20

<212> DNA

<213> Artificial Sequence

<220>

<223> DNA primer, BF2 primer

<400> 23

gcttgygaca aggactcyca 20

<210> 24

<211> 19

<212> DNA

<213> Artificial Sequence

<220>

<223> DNA primer, BR1 primer

<400> 24

gttyctacty cagagygat 19

<210> 25
<211> 210
<212> DNA
<213> Human

<400> 25
gtgtggagga ggaatgtgct gcgctgtcag tatctgggtt aagagcataa ggatctgcac 60
acctatgggc caagtgggag acagctgcca ccccctgact cggaaagttc cattttgggg 120
gcggaggatg caccacactt gtccctgcct gccaggtttg gcatgtttaa ggacttcttt 180
caaccgtttt atttgtttgg cccggaagtg 210

<210> 26
<211> 26
<212> DNA
<213> Artificial Sequence

<220>
<223> DNA primer, RB5-1 primer

<400> 26
gtgcatcctc cgcccccaaa atggaa 26

<210> 27
<211> 28
<212> DNA
<213> Artificial Sequence

<220>

<223> DNA primer, RB5-3 primer

<400> 27
gacagcgcag cacattcctc ctccacac 28

<210> 28
<211> 148
<212> DNA
<213> Human

<400> 28
cgcgtcccta accgccaccg cctcctcggg acgccatgga ggacccgcgc tgtgccccgc 60
tactgctact tttgctgcta ccgctgctgc tcacaccgcc cgccggggat gccgcggtca 120
tcaccggggc ttgcgacaag gactctca 148

<210> 29
<211> 28
<212> DNA
<213> Artificial Sequence

<220>
<223> DNA primer, RB3-1 primer

<400> 29
gagacagctg ccaccccctg actcggaa 28

<210> 30
<211> 28
<212> DNA

<213> Artificial Sequence

<220>

<223> DNA primer, RB3-2 primer

<400> 30

ggcggaggat gcaccacact tgtccctg 28

<210> 31

<211> 150

<212> DNA

<213> Rat

<400> 31

cctgcctgcc aggtttggca tgtttaagga cttctttcaa ccgttttatt tgtttggccc 60
ggaagtgatc actctgaagc aggagctgga aatgtgaacc tctactcact gaacaatgtc 120
tgtcaagtct cgcttgtaat tgtgtcaaag 150

<210> 32

<211> 18

<212> DNA

<213> Artificial Sequence

<220>

<223> DNA primer, RBv8-WF1 primer

<400> 32

taaccgccac cgcctcct 18

<210> 33

<211> 17

<212> DNA

<213> Artificial Sequence

<220>

<223> DNA primer, RBv8-WF2 primer

<400> 33

gggacgccat ggaggac 17

<210> 34

<211> 26

<212> DNA

<213> Artificial Sequence

<220>

<223> DNA primer, RBv8-WR1 primer

<400> 34

cgagacttga cagacattgt tcagtg 26

<210> 35

<211> 20

<212> DNA

<213> Artificial Sequence

<220>

<223> DNA primer, RBv8-WR2 primer

<400> 35

tttccagctc ctgcttcaga 20

<210> 36

<211> 356

<212> DNA

<213> Rat

<400> 36

gggacgccat ggaggacccg cgctgtgccc cgctactgct acttttgctg ctaccgctgc 60

tgctcacacc gcccgccggg gatgccgcgg tcatcaccgg ggcttgcgac aaggactctc 120

agtgtggagg aggaatgtgc tgcgctgtca gtatctgggt taagagcata aggatctgca 180

cacctatggg ccaagtggga gacagctgcc aaccccctgac tcggaaagtt ccattttggg 240

ggcggaggat gcaccacact tgtccctgcc tgccaggttt ggcatgttta aggacttctt 300

tcaaccgttt tatttgtttg gcccggaagt gatcactctg aagcaggagc tggaaa 356

<210> 37

<211> 107

<212> PRT

<213> Rat

<400> 37

Met Glu Asp Pro Arg Cys Ala Pro Leu Leu Leu Leu Leu Leu Leu Pro

5 10 15

Leu Leu Leu Thr Pro Pro Ala Gly Asp Ala Ala Val Ile Thr Gly Ala

20 25 30

Cys Asp Lys Asp Ser Gln Cys Gly Gly Gly Met Cys Cys Ala Val Ser

35 40 45

Ile Trp Val Lys Ser Ile Arg Ile Cys Thr Pro Met Gly Gln Val Gly
50 55 60

Asp Ser Cys His Pro Leu Thr Arg Lys Val Pro Phe Trp Gly Arg Arg
65 70 75 80

Met His His Thr Cys Pro Cys Leu Pro Gly Leu Ala Cys Leu Arg Thr
85 90 95

Ser Phe Asn Arg Phe Ile Cys Leu Ala Arg Lys
100 105

<210> 38
<211> 321
<212> DNA
<213> Rat

<400> 38
atggaggacc cgcgctgtgc cccgctactg ctacttttgc tgctaccgct gctgctcaca 60
ccgccccgccg gggatgccgc ggtcatcacc ggggcttgcg acaaggactc tcagtgtgga 120
ggaggaatgt gctgcgctgt cagtatctgg gttaagagca taaggatctg cacacctatg 180
ggccaagtgg gagacagctg ccacccccctg actcggaaag ttccattttg ggggcggagg 240
atgcaccaca cttgtccctg cctgccaggt ttggcatgtt taaggacttc tttcaaccgt 300
tttatttgtt tggcccggaa g 321

<210> 39
<211> 81
<212> PRT
<213> Rat or Mouse

<400> 39
Ala Val Ile Thr Gly Ala Cys Asp Lys Asp Ser Gln Cys Gly Gly Gly

5 10 15

Met Cys Cys Ala Val Ser Ile Trp Val Lys Ser Ile Arg Ile Cys Thr

20 25 30

Pro Met Gly Gln Val Gly Asp Ser Cys His Pro Leu Thr Arg Lys Val

35 40 45

Pro Phe Trp Gly Arg Arg Met His His Thr Cys Pro Cys Leu Pro Gly

50 55 60

Leu Ala Cys Leu Arg Thr Ser Phe Asn Arg Phe Ile Cys Leu Ala Arg

65 70 75 80

Lys

<210> 40

<211> 243

<212> DNA

<213> Rat

<400> 40

gcggtcatca ccggggcttg cgacaaggac tctcagtgtg gaggaggaat gtgctgcgct 60

gtcagtatct gggttaagag cataaggatc tgcacaccta tgggccaagt gggagacagc 120

tgccaccccc tgactcggaa agttccattt tgggggcgga ggatgcacca cacttgtccc 180

tgcctgccag gtttggcatg tttaaggact tctttcaacc gttttatttg tttggcccgg 240

aag 243

<210> 41

<211> 30

<212> DNA

<213> Artificial Sequence

<220>

<223> DNA primer, primer 1

<400> 41
gtcgacatgg agaccactgt ggggaccctg 30

<210> 42
<211> 30
<212> DNA
<213> Artificial Sequence

<220>
<223> DNA primer, primer 2

<400> 42
actagtttat ttcagtcgga tgcagtccac 30

<210> 43
<211> 1179
<212> DNA
<213> Rat

<400> 43
atggagacca ctgtggggac cctgggcgag aataccacaa acactttcac cgacttcttt 60
tctgcacgtg atggcagtgg agccgaaacc tccccttgc cattcacttt cagctatggt 120
gactatgaca tgccctcgga tgaagaggag gatgtgacca actctcggac tttctttgct 180
gccaagattg tcattggcat ggctttggtg ggcatcatgc tggtgtgtgg catcggcaac 240
ttcatcttca tcactgcgct ggcccgctac aaaaagcttc gcaacctcac caacctgctt 300
atcgccaacc tggccatttc ggacttcctg gtagccatcg tgtgctgccc ctttgagatg 360
gactactatg tggtacgcca gctctcctgg gagcacggcc atgtcctgtg cgcctccgtc 420

aactacttgc gcaccgtctc cctctacgtg tccactaacg ccctactggc cattgccatt 480
gacaggtatc tggccattgt gcacccgctg agaccgcgga tgaagtgtca aacggctgca 540
ggcctgatct tcctggtgtg gtctgtgtcc atcctcatcg ccatcccagc cgcctacttc 600
accactgaga cggtgttggt catcgtggaa agccaggaga agatcttctg cggccagatc 660
tggccggtgg atcagcagtt ctactacagg tcctatttcc ttttggtctt cggcctcgag 720
ttcgtgggtc ctgtaatcgc catgaccctg tgctatgcca gggtgtcccg agagctctgg 780
ttcaaggcgg tgcccggctt ccagacagag cagatccgcc ggaggctgcg ctgtcgccga 840
cggacggtac tggggctcgt gtgcgtcctt tccgcctatg tgctgtgctg ggctcccttc 900
tatggcttca ccatcgtgcg tgacttcttc ccctccgtgt ttgtgaaaga gaagcactac 960
ctcaccgcct tttatgtggt ggagtgcatc gccatgagca acagtatgat caatacgctg 1020
tgctttgtga ctgtcaggaa taacaccagt aagtacctca agaggatcct gcggctccag 1080
tggagggcct ctcctagcgg gagcaaggcc agcgctgacc tcgacctcag gaccacgggg 1140
attcctgcca cggaggaggt ggactgcatc cgactgaaa 1179

<210> 44

<211> 393

<212> PRT

<213> Rat

<400> 44

Met Glu Thr Thr Val Gly Thr Leu Gly Glu Asn Thr Thr Asn Thr Phe
5 10 15

Thr Asp Phe Phe Ser Ala Arg Asp Gly Ser Gly Ala Glu Thr Ser Pro
20 25 30

Leu Pro Phe Thr Phe Ser Tyr Gly Asp Tyr Asp Met Pro Ser Asp Glu
35 40 45

Glu Glu Asp Val Thr Asn Ser Arg Thr Phe Phe Ala Ala Lys Ile Val
50 55 60

Ile Gly Met Ala Leu Val Gly Ile Met Leu Val Cys Gly Ile Gly Asn

65 70 75 80

Phe Ile Phe Ile Thr Ala Leu Ala Arg Tyr Lys Lys Leu Arg Asn Leu

85 90 95

Thr Asn Leu Leu Ile Ala Asn Leu Ala Ile Ser Asp Phe Leu Val Ala

100 105 110

Ile Val Cys Cys Pro Phe Glu Met Asp Tyr Tyr Val Val Arg Gln Leu

115 120 125

Ser Trp Glu His Gly His Val Leu Cys Ala Ser Val Asn Tyr Leu Arg

130 135 140

Thr Val Ser Leu Tyr Val Ser Thr Asn Ala Leu Leu Ala Ile Ala Ile

145 150 155 160

Asp Arg Tyr Leu Ala Ile Val His Pro Leu Arg Pro Arg Met Lys Cys

165 170 175

Gln Thr Ala Ala Gly Leu Ile Phe Leu Val Trp Ser Val Ser Ile Leu

180 185 190

Ile Ala Ile Pro Ala Ala Tyr Phe Thr Thr Glu Thr Val Leu Val Ile

195 200 205

Val Glu Ser Gln Glu Lys Ile Phe Cys Gly Gln Ile Trp Pro Val Asp

210 215 220

Gln Gln Phe Tyr Tyr Arg Ser Tyr Phe Leu Leu Val Phe Gly Leu Glu

225 230 235 240

Phe Val Gly Pro Val Ile Ala Met Thr Leu Cys Tyr Ala Arg Val Ser

245 250 255

Arg Glu Leu Trp Phe Lys Ala Val Pro Gly Phe Gln Thr Glu Gln Ile

260 265 270

Arg Arg Arg Leu Arg Cys Arg Arg Arg Thr Val Leu Gly Leu Val Cys

275 280 285

Val Leu Ser Ala Tyr Val Leu Cys Trp Ala Pro Phe Tyr Gly Phe Thr

290 295 300

Ile Val Arg Asp Phe Phe Pro Ser Val Phe Val Lys Glu Lys His Tyr
305 310 315 320
Leu Thr Ala Phe Tyr Val Val Glu Cys Ile Ala Met Ser Asn Ser Met
325 330 335
Ile Asn Thr Leu Cys Phe Val Thr Val Arg Asn Asn Thr Ser Lys Tyr
340 345 350
Leu Lys Arg Ile Leu Arg Leu Gln Trp Arg Ala Ser Pro Ser Gly Ser
355 360 365
Lys Ala Ser Ala Asp Leu Asp Leu Arg Thr Thr Gly Ile Pro Ala Thr
370 375 380
Glu Glu Val Asp Cys Ile Arg Leu Lys
385 390

<210> 45
<211> 31
<212> DNA
<213> Artificial Sequence

<220>
<223> DNA primer, probe 1

<400> 45
cctcaccaay ctgctyatyg ccaacctggc c 31

<210> 46
<211> 26
<212> DNA
<213> Artificial Sequence

<220>

<223> DNA primer, probe 2

<400> 46

gtggtrcgsc agctctcctg ggagca 26

<210> 47

<211> 23

<212> DNA

<213> Artificial Sequence

<220>

<223> DNA primer, primer 1

<400> 47

tcccgggagc tctggttcaa ggc 23

<210> 48

<211> 27

<212> DNA

<213> Artificial Sequence

<220>

<223> DNA primer, primer 2

<400> 48

gagtgcatcg ccatgagcaa cagcatg 27

<210> 49

<211> 23
<212> DNA
<213> Artificial Sequence

<220>
<223> DNA primer, primer 3

<400> 49
ggcttgaacc agagctcccg gga 23

<210> 50
<211> 1263
<212> DNA
<213> Rat

<400> 50
atggtatcag ttctgtccaa cagggacctc cacacactgg ccccagctga agtgctgaac 60
tccacgtggg cctatctccc tgacacatac cagcctacct gccacatcat caacatggga 120
gaccagaacg gaaacacaag ctttgcacca gacttgaacc caccccaaga ccacgtctcc 180
ttgctcccct taaactacag ttatggagat tatgacatcc ccctggatga cgatgaggat 240
gtgaccaaga cacagacctt ctttgcagcc aaaatcgtca ttggcgtagc cctggcaggc 300
atcatgctag tctgcggcgt tggcaacttt gtcttcattg ctgccctcgc ccgctacaag 360
aagctgcgca accttaccaa cctcctcatc gctaacctgg ccatctctga cttcctggtg 420
gcgatcgtct gctgcccctt tgagatggac tactacgtag tacgtcagct ttcctgggag 480
catggtcacg tgctttgtgc ctccgtcaac taccttcgta cagtctccct gtacgtctcc 540
accaatgctc tgctggccat cgctattgac agatatctcg ctattgtcca ccccttaaaa 600
cggatgaatt accagaccgc ctccttcctg atcgctttgg tctggatggt ctccatcctc 660
atcgccatcc catctgccta cttcaccaca gaaaccatcc ttgttatcgt caagaatcag 720
gaaaagctct tctgtggtca gatctggccc gtggaccagc agctctacta caaatcctac 780

ttcctcttcg tcttcgggct tgagttcgtg ggtcccgtgg tcactatgac cctgtgctat 840
gccaggatct cccaggagct ctggttcaag gctgtacctg gtttccagac ggagcagatc 900
cgcaagcgac tgcgctgccg ccgaaagaca gtgctattgc tcatgggtat cctcacagcc 960
tacgtgctgt gctgggcgcc tttctatggc tttaccatag tgcgagactt cttccccacg 1020
ctggttgtga aggagaagca ctacctcacc gccttctatg tcgtcgagtg catcgccatg 1080
agcaacagca tgatcaatac tatatgcttc gtgacggtca agaacaacac catgaaatac 1140
ttcaagaaga tgctgctgct gcactggcgg ccctctcact acgggagtaa gtccagcgcg 1200
gacctcgacc tcaaaaccag tggggttcct gccaccgaag aggtggactg tatcaggcta 1260
aag 1263

<210> 51
<211> 421
<212> PRT
<213> Rat

<400> 51
Met Val Ser Val Leu Ser Asn Arg Asp Leu His Thr Leu Ala Pro Ala
5 10 15
Glu Val Leu Asn Ser Thr Trp Ala Tyr Leu Pro Asp Thr Tyr Gln Pro
20 25 30
Thr Cys His Ile Ile Asn Met Gly Asp Gln Asn Gly Asn Thr Ser Phe
35 40 45
Ala Pro Asp Leu Asn Pro Pro Gln Asp His Val Ser Leu Leu Pro Leu
50 55 60
Asn Tyr Ser Tyr Gly Asp Tyr Asp Ile Pro Leu Asp Asp Asp Glu Asp
65 70 75 80
Val Thr Lys Thr Gln Thr Phe Phe Ala Ala Lys Ile Val Ile Gly Val
85 90 95
Ala Leu Ala Gly Ile Met Leu Val Cys Gly Val Gly Asn Phe Val Phe

100 105 110

Ile Ala Ala Leu Ala Arg Tyr Lys Lys Leu Arg Asn Leu Thr Asn Leu

115 120 125

Leu Ile Ala Asn Leu Ala Ile Ser Asp Phe Leu Val Ala Ile Val Cys

130 135 140

Cys Pro Phe Glu Met Asp Tyr Tyr Val Val Arg Gln Leu Ser Trp Glu

145 150 155 160

His Gly His Val Leu Cys Ala Ser Val Asn Tyr Leu Arg Thr Val Ser

165 170 175

Leu Tyr Val Ser Thr Asn Ala Leu Leu Ala Ile Ala Ile Asp Arg Tyr

180 185 190

Leu Ala Ile Val His Pro Leu Lys Arg Met Asn Tyr Gln Thr Ala Ser

195 200 205

Phe Leu Ile Ala Leu Val Trp Met Val Ser Ile Leu Ile Ala Ile Pro

210 215 220

Ser Ala Tyr Phe Thr Thr Glu Thr Ile Leu Val Ile Val Lys Asn Gln

225 230 235 240

Glu Lys Leu Phe Cys Gly Gln Ile Trp Pro Val Asp Gln Gln Leu Tyr

245 250 255

Tyr Lys Ser Tyr Phe Leu Phe Val Phe Gly Leu Glu Phe Val Gly Pro

260 265 270

Val Val Thr Met Thr Leu Cys Tyr Ala Arg Ile Ser Gln Glu Leu Trp

275 280 285

Phe Lys Ala Val Pro Gly Phe Gln Thr Glu Gln Ile Arg Lys Arg Leu

290 295 300

Arg Cys Arg Arg Lys Thr Val Leu Leu Leu Met Gly Ile Leu Thr Ala

305 310 315 320

Tyr Val Leu Cys Trp Ala Pro Phe Tyr Gly Phe Thr Ile Val Arg Asp

325 330 335

Phe Phe Pro Thr Leu Val Val Lys Glu Lys His Tyr Leu Thr Ala Phe
340 345 350

Tyr Val Val Glu Cys Ile Ala Met Ser Asn Ser Met Ile Asn Thr Ile
355 360 365

Cys Phe Val Thr Val Lys Asn Asn Thr Met Lys Tyr Phe Lys Lys Met
370 375 380

Leu Leu Leu His Trp Arg Pro Ser His Tyr Gly Ser Lys Ser Ser Ala
385 390 395 400

Asp Leu Asp Leu Lys Thr Ser Gly Val Pro Ala Thr Glu Glu Val Asp
405 410 415

Cys Ile Arg Leu Lys
420

<210> 52
<211> 384
<212> PRT
<213> Human

<400> 52

Met Ala Ala Gln Asn Gly Asn Thr Ser Phe Thr Pro Asn Phe Asn Pro
5 10 15

Pro Gln Asp His Ala Ser Ser Leu Ser Phe Asn Phe Ser Tyr Gly Asp
20 25 30

Tyr Asp Leu Pro Met Asp Glu Asp Glu Asp Met Thr Lys Thr Arg Thr
35 40 45

Phe Phe Ala Ala Lys Ile Val Ile Gly Ile Ala Leu Ala Gly Ile Met
50 55 60

Leu Val Cys Gly Ile Gly Asn Phe Val Phe Ile Ala Ala Leu Thr Arg
65 70 75 80

Tyr Lys Lys Leu Arg Asn Leu Thr Asn Leu Leu Ile Ala Asn Leu Ala
85 90 95

Ile Ser Asp Phe Leu Val Ala Ile Ile Cys Cys Pro Phe Glu Met Asp
100 105 110

Tyr Tyr Val Val Arg Gln Leu Ser Trp Glu His Gly His Val Leu Cys
115 120 125

Ala Ser Val Asn Tyr Leu Arg Thr Val Ser Leu Tyr Val Ser Thr Asn
130 135 140

Ala Leu Leu Ala Ile Ala Ile Asp Arg Tyr Leu Ala Ile Val His Pro
145 150 155 160

Leu Lys Pro Arg Met Asn Tyr Gln Thr Ala Ser Phe Leu Ile Ala Leu
165 170 175

Val Trp Met Val Ser Ile Leu Ile Ala Ile Pro Ser Ala Tyr Phe Ala
180 185 190

Thr Glu Thr Val Leu Phe Ile Val Lys Ser Gln Glu Lys Ile Phe Cys
195 200 205

Gly Gln Ile Trp Pro Val Asp Gln Gln Leu Tyr Tyr Lys Ser Tyr Phe
210 215 220

Leu Phe Ile Phe Gly Val Glu Phe Val Gly Pro Val Val Thr Met Thr
225 230 235 240

Leu Cys Tyr Ala Arg Ile Ser Arg Glu Leu Trp Phe Lys Ala Val Pro
245 250 255

Gly Phe Gln Thr Glu Gln Ile Arg Lys Arg Leu Arg Cys Arg Arg Lys
260 265 270

Thr Val Leu Val Leu Met Cys Ile Leu Thr Ala Tyr Val Leu Cys Trp
275 280 285

Ala Pro Phe Tyr Gly Phe Thr Ile Val Arg Asp Phe Phe Pro Thr Val
290 295 300

Phe Val Lys Glu Lys His Tyr Leu Thr Ala Phe Tyr Val Val Glu Cys

305 310 315 320

Ile Ala Met Ser Asn Ser Met Ile Asn Thr Val Cys Phe Val Thr Val

325 330 335

Lys Asn Asn Thr Met Lys Tyr Phe Lys Lys Met Met Leu Leu His Trp

340 345 350

Arg Pro Ser Gln Arg Gly Ser Lys Ser Ser Ala Asp Leu Asp Leu Arg

355 360 365

Thr Asn Gly Val Pro Thr Thr Glu Glu Val Asp Cys Ile Arg Leu Lys

370 375 380

<210> 53

<211> 393

<212> PRT

<213> Mouse

<400> 53

Met Glu Thr Thr Val Gly Ala Leu Gly Glu Asn Thr Thr Asp Thr Phe

5 10 15

Thr Asp Phe Phe Ser Ala Leu Asp Gly His Glu Ala Gln Thr Gly Ser

20 25 30

Leu Pro Phe Thr Phe Ser Tyr Gly Asp Tyr Asp Met Pro Leu Asp Glu

35 40 45

Glu Glu Asp Val Thr Asn Ser Arg Thr Phe Phe Ala Ala Lys Ile Val

50 55 60

Ile Gly Met Ala Leu Val Gly Ile Met Leu Val Cys Gly Ile Gly Asn

65 70 75 80

Phe Ile Phe Ile Thr Ala Leu Ala Arg Tyr Lys Lys Leu Arg Asn Leu

85 90 95

Thr Asn Leu Leu Ile Ala Asn Leu Ala Ile Ser Asp Phe Leu Val Ala

100 105 110

Ile Val Cys Cys Pro Phe Glu Met Asp Tyr Tyr Val Val Arg Gln Leu

115 120 125

Ser Trp Glu His Gly His Val Leu Cys Ala Ser Val Asn Tyr Leu Arg

130 135 140

Thr Val Ser Leu Tyr Val Ser Thr Asn Ala Leu Leu Ala Ile Ala Ile

145 150 155 160

Asp Arg Tyr Leu Ala Ile Val His Pro Leu Arg Pro Arg Met Lys Cys

165 170 175

Gln Thr Ala Ala Gly Leu Ile Phe Leu Val Trp Ser Val Ser Ile Leu

180 185 190

Ile Ala Ile Pro Ala Ala Tyr Phe Thr Thr Glu Thr Val Leu Val Ile

195 200 205

Val Glu Arg Gln Glu Lys Ile Phe Cys Gly Gln Ile Trp Pro Val Asp

210 215 220

Gln Gln Phe Tyr Tyr Arg Ser Tyr Phe Leu Leu Val Phe Gly Leu Glu

225 230 235 240

Phe Val Gly Pro Val Val Ala Met Thr Leu Cys Tyr Ala Arg Val Ser

245 250 255

Arg Glu Leu Trp Phe Lys Ala Val Pro Gly Phe Gln Thr Glu Gln Ile

260 265 270

Arg Arg Thr Val Arg Cys Arg Arg Arg Thr Val Leu Gly Leu Val Cys

275 280 285

Val Leu Ser Ala Tyr Val Leu Cys Trp Ala Pro Phe Tyr Gly Phe Thr

290 295 300

Ile Val Arg Asp Phe Phe Pro Ser Val Phe Val Lys Glu Lys His Tyr

305 310 315 320

Leu Thr Ala Phe Tyr Val Val Glu Cys Ile Ala Met Ser Asn Ser Met

325 330 335

Ile Asn Thr Leu Cys Phe Val Thr Val Arg Asn Asn Thr Ser Lys Tyr
340 345 350
Leu Lys Arg Ile Leu Arg Leu Gln Trp Arg Ala Ser Pro Ser Gly Ser
355 360 365
Lys Ala Ser Ala Asp Leu Asp Leu Arg Thr Thr Gly Ile Pro Ala Thr
370 375 380
Glu Glu Val Asp Cys Ile Arg Leu Lys
385 390

<210> 54
<211> 381
<212> PRT
<213> Mouse

<400> 54
Met Gly Pro Gln Asn Arg Asn Thr Ser Phe Ala Pro Asp Leu Asn Pro
5 10 15
Pro Gln Asp His Val Ser Leu Asn Tyr Ser Tyr Gly Asp Tyr Asp Leu
20 25 30
Pro Leu Gly Glu Asp Glu Asp Val Thr Lys Thr Gln Thr Phe Phe Ala
35 40 45
Ala Lys Ile Val Ile Gly Val Ala Leu Ala Gly Ile Met Leu Val Cys
50 55 60
Gly Ile Gly Asn Phe Val Phe Ile Ala Ala Leu Ala Arg Tyr Lys Lys
65 70 75 80
Leu Arg Asn Leu Thr Asn Leu Leu Ile Ala Asn Leu Ala Ile Ser Asp
85 90 95
Phe Leu Val Ala Ile Val Cys Cys Pro Phe Glu Met Asp Tyr Tyr Val
100 105 110

Val Arg Gln Leu Ser Trp Ala His Gly His Val Leu Cys Ala Ser Val
115 120 125
Asn Tyr Leu Arg Thr Val Ser Leu Tyr Val Ser Thr Asn Ala Leu Leu
130 135 140
Ala Ile Ala Ile Asp Arg Tyr Leu Ala Ile Val His Pro Leu Lys Pro
145 150 155 160
Arg Met Asn Tyr Gln Thr Ala Ser Phe Leu Ile Ala Leu Val Trp Met
165 170 175
Val Ser Ile Leu Ile Ala Val Pro Ser Ala Tyr Phe Thr Thr Glu Thr
180 185 190
Ile Leu Val Ile Val Lys Asn Gln Glu Lys Ile Phe Cys Gly Gln Ile
195 200 205
Trp Ser Val Asp Gln Gln Leu Tyr Tyr Lys Ser Tyr Phe Leu Phe Val
210 215 220
Phe Gly Leu Glu Phe Val Gly Pro Val Val Thr Met Thr Leu Cys Tyr
225 230 235 240
Ala Arg Ile Ser Gln Glu Leu Trp Phe Lys Ala Val Pro Gly Phe Gln
245 250 255
Thr Glu Gln Ile Arg Lys Arg Leu Arg Cys Arg Arg Lys Thr Val Leu
260 265 270
Leu Leu Met Gly Ile Leu Thr Ala Tyr Val Leu Cys Trp Ala Pro Phe
275 280 285
Tyr Gly Phe Thr Ile Val Arg Asp Phe Phe Pro Thr Val Val Val Lys
290 295 300
Glu Lys His Tyr Leu Thr Ala Phe Tyr Val Val Glu Cys Ile Ala Met
305 310 315 320
Ser Asn Ser Met Ile Asn Thr Ile Cys Phe Val Thr Val Lys Asn Asn
325 330 335
Thr Met Lys Tyr Phe Lys Lys Met Leu Arg Leu His Trp Arg Pro Ser

340 345 350

His Tyr Gly Ser Lys Ser Ser Ala Asp Leu Asp Leu Lys Thr Ser Gly

355 360 365

Val Pro Ala Thr Glu Glu Val Asp Cys Ile Arg Leu Lys

370 375 380

<210> 55

<211> 107

<212> PRT

<213> Mouse

<400> 55

Met Gly Asp Pro Arg Cys Ala Pro Leu Leu Leu Leu Leu Leu Leu Pro

5 10 15

Leu Leu Phe Thr Pro Pro Ala Gly Asp Ala Ala Val Ile Thr Gly Ala

20 25 30

Cys Asp Lys Asp Ser Gln Cys Gly Gly Gly Met Cys Cys Ala Val Ser

35 40 45

Ile Trp Val Lys Ser Ile Arg Ile Cys Thr Pro Met Gly Gln Val Gly

50 55 60

Asp Ser Cys His Pro Leu Thr Arg Lys Val Pro Phe Trp Gly Arg Arg

65 70 75 80

Met His His Thr Cys Pro Cys Leu Pro Gly Leu Ala Cys Leu Arg Thr

85 90 95

Ser Phe Asn Arg Phe Ile Cys Leu Ala Arg Lys

100 105

<210> 56

<211> 321

<212> DNA

<213> Mouse

<400> 56

atgggggacc cgcgctgtgc cccgctactg ctacttctgc tgctaccgct gctgttcaca 60

ccgcccgccg gggatgccgc ggtcatcacc ggggcttgcg acaaggactc tcagtgcgga 120

ggaggcatgt gctgtgctgt cagtatctgg gttaagagca taaggatctg cacacctatg 180

ggccaagtgg gcgacagctg ccacccctg actcggaaag ttccattttg ggggcggagg 240

atgcaccaca cctgcccctg cctgccaggc ttggcgtgtt taaggacttc tttcaaccgg 300

tttatttgct tggcccggaa a 321

<210> 57

<211> 243

<212> DNA

<213> Mouse

<400> 57

gcggtcatca ccggggcttg cgacaaggac tctcagtgcg gaggaggcat gtgctgtgct 60

gtcagtatct gggttaagag cataaggatc tgcacaccta tgggccaagt gggcgacagc 120

tgccaccccc tgactcggaa agttccattt tgggggcgga ggatgcacca cacctgcccc 180

tgcctgccag gcttggcgtg tttaaggact tctttcaacc ggtttatttg cttggcccgg 240

aaa 243

<210> 58

<211> 21

<212> DNA

<213> Artificial Sequence

<220>

<223> DNA primer, primer 3

<400> 58

tcatgttgct ccactggaag g 21

<210> 59

<211> 21

<212> DNA

<213> Artificial Sequence

<220>

<223> DNA primer, primer 4

<400> 59

ccaattgtct tgaggtccag g 21

<210> 60

<211> 29

<212> DNA

<213> Artificial Sequence

<220>

<223> DNA probe, ZAQC probe

<400> 60

ttcttacaat ggcggtaagt ccagtgcag 29

<210> 61

<211> 31

<212> DNA

<213> Artificial Sequence

<220>

<223> DNA primer, ZAQC Sal primer

<400> 61

gtcgacatgg agaccaccat ggggttcatg g 31

<210> 62

<211> 36

<212> DNA

<213> Artificial Sequence

<220>

<223> DNA primer, ZAQC Spe primer

<400> 62

actagtttat tttagtctga tgcagtccac ctcttc 36

<210> 63

<211> 1179

<212> DNA

<213> Mouse

<400> 63

atggagacca ctgtcggggc tctgggtgag aataccacag acaccttcac cgacttcttt 60

tctgcactcg atggccatga agcccaaacc ggctcgttac cattcacttt cagctacggt 120

gactatgaca tgcccctgga tgaagaggaa gatgtgacca attctcggac tttctttgct 180

gccaagattg tcattggcat ggctttggtg ggtatcatgc tagtgtgtgg catcggcaac 240
ttcatcttta tcactgccct ggcccgctac aaaaagctcc gcaacctcac caacctgctt 300
atcgccaacc tggccatttc agacttcctc gtggccatcg tgtgctgccc ctttgagatg 360
gactactatg tggtgcgcca gctctcctgg gagcatggtc atgtcctgtg cgcctctgtc 420
aactacttgc gtaccgtctc cctctacgtc tccactaacg ccctactggc cattgccatt 480
gacaggtatc tggccattgt gcacccgctg agaccgcgga tgaagtgtca aacagccgcc 540
ggcctgatct tcctggtgtg gtcagtatcc atcctcatcg ccattccagc tgcctacttc 600
accactgaga ccgtgctggt catcgtggag agacaggaga agatcttctg tggtcagatc 660
tggccggtgg atcagcagtt ctactacagg tcctatttcc ttttggtttt cggcctcgag 720
ttcgtgggcc ccgtagtcgc catgaccttg tgctatgcca gggtgtcccg ggagctctgg 780
ttcaaggcgg tgccaggctt ccagacagag cagatccgcc ggacggtgcg ctgccgccgc 840
aggacggtgc tggggctcgt gtgcgtcctc tctgcctatg tgctgtgctg ggctcccttc 900
tatggcttca ctatcgtgcg tgacttcttc ccctccgtgt ttgtgaagga gaagcactac 960
ctcaccgcct tctatgtggt ggagtgcatc gccatgagca acagcatgat caatacgctc 1020
tgctttgtga ctgtcaggaa taacaccagt aagtacctca agaggatcct gcggcttcag 1080
tggagggcct ctcccagcgg gagcaaggcc agcgctgacc tcgacctcag gaccacggga 1140
atacctgcca ccgaggaggt ggactgcatc cgactgaaa 1179

<210> 64
<211> 1143
<212> DNA
<213> Mouse

<400> 64
atgggacccc agaacagaaa cactagcttt gcaccagact tgaatccacc ccaagaccat 60
gtctccttaa actacagtta tggtgattat gacctccccc tgggtgagga tgaggatgtg 120
accaagacac agaccttctt tgcagccaaa attgtcattg gcgtggcact ggcaggcatc 180
atgctggtct gcggcattgg caactttgtc ttcattgctg ccctcgcccg ctacaagaag 240
ctgcgcaacc ttaccaacct cctcattgct aacctggcca tctctgactt cctggtggcg 300

atcgtctgct gcccctttga gatggactat tatgtagtac ggcagctttc ctgggcgcat 360
ggtcacgtgc tttgtgcctc cgtcaactac cttcgtacgg tctccctgta cgtctccacc 420
aacgctctgc tggccatcgc tattgacaga tacctcgcta ttgtccaccc tttgaaacca 480
cggatgaatt atcagaccgc ttccttcctg atcgctttgg tctggatggt ctccatcctc 540
atcgctgtcc catctgccta cttcaccaca gaaaccatcc tcgttatcgt caagaatcaa 600
gaaaaaatct tctgtggtca gatctggtcg gtggaccagc agctctacta caaatcctac 660
ttcctcttcg tcttcgggct tgagttcgtg ggtcccgtgg tcactatgac cctgtgctat 720
gccaggatct cccaagagct ctggttcaag gctgtacctg gcttccagac ggagcaaatc 780
cgcaagcggc tgcgttgccg ccgcaagaca gtgctactgc tcatgggcat cctcacagcc 840
tacgtgctgt gctgggcgcc gttctatggc tttaccatag tgcgagactt cttccccacg 900
gtagttgtga aggagaagca ctacctcacc gccttctacg tcgtggagtg cattgccatg 960
agcaacagca tgatcaatac tatatgcttc gtgacggtca agaacaacac catgaaatac 1020
ttcaagaaga tgctgcggct ccactggcgg ccctctcact acgggagtaa gtccagcgct 1080
gacctcgacc tcaaaaccag cggggtgcct gccactgaag aggtggattg tatcagacta 1140
aag 1143

<210> 65
<211> 82
<212> DNA
<213> Artificial Sequence

<220>
<223> DNA oligomer

<400> 65
tatggcggtg attaccggtg cgtgcgataa agatagccag tgcggtggcg gtatgtgctg 60
tgcggtgagc atttgggtga aa 82

<210> 66

<211> 82

<212> DNA

<213> Artificial Sequence

<220>

<223> DNA oligomer

<400> 66

agcattcgta tttgcacccc gatgggcaaa ctgggcgata gctgccatcc gctgacccgt 60

aaagtgccgt tttttggccg cc 82

<210> 67

<211> 87

<212> DNA

<213> Artificial Sequence

<220>

<223> DNA oligomer

<400> 67

gtatgcatca tacctgcccg tgcctgccgg gcctggcgtg cctgcgcacc agctttaacc 60

gctttatttg cctggcgcag aaatagg 87

<210> 68

<211> 88

<212> DNA

<213> Artificial Sequence

<220>

<223> DNA oligomer

<400> 68
cgaatgcttt tcacccaaat gctcaccgca cagcacatac cgccaccgca ctggctatct 60
ttatcgcacg caccggtaat caccgcca 88

<210> 69
<211> 85
<212> DNA
<213> Artificial Sequence

<220>
<223> DNA oligomer

<400> 69
atgatgcata cggcggccaa aaaacggcac tttacgggtc agcggatggc agctatcgcc 60
cagtttgccc atcggggtgc aaata 85

<210> 70
<211> 80
<212> DNA
<213> Artificial Sequence

<220>
<223> DNA oligomer

<400> 70
gatccctatt tctgcgccag gcaaataaag cggttaaagc tggtgcgcag gcacgccagg 60
cccggcaggc acgggcaggt 80

<210> 71
<211> 243
<212> DNA
<213> Artificial Sequence

<220>
<223> synthetic DNA

<400> 71
gcggtgatta ccggtgcgtg cgataaagat agccagtgcg gtggcggtat gtgctgtgcg 60
gtgagcattt gggtgaaaag cattcgtatt tgcaccccga tgggcaaact gggcgatagc 120
tgccatccgc tgacccgtaa agtgccgttt tttggccgcc gtatgcatca tacctgcccg 180
tgcctgccgg gcctggcgtg cctgcgcacc agctttaacc gctttatttg cctggcgcag 240
aaa 243

## Description

TECHNICAL FIELD

The present invention relates to a method of screening a compound or its salt, etc. useful for the prevention/treatment of digestive diseases, which comprises using (i) a peptide or its salt containing the same or substantially the same amino acid sequence as the amino acid sequence represented by SEQ ID NO: 19 or SEQ ID NO: 39; and (ii) a protein or its salt containing the same or substantially the same amino acid sequence as the amino acid sequence represented by SEQ ID NO: 1, SEQ ID NO: 44, SEQ ID NO: 51, SEQ ID NO: 52, SEQ ID NO: 53 or SEQ ID NO: 54, which is an orphan receptor protein; and the like.

BACKGROUND ART

Important biological functions including maintenance of homeostasis in the living body, reproduction, development of individuals, metabolism, growth, control of the nervous, circulatory, immune, digestive or metabolic system, sensory adaptation, etc. are regulated by cells that receive endogenous factors such as various hormones and neurotransmitters or sensory stimulation like light or odor, via specific receptors present on cell membranes reserved for these endogenous factors or stimulation and interact with them. Many of these receptors for hormones or neurotransmitters, which take part in such functional regulation, are coupled to guanine nucleotide-binding proteins (hereinafter, sometimes merely referred to as G proteins), and are characterized by developing a variety of functions through mediation of intracellular signal transduction via activation of the G proteins. In addition, these receptor proteins possess common seven transmembrane regions. Based on the foregoing, these receptors are thus collectively referred to as G protein-coupled receptors or seven transmembrane receptors. As such, it is known that various hormones or neurotransmitters and their receptor proteins are present and interact with each other to play important roles for regulating the biological functions. However, it often remains unclear if there are any other unknown substances (hormones, neurotransmitters, etc.) and receptors to these substances.

In recent years, accumulated sequence information of human genome DNA or various human tissue-derived cDNAs by random sequencing and rapid progress in gene analysis technology have been accelerating the investigation of human genome. With such advance, it has been clarified that there are many genes supposed to encode proteins with unknown functions. G protein-coupled receptors not only have seven transmembrane domains but many common sequences are present in their nucleic acids or amino acids. Thus, these receptors can be precisely identified to be G protein-coupled receptors in such proteins. On the other hand, these G protein-coupled receptor genes are obtained also by polymerase chain reaction (hereinafter abbreviated as PCR) utilizing such a structural similarity. In these G protein-coupled receptors thus obtained so far, ligands to some receptors that are subtypes having high homology in structure to known receptors may be readily predictable but in most cases, their endogenous ligands are unpredictable so that ligands corresponding to these receptors are hardly found. For this reason, these receptors are termed orphan receptors. It is likely that unidentified endogenous ligands to such orphan receptors would participate in biological phenomena poorly analyzed because the ligands were unknown. When such ligands are associated with important physiological effects or pathologic conditions, it is expected that development of these receptor agonists or antagonists will result in breakthrough new drugs (Stadel, J. et al., TiPS, 18, 430-437, 1997; Marchese, A. et al., TiPS, 20, 370-375, 1999; Civelli, O. et al., Brain Res., 848, 63-65, 1999). Until now, however, there are few examples to actually identify ligands to orphan G protein-coupled receptors.

Recently, some groups attempted to investigate ligands to these orphan receptors and reported isolation/structural determination of ligands, which are novel physiologically active peptides. Independently, Reinsheid et al. and Meunier et al. introduced a cDNA coding for orphan G protein-coupled receptor LC132 or ORL1 into animal cells to express a receptor, isolated a novel peptide from porcine brain or rat brain extract, which was named orphanin FQ or nociceptin, with reference to its response and determined its sequence (Reinsheid, R. K. et al., Science, 270, 792-794, 1995; Meunier, J.-C. et al., Nature, 377, 532-535, 1995). This peptide was reported to be associated with pain. Further research on the receptor in knockout mice reveals that the peptide takes part in memory (Manabe, T. et al., Nature, 394, 577-581, 1998).

Subsequently, novel peptides such as PrRP (prolactin releasing peptide), orexin, apelin, ghrelin and GALP (galanin-like peptide), etc. were isolated as ligands to orphan G protein-coupled receptors (Hinuma, S. et al., Nature, 393, 272-276, 1998; Sakurai, T. et al., Cell, 92, 573-585, 1998; Tatemoto, K. et al., Biohem. Biophys. Res. Commun., 251, 471-476, 1998; Kojima, M. et al., Nature, 402, 656-660, 1999; Ohtaki, T. et al., J. Biol. Chem., 274, 37041-37045, 1999). On the other hand, some receptors to physiologically active peptides, which were so far unknown, were clarified. It was revealed that a receptor to motilin associated with contraction of intestinal tracts was GPR38 (Feighner, S. D. et al., Science, 284, 2184-2188, 1999). Furthermore, SLC-1 was identified to be a receptor to melanin concentrating hormone (MCH) (Chambers, J. et al., Nature, 400, 261-265, 1999; Saito, Y. et al., Nature, 400, 265-269, 1999; Shimomura, Y. et al., Biochem. Biophys. Res. Commun., 261, 622-626, 1999; Lembo, P. M. C. et al., Nature Cell Biol., 1, 267-271, 1999; Bachner, D. et al., FEBS Lett., 457, 522-524, 1999). Also, GPR14 (SENR) was reported to be a receptor to urotensin II (Ames, R. S. et al., Nature, 401, 282-286, 1999; Mori, M. et al., Biochem. Biophys. Res. Commun., 265, 123-129, 1999; Nothacker, H. -P. et al., Nature Cell Biol., 1, 383-385, 1999, Liu, Q. et al., Biochem. Biophys. Res. Commun., 266, 174-178, 1999). It was shown that MCH took part in obesity since its knockout mouse showed the reduced body weight and lean phenotype (Shimada, M. et al., Nature, 396, 670-674, 1998), and because its receptor was identified, it became possible to explore a receptor antagonist likely to be an anti-obesity agent. It is further reported that urotensin II shows a potent action on the cardiocirculatory system, since it induces heart ischemia by intravenous injection to monkey (Ames, R. S. et al., Nature, 401, 282-286, 1999).

As described above, orphan receptors and ligands thereto often take part in a new physiological activity, and it is expected that their identification will lead to development of new drugs. However, it is known that research on ligands to orphan receptors is accompanied by many difficulties. The presence of many orphan receptors was unraveled, but due to the foregoing problems, only a very small part of ligands to these receptors were discovered so far.

The present inventors found a novel receptor ZAQ (a protein having the same or substantially the same amino acid sequence as the amino acid sequence represented by SEQ ID NO: 1 in the specification: hereinafter sometimes merely referred to as ZAQ in the specification), but its ligand was unidentified yet.

The problems to be solved were to pursue a ligand to the orphan receptor protein ZAQ and establish a method for screening a compound, etc. characterized by using the ligand.

## DISCLOSURE OF THE INVENTION

The present inventors prepared human type Bv8 peptide (FEBS Letters, 462, 177-181, 1999) and found that this peptide had a ZAQ binding activity. Furthermore, the inventors successfully cloned cDNA encoding rat type Bv8.

Based on these findings, the inventors have found that therapeutic drugs for diseases associated with ZAQ (ZAQ antagonists or agonists, etc., specifically drugs for the prevention/treatment of digestive diseases, etc.) can be screened by the screening system using human type/mouse type/rat type Bv8 peptide. As a result of extensive investigations, the inventors have come to accomplish the present invention.

That is, the present invention provides the following features:

(1) A method of screening a compound or its salt that alters the binding property of a peptide or its salt containing the same or substantially the same amino acid sequence as the amino acid sequence represented by SEQ ID NO: 19 or SEQ ID NO: 39, to a protein or its salt containing the same or substantially the same amino acid sequence as the amino acid sequence represented by SEQ ID NO: 1, SEQ ID NO: 44, SEQ ID NO: 51, SEQ ID NO: 52, SEQ ID NO: 53 or SEQ ID NO: 54, which comprises using a peptide, its partial peptide or a salt thereof, containing the same or substantially the same amino acid sequence as the amino acid sequence represented by SEQ ID NO: 19 or SEQ ID NO: 39, and a protein, its partial protein or a salt thereof, containing the same or substantially the same amino acid sequence as the amino acid sequence represented by SEQ ID NO: 1, SEQ ID NO: 44, SEQ ID NO: 51, SEQ ID NO: 52, SEQ ID NO: 53 or SEQ ID NO: 54;

(2) A kit for screening a compound or its salt that alters the binding property of a peptide or its salt containing the same or substantially the same amino acid sequence as the amino acid sequence represented by SEQ ID NO: 19 or SEQ ID NO: 39, to a protein or its salt containing the same or substantially the same amino acid sequence as the amino acid sequence represented by SEQ ID NO: 1, SEQ ID NO: 44, SEQ ID NO: 51, SEQ ID NO: 52, SEQ ID NO: 53 or SEQ ID NO: 54, comprising a peptide, its partial peptide or a salt thereof, containing the same or substantially the same amino acid sequence as the amino acid sequence represented by SEQ ID NO: 19 or SEQ ID NO: 39, and a protein, its partial protein or a salt thereof, containing the same or substantially the same amino acid sequence as the amino acid sequence represented by SEQ ID NO: 1, SEQ ID NO: 44, SEQ ID NO: 51, SEQ ID NO: 52, SEQ ID NO: 53 or SEQ ID NO: 54;

(3) A compound or its salt that alters the binding property of a peptide or its salt containing the same or substantially the same amino acid sequence as the amino acid sequence represented by SEQ ID NO: 19 or SEQ ID NO: 39, to a protein or its salt containing the same or substantially the same amino acid sequence as the amino acid sequence represented by SEQ ID NO: 1, SEQ ID NO: 44, SEQ ID NO: 51, SEQ ID NO: 52, SEQ ID NO: 53 or SEQ ID NO: 54, which is obtainable using the screening method according to (1) or using the screening kit according to (2);

(4) A pharmaceutical comprising the compound or its salt according to (3);

(5) The pharmaceutical according to (4), which is an agent for the prevention/treatment of a digestive disease;

(6) A peptide containing the same or substantially the same amino acid sequence as the amino acid sequence represented by SEQ ID NO: 37, or a salt thereof;

(7) A DNA encoding the peptide according to (6);

(8) The DNA according to (7) containing the base sequence represented by SEQ ID NO: 38;
(9) A DNA containing the base sequence represented by SEQ ID NO: 40;
(10) A recombinant vector containing the DNA according to (8);
(11) A transformant transformed by the recombinant vector according to (10);
(12) A method of manufacturing the peptide or its salt according to (6), which comprises culturing the transformant of (11) and producing/accumulating the peptide according to (6);
(13) An antibody to a peptide or its salt containing the same or substantially the same amino acid sequence as the amino acid sequence represented by SEQ ID NO: 39;
(14) A diagnostic agent comprising the antibody according to (13);
(15) The diagnostic agent according to (14), which is a diagnostic agent for digestive diseases;
(16) A non-human mammal bearing the DNA according to (7), or its variant DNA, which is exogenous;
(17) The mammal according to (16), wherein the non-human mammal is a rodent;
(18) The mammal according to (17), wherein the rodent is rat;
(19) A recombinant vector bearing the DNA according to (9), or its variant DNA, which is exogenous and capable of expressing in a non-human mammal;
(20) A non-human mammal embryonic stem cell, in which the DNA according to (7) or (9) is inactivated;
(21) The embryonic stem cell according to (20), wherein the DNA is inactivated by introducing a reporter gene therein;
(22) The embryonic stem cell according to (21), wherein the non-human mammal is a rodent;
(23) A non-human mammal deficient in expressing the DNA according to (7) or (9), wherein the said DNA is inactivated;
(24) The non-human mammal according to (23), wherein the DNA is inactivated by inserting a reporter gene therein and the reporter gene is capable of expressing under control of a promoter to the DNA of the invention;
(25) The non-human mammal according to (23), wherein the non-human mammal is a rodent;
(26) A method of screening a compound or its salt that promotes or inhibits the promoter activity to the DNA according to (7) or (9), which comprises administering a test compound to the mammal according to (24) and detecting expression of the reporter gene;
(27) A method of preventing and/or treating digestive diseases, which comprises administering to a mammal an effective dose of the compound or its salt according to (3);
(28) Use of the compound or its salt according to (3), for manufacturing a preventive and/or therapeutic agent of digestive diseases; etc. The present invention further provides the following features:
(29) A method of screening a compound or its salt that alters the binding property between the peptide of the invention and the protein of the invention, which comprises assaying binding amounts of the peptide of the invention to the protein of the invention, when a peptide, its partial peptide or a salt thereof, containing the same or substantially the same amino acid sequence as the amino acid sequence represented by SEQ ID NO: 19 or SEQ ID NO: 39 (hereinafter sometimes merely referred to as the peptide of the invention) is brought in contact with a protein, its partial peptide or a salt thereof, containing the same or substantially the same amino acid sequence as the amino acid sequence represented by SEQ ID NO: 1, SEQ ID NO: 44, SEQ ID NO: 51, SEQ ID NO: 52, SEQ ID NO: 53 or SEQ ID NO: 54 (hereinafter sometimes merely referred to as the protein of the invention), and when the peptide of the invention and a test compound are brought in contact with the protein of the invention; and comparing the binding amounts;
(30) A method of screening a compound or its salt that alters the binding property between the peptide of the invention and the protein of the invention, which comprises, when the peptide of the invention is brought in contact with a cell or its cell membrane fraction containing the protein of the invention and when the peptide of the invention and a test compound are brought in contact with a cell or its cell membrane fraction containing the protein of the invention, assaying binding amounts of the peptide of the invention to the cell or the membrane fraction; and comparing the binding amounts;
(31) The screening method according to (30), wherein the protein of the invention is the protein of the invention expressed on the cell membrane by culturing a transformant bearing a DNA encoding the protein of the invention;
(32) The screening method according to (29) through (31), wherein the peptide of the invention is a labeled ligand;
(33) A method of screening a compound or its salt that alters the binding property between the peptide of the invention and the protein of the invention, which comprises assaying cell stimulating activities mediated by the protein of the invention, when the peptide of the invention is brought in contact with the protein of the invention and when the peptide of the invention and a test compound are brought in contact with the protein of the invention; and comparing the binding amounts;
(34) A method of screening a compound or its salt that alters the binding property between the peptide of the invention and the protein of the invention, which comprises assaying cell stimulating activities mediated by the protein of the invention, when the peptide of the invention is brought in contact with a cell or its cell membrane fraction containing the protein of the invention and when the peptide of the invention and a test compound are brought in contact with a cell or its cell membrane fraction containing the protein of the invention; and comparing the activities;

(35) The screening method according to (34), wherein the protein of the invention is the protein of the invention expressed on the cell membrane by culturing a transformant bearing a DNA encoding the protein of the invention;

(36) A method of screening a compound or its salt that alters the binding property between the peptide of the invention and the protein of the invention, which comprises assaying GTPγS-binding promoting activities to a cell membrane fraction of the protein of the invention in the presence of labeled GTPγS, when the peptide of the invention is brought in contact with the cell membrane fraction of the protein of the invention and when the peptide of the invention and a test compound are brought in contact with the cell membrane fraction of the protein of the invention; and comparing the activities;

(37) A method of screening a compound or its salt that alters the binding property between the peptide of the invention and the protein of the invention, which comprises, when the peptide of the invention is brought in contact with a cell wherein the protein of the invention is expressed and when the peptide of the invention and a test compound are brought in contact with a cell wherein the protein of the invention is expressed, assaying intracellular cAMP production suppressing activities on the cell in the presence of a substance capable of increasing the intracellular cAMP level; and comparing the activities;

(38) A method of screening a compound or its salt that alters the binding property between the peptide of the invention and the protein of the invention, which comprises assaying enzyme activities of a reporter gene protein in the presence of a substance capable of increasing the intracellular cAMP level, when the peptide of the invention is brought in contact with a CRE-reporter gene vector-transfected cell wherein the protein of the invention is expressed and when the peptide of the invention and a test compound are brought in contact with a CRE-reporter gene vector-transfected cell wherein the protein of the invention is expressed; and comparing the activities;

(39) A method of screening a compound or its salt that alters the binding property between the peptide of the invention and the protein of the invention, which comprises the arachidonic acid releasing activities, when the peptide of the invention is brought in contact with a labeled arachidonic acid-containing cell wherein the protein of the invention is expressed and when the peptide of the invention and a test compound are brought in contact with a labeled arachidonic acid-containing cell wherein the protein of the invention is expressed; and comparing the activities;

(40) A method of screening a compound or its salt that alters the binding property between the peptide of the invention and the protein of the invention, which comprises assaying intracellular calcium level increasing activities when the peptide of the invention is brought in contact with a cell wherein the protein of the invention is expressed and when the peptide of the invention and a test compound are brought in contact with a cell wherein the protein of the invention is expressed; and comparing the activities;

(41) A method of screening a compound or its salt that alters the binding property between the peptide of the invention and the protein of the invention, which comprises assaying inositol triphosphate producing activities in the presence of labeled inositol, when the peptide of the invention is brought in contact with a cell wherein the protein of the invention is expressed and when the peptide of the invention and a test compound are brought in contact with a cell wherein the protein of the invention is expressed; and comparing the activities;

(42) A method of screening a compound or its salt that alters the binding property between the peptide of the invention and the protein of the invention, which comprises assaying enzyme activities of a reporter gene protein, when the peptide of the invention is brought in contact with a TRE-reporter gene vector-transfected cell wherein the protein of the invention is expressed and when the peptide of the invention and a test compound are brought in contact with a TRE-reporter gene vector-transfected cell wherein the protein of the invention is expressed; and comparing the activities;

(43) A method of screening a compound or its salt that alters the binding property between the peptide of the invention and the protein of the invention, which comprises assaying cell growth, when the peptide of the invention is brought in contact with a cell wherein the protein of the invention is expressed and when the peptide of the invention and a test compound are brought in contact with a cell wherein the protein of the invention is expressed; and comparing the cell growth;

(44) A method of screening a compound or its salt that alters the binding property between the peptide of the invention and the protein of the invention, which comprises assaying efflux activities of labeled rubidium in the presence of labeled rubidium, when the peptide of the invention is brought in contact with a cell wherein the protein of the invention is expressed and when the peptide of the invention and a test compound are brought in contact with a cell wherein the protein of the invention is expressed; and comparing the activities;

(45) A method of screening a compound or its salt that alters the binding property between the peptide of the invention and the protein of the invention, which comprises assaying extracellular pH changes, when the peptide of the invention is brought in contact with a cell wherein the protein of the invention is expressed and when the peptide of the invention and a test compound are brought in contact with a cell wherein the protein of the invention is expressed; and comparing the changes;

(46) A method of screening a compound or its salt that alters the binding property between the peptide of the invention and the protein of the invention, which comprises culturing histidine synthesis gene-transfected yeast wherein the protein of the invention is expressed in a histidine-deficient medium, allowing the yeast to contact with the peptide of the invention or with the peptide of the invention and a test compound, assaying and comparing growth of the yeast;

(47) A method of screening a compound or its salt that alters the binding property between the peptide of the invention and the protein of the invention, which comprises measuring changes in cell membrane potentials, when the peptide of the invention is brought in contact with *Xenopus laevis* oocytes wherein RNA of the gene encoding the protein of the invention is introduced and when the peptide of the invention and a test compound are brought in contact with *Xenopus laevis* oocytes wherein RNA of the gene encoding the protein of the invention is introduced; and comparing the changes;

(48) A compound or its salt that alters the binding property between the peptide of the invention and the protein of the invention, which is obtainable by the screening method according to (29) through (47);

(49) A pharmaceutical comprising the compound or its salt according to (48);

(50) The screening kit according to (2), comprising a cell containing the peptide of the invention;

(51) The screening kit according to (2), comprising a cell membrane fraction containing a cell membrane fraction containing the protein of the invention;

(52) The screening kit according to (2), comprising a protein expressed on a cell membrane of the transformant as defined below, by culturing a transformant transformed with a recombinant vector bearing a DNA containing the DNA encoding the protein of the invention;

(53) A compound or its salt that alters the binding property between the peptide of the invention and the protein of the invention, which is obtainable by the screening kit according to (50) through (52);

(54) A pharmaceutical comprising a compound or its salt that alters the binding property between the peptide of the invention and the protein of the invention, which is obtainable by the screening kit according to (50) through (52);

(55) A method for quantification of the peptide of the invention, which comprises contacting the antibody according to (11) with the peptide of the invention;

(56) A method for quantification of the peptide of the invention in a test sample fluid, which comprises competitively reacting the antibody according to (11) with a test sample fluid and a labeled form of the peptide of the invention, and measuring a ratio of the labeled form of the peptide of the invention, which is bound to the antibody;

(57) A method for quantification of the peptide of the invention in a test sample fluid, which comprises reacting a test sample fluid simultaneously or sequentially with the antibody according to (11) immobilized on a carrier and a labeled form of the antibody according to (11), and then assaying the activity of a labeling agent on the insoluble carrier;

(58) A polynucleotide according to (7), which is hybridizable to the DNA under high stringent conditions;

(59) A polynucleotide, which has a base sequence complementary to the base sequence of the DNA according to (7) or its partial base sequence; etc.

BRIEF DESCRIPTION OF THE DRAWINGS

FIG. 1 shows the base sequence of the DNA encoding human brain-derived protein obtained in EXAMPLE 1 (ZAQC) and the amino acid sequence deduced therefrom (continued to FIG. 2).

FIG. 2 shows the base sequence of the DNA encoding human brain-derived protein obtained in EXAMPLE 1 (ZAQC) and the amino acid sequence deduced therefrom (continued from FIG. 1 and to FIG. 3).

FIG. 3 shows the base sequence of the DNA encoding human brain-derived protein obtained in EXAMPLE 1 (ZAQC) and the amino acid sequence deduced therefrom (continued from FIG. 2).

FIG. 4 shows the base sequence of the DNA encoding human brain-derived protein obtained in EXAMPLE 1 (ZAQT) and the amino acid sequence deduced therefrom (continued to FIG. 5).

FIG. 5 shows the base sequence of the DNA encoding human brain-derived protein obtained in EXAMPLE 1 (ZAQT) and the amino acid sequence deduced therefrom (continued from FIG. 4 and to FIG. 6).

FIG. 6 shows the base sequence of the DNA encoding human brain-derived protein obtained in EXAMPLE 1 (ZAQT) and the amino acid sequence deduced therefrom (continued from FIG. 5).

FIG. 7 shows a hydrophobic plot of the human brain-derived protein obtained in EXAMPLE 1.

FIG. 8 shows the results of analysis on expression distribution of ZAQ performed in EXAMPLE 1.

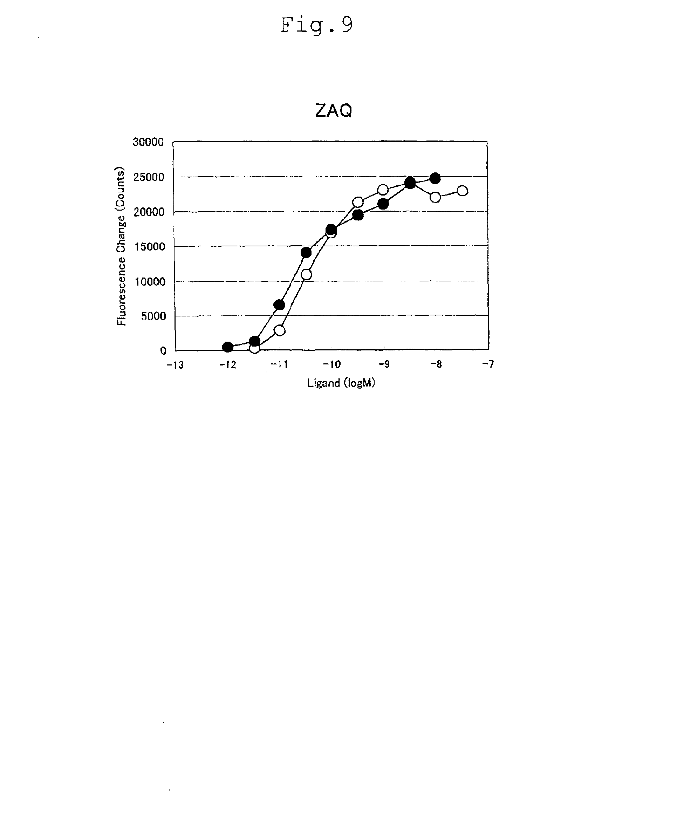

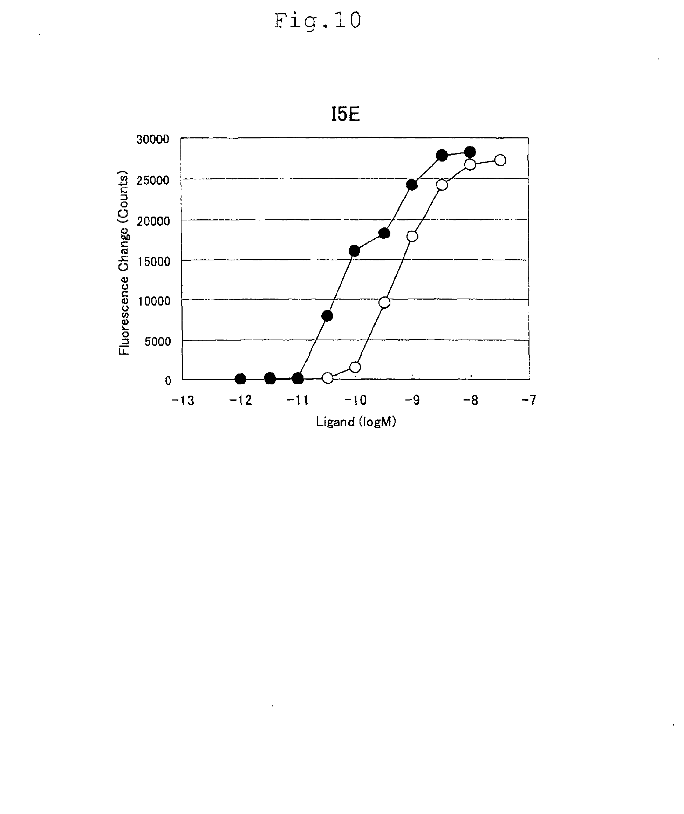

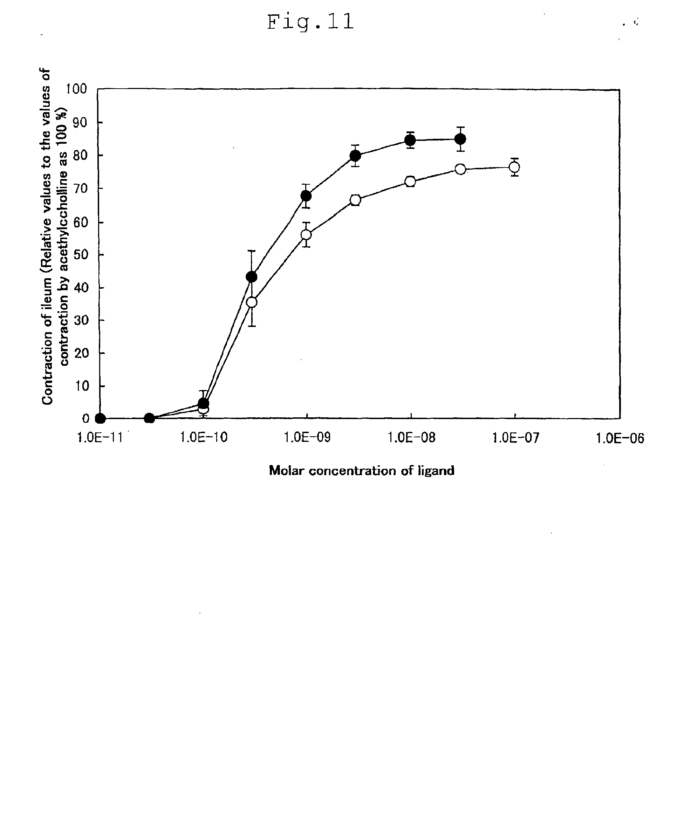

FIG. 9 shows the results of assay for the ZAQ receptor activating activity of human type Bv8 peptide and MIT1, which was performed in EXAMPLE 4, wherein -o- and -•- denote human type Bv8 peptide and MIT1, respectively. FIG. 10 shows the results of assay for the I5E receptor activating activity of human type Bv8 peptide and MIT1, which was performed in EXAMPLE 4, wherein - o- and -•- denote human type Bv8 peptide and MIT1, respectively. FIG. 11 shows the results of contraction experiments on human type Bv8 peptide and MIT1 performed in EXAMPLE 8, wherein -o- and -•- denote human type Bv8 peptide and MIT1, respectively.

BEST MODE FOR CARRYING OUT THE INVENTION

The peptide characterized by containing the same or substantially the same amino acid sequence as the amino acid sequence represented by SEQ ID NO: 19 or SEQ ID NO: 39, or its salt (hereinafter sometimes merely referred to as the peptide of the invention) is a peptide capable of binding to a protein containing the same or substantially the same amino acid sequence as the amino acid sequence represented by SEQ ID NO: 1, SEQ ID NO: 44, SEQ ID NO: 51, SEQ ID NO: 52, SEQ ID NO: 53 or SEQ ID NO: 54, or its salt (hereinafter sometimes merely referred to as the protein of the invention), and is a peptide or its salt capable of binding to the protein of the invention or its salt to activate the same. The capabilities of the peptide of the invention to bind to the protein of the invention and to activate the protein of the invention can be determined by the methods, which will be later described.
The peptide of the invention may be any peptide derived from any cell of human or non-human mammals (e. g., guinea pig, rat, mouse, rabbit, swine, sheep, bovine, monkey, etc.) (e.g., splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.); hemocyte type cells (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.); or any tissues where such cells are present, such as brain or any of brain regions (e.g., olfactory bulb, amygdaloid nucleus, basal ganglia, hippocampus, thalamus, hypothalamus, subthalamic nucleus, cerebral cortex, medulla oblongata, cerebellum, occipital lobes, frontal lobe, lateral lobe, putamen, caudate nucleus, corpus callosum, substantia nigra), spinal cord, pituitary gland, stomach, pancreas, kidneys, liver, gonads, thyroid gland, gallbladder, bone marrow, adrenal glands, skin, muscle, lung, digestive tracts (e.g., colon, small intestine), vascular vessels, heart, thymus, spleen, submandibular gland, peripheral blood, peripheral blood cells, prostate, testicles, testes, ovaries, placenta, uterus, bones, joints, skeletal muscles, etc. (especially, brain and each region of the brain). The peptide may also be a synthetic peptide.
When the peptide of the invention has a signal sequence, the peptide can be efficiently secreted extracellularly.
The amino acid sequence which is substantially the same amino acid sequence as the amino acid sequence represented by SEQ ID NO: 19 or SEQ ID NO: 39 includes an amino acid sequence having at least about 60% homology (preferably at least about 70% homology, further preferably at least about 80%, more preferably at least about 85%, much more preferably at least about 85%, particularly preferably at least about 90%, and most preferably at least about 95% homology) to the amino acid sequence represented by SEQ ID NO: 19 or SEQ ID NO: 39.
Examples of the peptide which has substantially the same amino acid sequence as the amino acid sequence represented by SEQ ID NO: 19 or SEQ ID NO: 39 include a peptide containing substantially the same amino acid sequence as the amino acid sequence represented by SEQ ID NO: 19 or SEQ ID NO: 39 and having an activity substantially equivalent to that of the amino acid sequence represented by SEQ ID NO: 19 or SEQ ID NO: 39, etc.
Hereinafter, the peptide containing the amino acid sequence represented by SEQ ID NO: 19 and the peptide containing the amino acid sequence represented by SEQ ID NO: 39 are sometimes referred to as human type Bv8 mature peptide, and as rat type Bv8 mature peptide or mouse type Bv8 mature peptide, respectively.
Preferred examples of the peptides, which contain the same or substantially the same amino acid sequence as the amino acid sequence represented by SEQ ID NO: 19, are peptides containing the same or substantially the same amino acid sequence as the amino acid sequence represented by SEQ ID NO: 19 and having an activity substantially equivalent to that of the amino acid sequence represented by SEQ ID NO: 19. Specifically, the peptides include peptides containing the amino acid sequence represented by SEQ ID NO: 17 or SEQ ID NO: 19, and the like.
Preferred examples of the peptides, which contain the same or substantially the same amino acid sequence as the amino acid sequence represented by SEQ ID NO: 39, are peptides containing the same or substantially the same amino acid sequence as the amino acid sequence represented by SEQ ID NO: 39 and having an activity substantially equivalent to that of the amino acid sequence represented by SEQ ID NO: 39. Specifically, the peptides include peptides containing the amino acid sequence represented by SEQ ID NO: 37, SEQ ID NO: 39 or SEQ ID NO: 55, and the like.
Hereinafter, the peptide containing the amino acid sequence represented by SEQ ID NO: 17 is sometimes referred to as rat type Bv8 precursor peptide. The peptide containing the amino acid sequence represented by SEQ ID NO: 55 is sometimes referred to as mouse type Bv8 precursor peptide.

The substantially equivalent activity refers to, for example, a binding activity to the protein of the invention, a signal transduction activity mediated by the protein of the invention, etc. The term substantially equivalent is used to mean that these activities are equivalent in nature. It is thus preferred that these activities such as a binding activity to the protein of the invention, a signal transduction activity mediated by the protein of the invention, etc. are equivalent in potency (e.g., about 0.5 to about 2 times), and it is allowable that even differences among grades such as the strength of these activities and molecular weight of the peptide are present.

These activities can be assayed by a modification of publicly known methods, and may also be assayed by, for example, the screening methods that will be later described.

As the peptide of the invention, there are also employed peptides containing (i) an amino acid sequence wherein at least 1 or 2 (preferably approximately 1 to 30, more preferably approximately 1 to 20) amino acids are deleted in the amino acid sequence represented by SEQ ID NO: 19 or SEQ ID NO: 39, (ii) an amino acid sequence wherein at least 1 or 2 (preferably approximately 1 to 40, more preferably approximately 1 to 30, particularly preferably approximately 1 to 20) amino acids are added to the amino acid sequence represented by SEQ ID NO: 19 or SEQ ID NO: 39, (iii) an amino acid sequence wherein at least 1 or 2 (preferably approximately 1 to 40, more preferably approximately 1 to 30, particularly preferably approximately 1 to 20) amino acids in the amino acid sequence represented by SEQ ID NO: 19 or SEQ ID NO: 39 are substituted with other amino acids, or (iv) an amino acid sequence in combination thereof; or the like.

The partial peptides of the peptide of the invention may be any partial peptides so long as they can be employed for the screening methods for pharmaceuticals, etc., which will be later described, and may have an activity substantially equivalent to the activity of the peptide of the invention. In the partial peptides, the number of amino acids is at least 10 and preferably at least 20, in the amino acid sequence, which constitutes the peptide of the invention.

Herein, the term "substantially equivalent activity" is intended to mean the same significance as defined above. The "substantially equivalent activity" can be assayed in the same way as described above.

The partial peptides may be those (i) wherein at least 1 or 2 (preferably several (1 to 4)) amino acids are deleted in the amino acid sequences described above, (ii) wherein at least 1 or 2 (preferably approximately 1 to 20, more preferably approximately 1 to 10 and most preferably several (1 to 5)) amino acids are added to the amino acid sequences described above, or (iii) wherein at least 1 or 2 (preferably several (1 to 4)) amino acids in the amino acid sequences described above are substituted with other amino acids.

Hereinafter the peptide of the invention and the partial peptides of the peptide of the invention are sometimes collectively referred to as the peptide of the invention.

The protein of the invention may be any protein derived from any cells of human or non-human mammals (e.g., guinea pig, rat, mouse, rabbit, swine, sheep, bovine, monkey, etc.) (e.g., splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.); or hemocyte type cells (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.); or any tissues where such cells are present, such as brain or any of brain regions (e.g., olfactory bulb, amygdaloid nucleus, basal ganglia, hippocampus, thalamus, hypothalamus, subthalamic nucleus, cerebral cortex, medulla oblongata, cerebellum, occipital lobes, frontal lobe, lateral lobe, putamen, caudate nucleus, corpus callosum, substantia nigra), spinal cord, pituitary gland, stomach, pancreas, kidneys, liver, gonads, thyroid gland, gallbladder, bone marrow, adrenal glands, skin, muscle, lung, digestive tracts (e.g., colon, small intestine), vascular vessels, heart, thymus, spleen, submandibular gland, peripheral blood, peripheral blood cells, prostate, testes, testicles, ovaries, placenta, uterus, bones, joints, skeletal muscles, etc. (especially, brain and each region of the brain); the proteins may also be synthetic proteins.

When the protein of the invention has a signal sequence, the peptide or protein can be efficiently secreted extracellularly.

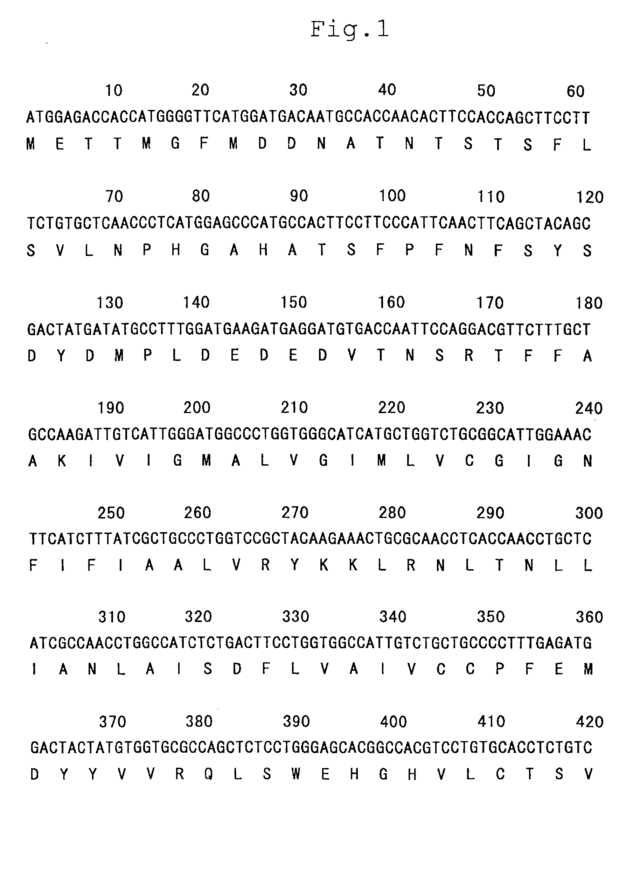

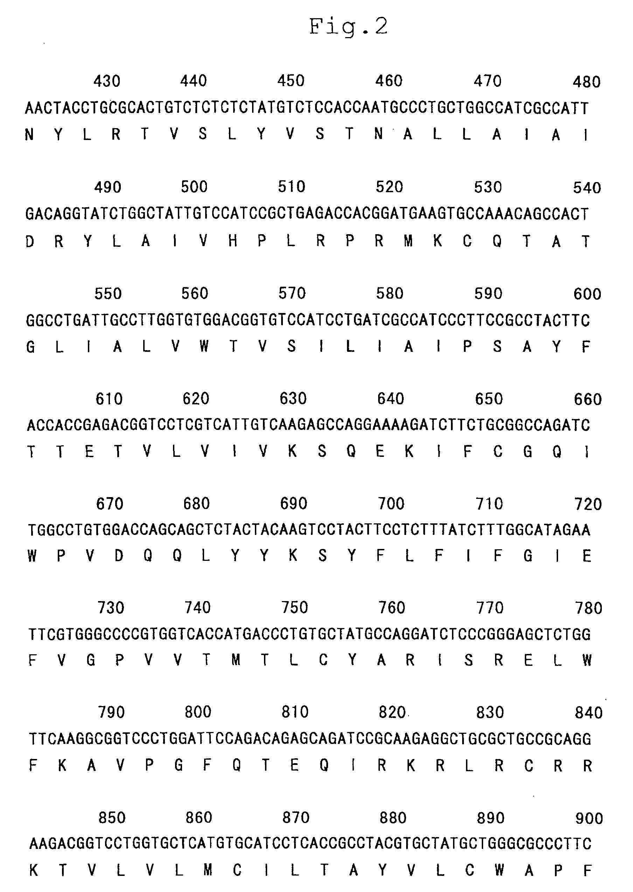

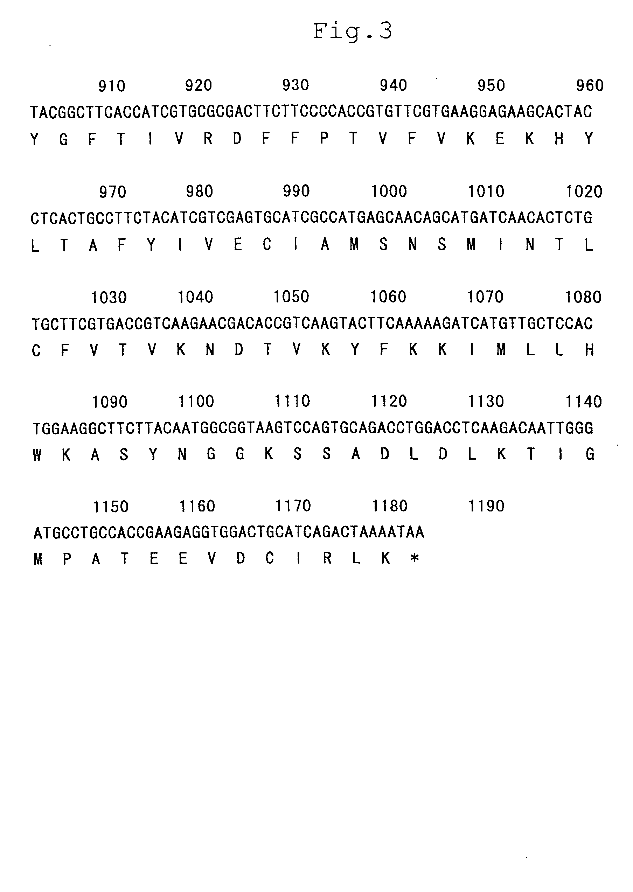

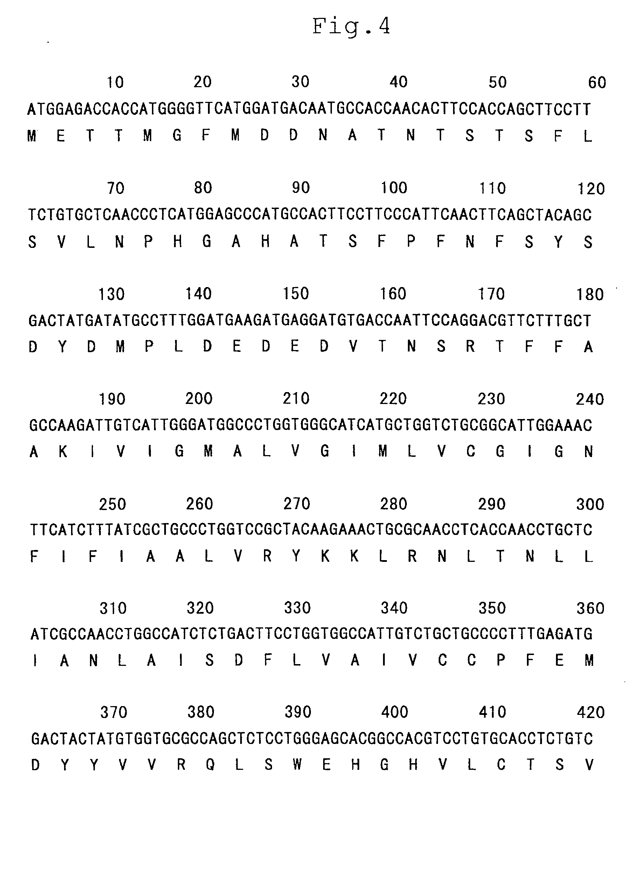

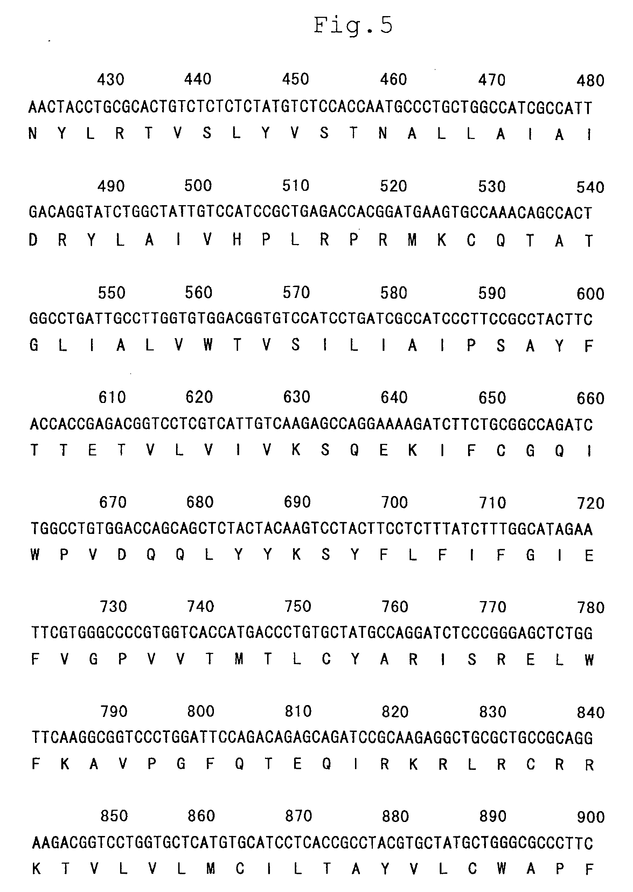

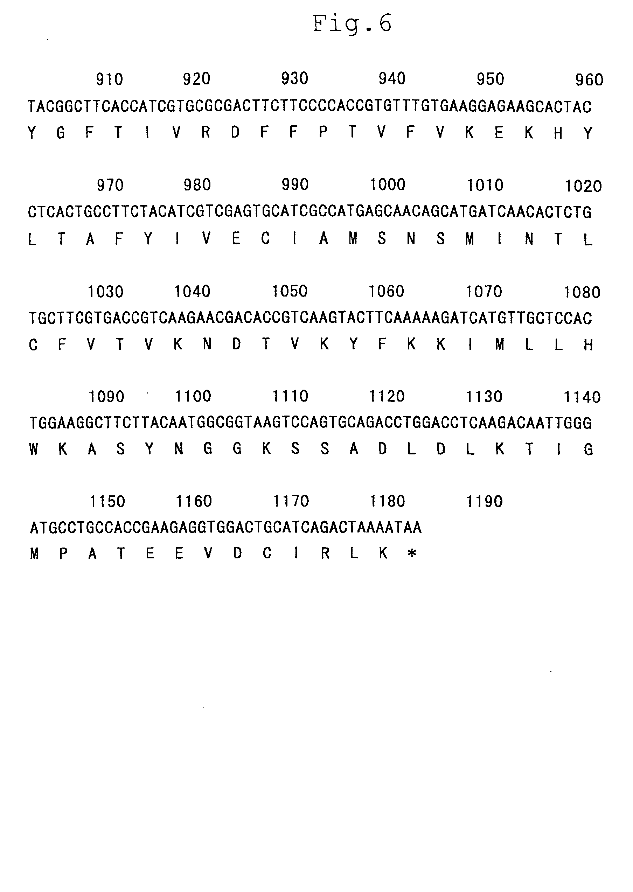

As the protein of the invention (G protein-coupled receptor protein), there are preferably used receptor proteins containing the same or substantially the same amino acid sequence as the amino acid sequence represented by SEQ ID NO: 1 (the amino acid sequence shown in FIGS. 1 through 3 or FIGS. 4 through 6), and the like.

The amino acid sequence which is substantially the same amino acid sequence as the amino acid sequence represented by SEQ ID NO: 1 includes, e.g., an amino acid sequence having at least about 90% homology, preferably at least about 95% homology and more preferably at least about 98% homology to the amino acid sequence represented by SEQ ID NO: 1, and the like.

As the protein containing substantially the same amino acid sequence as the amino acid sequence represented by SEQ ID NO: 1, preferred are, for example, proteins containing substantially the same amino acid sequence as the amino acid sequence represented by SEQ ID NO: 1 and having an activity substantially equivalent to that of the protein having the amino acid sequence represented by SEQ ID NO: 1, and the like.

As the protein of the invention containing the same or substantially the same amino acid sequence as the amino acid sequence represented by SEQ ID NO: 1, preferred are, for example, proteins containing the same or substantially the same amino acid sequence as the amino acid sequence represented by SEQ ID NO: 1 and having an activity substantially equivalent to that of the amino acid sequence represented by SEQ ID NO: 1, and the like.

The substantially equivalent activity refers to, for example, a binding activity to the peptide of the invention, a signal transduction activity, etc. The term substantially equivalent is used to mean that these activities are equivalent in nature. It is thus preferred that these activities such as a binding activity to the peptide of the invention, a signal transduction activity, etc. are equivalent in potency (e.g., about 0.5 to about 2 times), and it is allowable that even differences among grades such as the strength of these activities and molecular weight of the protein are present.

These activities such as a binding activity to the peptide of the invention, a signal transduction activity, etc. can be assayed by publicly known methods with a modification.

Hereinafter the protein having the amino acid sequence represented by SEQ ID NO: 1 is sometimes referred to as ZAQ.

Also as the protein of the invention, there are employed proteins containing (i) an amino acid sequence wherein at least 1 or 2 (preferably approximately 1 to 30, more preferably approximately 1 to 10, and much more preferably approximately several (1 or 2)) amino acids are deleted in the amino acid sequence represented by SEQ ID NO: 1, (ii) an amino acid sequence wherein at least 1 or 2 (preferably approximately 1 to 30, more preferably approximately 1 to 10, and much more preferably approximately several (1 or 2)) amino acids are added to the amino acid sequence represented by SEQ ID NO: 1, (iii) an amino acid sequence wherein at least 1 or 2 (preferably approximately 1 to 30, more preferably approximately 1 to 10, and much more preferably approximately several (1 or 2)) amino acids in the amino acid sequence represented by SEQ ID NO: 1 are substituted with other amino acids, or (iv) an amino acid sequence in combination thereof; or the like.

The amino acid sequence which is substantially the same amino acid sequence as the amino acid sequence represented by SEQ ID NO: 44 includes, e.g., an amino acid sequence having at least about 97% homology, preferably at least about 98% homology, more preferably at least about 99% homology and most preferably about 99.5% homology, to the amino acid sequence represented by SEQ ID NO: 44, and the like.

As the protein having the same or substantially the same amino acid sequence as the amino acid sequence represented by SEQ ID NO: 44, preferred are, for example, proteins containing the same or substantially the same amino acid sequence as the amino acid sequence represented by SEQ ID NO: 44 and having an activity substantially equivalent to that of the protein containing substantially the same amino acid sequence as the amino acid sequence represented by SEQ ID NO: 44, and the like.

The amino acid sequence which is substantially the same amino acid sequence as the amino acid sequence represented by SEQ ID NO: 51 includes, e.g., an amino acid sequence having at least about 95% homology, preferably at least about 96% homology, more preferably at least about 97% homology, and most preferably at least about 98% homology, to the amino acid sequence represented by SEQ ID NO: 51, and the like.

As the protein having substantially the same amino acid sequence as the amino acid sequence represented by SEQ ID NO: 51, preferred are, for example, proteins containing substantially the same amino acid sequence as the amino acid sequence represented by SEQ ID NO: 51 and having an activity substantially equivalent to that of the protein containing substantially the same amino acid sequence as the amino acid sequence represented by SEQ ID NO: 51, and the like.

As the protein having the same or substantially the same amino acid sequence as the amino acid sequence represented by SEQ ID NO: 51, preferred are, for example, proteins containing the same or substantially the same amino acid sequence as the amino acid sequence represented by SEQ ID NO: 51 and having an activity substantially equivalent to that of the amino acid sequence represented by SEQ ID NO: 51, and the like.

The amino acid sequence which is substantially the same amino acid sequence as the amino acid sequence represented by SEQ ID NO: 52 includes, e.g., an amino acid sequence having at least about 90% homology, preferably at least about 95% homology and more preferably at least about 98% homology, to the amino acid sequence represented by SEQ ID NO: 52, and the like.

As the protein having substantially the same amino acid sequence as the amino acid sequence represented by SEQ ID NO: 52, preferred are, for example, proteins containing substantially the same amino acid sequence as the amino acid sequence represented by SEQ ID NO: 52 and having an activity substantially equivalent to that of the protein containing substantially the same amino acid sequence as the amino acid sequence represented by SEQ ID NO: 52 and the like.

As the protein having the same or substantially the same amino acid sequence as the amino acid sequence represented by SEQ ID NO: 52, preferred are, for example, proteins containing the same or substantially the same amino acid sequence as the amino acid sequence represented by SEQ ID NO: 52 and having an activity substantially equivalent to that of the amino acid sequence represented by SEQ ID NO: 52, and the like. Specifically, there are proteins described in WO 98/46620, and the like.

The amino acid sequence which is substantially the same amino acid sequence as the amino acid sequence represented by SEQ ID NO: 53 includes, e.g., an amino acid sequence having at least about 95% homology, preferably at least about 96% homology, more preferably at least about 97% and most preferably at least about 98% homology, to the amino acid sequence represented by SEQ ID NO: 53, and the like.

As the protein having substantially the same amino acid sequence as the amino acid sequence represented by SEQ ID NO: 53, preferred are, for example, proteins containing substantially the same amino acid sequence as the amino acid sequence represented by SEQ ID NO: 53 and having an activity substantially equivalent to that of the protein containing substantially the same amino acid sequence as the amino acid sequence represented by SEQ ID NO: 53, and the like.

As the protein having the same or substantially the same amino acid sequence as the amino acid sequence represented by SEQ ID NO: 53, preferred are, for example, proteins containing the same or substantially the same amino acid sequence as the amino acid sequence represented by SEQ ID NO: 53 and having an activity substantially equivalent to that of the amino acid sequence represented by SEQ ID NO: 53, and the like. Specifically, there are proteins described in Biochem. Biophys. Acta, 1491, 369-375, 2000, and the like.

The amino acid sequence which is substantially the same amino acid sequence as the amino acid sequence represented by SEQ ID NO: 54 includes, e.g., an amino acid sequence having at least about 95% homology, preferably at least about 96% homology, more preferably at least about 97% and most preferably at least about 98% homology, to the amino acid sequence represented by SEQ ID NO: 54, and the like.

As the protein having substantially the same amino acid sequence as the amino acid sequence represented by SEQ ID NO: 54, preferred are, for example, proteins containing substantially the same amino acid sequence as the amino acid sequence represented by SEQ ID NO: 54 and having an activity substantially equivalent to that of the protein containing substantially the same amino acid sequence as the amino acid sequence represented by SEQ ID NO: 54, and the like.

As the protein having the same or substantially the same amino acid sequence as the amino acid sequence represented by SEQ ID NO: 54, preferred are, for example, proteins containing the same or substantially the same amino acid sequence as the amino acid sequence represented by SEQ ID NO: 54 and having an activity substantially equivalent to that of the amino acid sequence represented by SEQ ID NO: 54, and the like. Specifically, there are proteins described in WO 98/46620, and the like.

The substantially equivalent activity refers to, for example, a binding activity to the peptide of the invention, a signal transduction activity, etc. The term substantially equivalent is used to mean that these activities are equivalent in nature. It is thus preferred that these activities such as a binding activity to the peptide of the invention, a signal transduction activity, etc. are equivalent in potency (e.g., about 0.5 to about 2 times), and it is allowable that even differences among grades such as the strength of these activities and molecular weight of the protein are present.

These activities such as a binding activity, a signal transduction activity, etc. can be assayed by publicly known methods with a modification.

As the protein of the invention, there are also employed proteins containing (i) an amino acid sequence wherein at least 1 or 2 (preferably approximately 1 to 30, more preferably approximately 1 to 10 and most preferably several (1 or 2)) amino acids are deleted in the amino acid sequence represented by SEQ ID NO: 44, SEQ ID NO: 51, SEQ ID NO: 52, SEQ ID NO: 53 or SEQ ID NO: 54, (ii) an amino acid sequence wherein at least 1 or 2 (preferably approximately 1 to 30, more preferably approximately 1 to 10 and most preferably approximately several (1 or 2)) amino acids are added to the amino acid sequence represented by SEQ ID NO: 44, SEQ ID NO: 51, SEQ ID NO: 52, SEQ ID NO: 53 or SEQ ID NO: 54, (iii) an amino acid sequence wherein at least 1 or 2 (preferably approximately 1 to 30, more preferably approximately 1 to 10 and most preferably approximately several (1 or 2)) amino acids in the amino acid sequence represented by SEQ ID NO: 44, SEQ ID NO: 51, SEQ ID NO: 52, SEQ ID NO: 53 or SEQ ID NO: 54 are substituted with other amino acids, or (iv) an amino acid sequence in combination thereof; or the like.

Specific examples of the protein of the invention include human-derived proteins containing the amino acid sequence represented by SEQ ID NO: 1 or SEQ ID NO: 52, rat-derived proteins containing the amino acid sequence represented by SEQ ID NO: 44 or SEQ ID NO: 51, mouse-derived proteins containing the amino acid sequence represented by SEQ ID NO: 53 or SEQ ID NO: 54, and the like.

Throughout the present specification, the peptides and proteins are represented in accordance with the conventional way of describing peptides, that is, the N-terminus (amino terminus) at the left hand and the C-terminus (carboxyl terminus) at the right hand. In the proteins of the invention including the protein containing the amino acid sequence represented by SEQ ID NO:47, the C-terminus may be in the form of a carboxyl group (-COOH), a carboxylate ($-COO^-$), an amide ($-CONH_2$) or an ester (-COOR).

Herein, examples of the ester group represented by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such

as phenyl, α-naphthyl, etc.; a $C_{7-14}$ aralkyl such as a phenyl-$C_{1-2}$ alkyl group, e.g., benzyl, phenethyl, etc.; an α-naphthyl-$C_{1-2}$ alkyl group such as α-naphthylmethyl, etc.; and the like. In addition, pivaloyloxymethyl or the like, which is used widely as an ester for oral administration, may also be used.

Where the peptide of the invention and the protein of the invention (the peptide/protein of the invention) contain a carboxyl group (or a carboxylate) at a position other than the C-terminus, it may be amidated or esterified and such an amide or ester is also included within the peptide/protein of the invention. The ester group in this case may be the same ester group as that described with respect to the above C-terminal group; etc.

Furthermore, examples of the peptide/protein of the invention include variants of the peptide/protein of the invention described above, wherein the amino group at the N-terminal methionine residue is protected with a protecting group (e.g., a $C_{1-6}$ acyl group such as a $C_{1-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{2-6}$ alkanoyl group, e.g., formyl group, acetyl group, etc.), conjugated peptides/proteins such as glycopeptide/glycoproteins having sugar chains; and the like.

Specific examples of the peptide of the invention include a human-derived peptide containing the amino acid sequence represented by SEQ ID NO: 19, a rat-derived or mouse-derived peptide containing the amino acid sequence represented by SEQ ID NO: 39, etc.

Specific examples of the protein of the invention include a human-derived (preferably human brain-derived) protein containing the amino acid sequence represented by SEQ ID NO: 1 or SEQ ID NO: 52, a rat-derived protein containing the amino acid sequence represented by SEQ ID NO: 44 or SEQ ID NO: 51, or a mouse-derived peptide containing the amino acid sequence represented by SEQ ID NO: 53 or SEQ ID NO: 54, etc.

The partial peptides of the protein of the invention (hereinafter sometimes merely referred to as "the partial peptide of the invention") may be any partial peptides of the protein of the invention; in the protein molecules of the invention, there may be employed, e.g., the site exposed outside cell membranes and having a substantially equivalent ligand binding activity, and the like are employed.

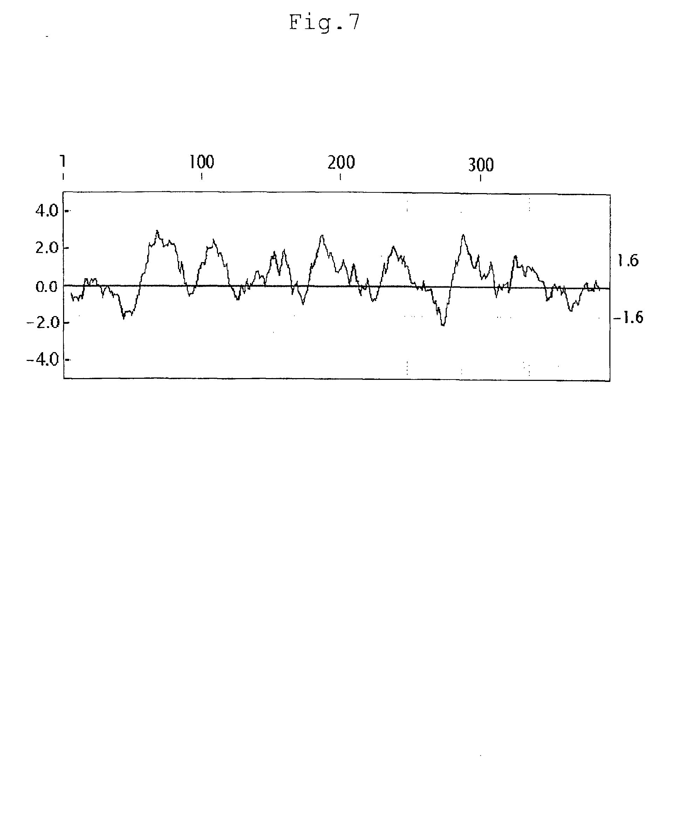

Specific examples of the partial peptides of the protein having the amino acid sequence represented by SEQ ID NO: 1 are peptides containing the part which has been analyzed to be an extracellular domain (hydrophilic domain) in the hydrophobic plotting analysis shown in FIG. 7. A peptide containing a hydrophobic domain part can be used as well. In addition, a peptide containing each domain separately as well as a peptide containing plural domains together may also be employed.

In the partial peptides of the invention, the number of amino acids is at least 20, preferably at least 50 and more preferably at least 100, in the amino acid sequence, which constitutes the protein of the invention.

The term substantially the same amino acid sequence is used to mean an amino acid sequence having at least about 50% homology, preferably at least about 70% homology, more preferably at least about 80% homology, much more preferably at least about 90% homology, and most preferably at least about 95% homology, to these amino acid sequences.

Herein, the term "substantially equivalent ligand binding activity" is intended to mean the same significance as defined above. The "substantially equivalent ligand binding activity" can be assayed by publicly known methods with a modification.

The partial peptides of the invention may be those wherein at least 1 or 2 (preferably approximately 1 to 10, more preferably several (1 or 2)) amino acids are deleted in the amino acid sequence represented by SEQ ID NO: 1, SEQ ID NO: 44, SEQ ID NO: 51, SEQ ID NO: 52, SEQ ID NO: 53 or SEQ ID NO: 54; those wherein at least 1 or 2 (preferably approximately 1 to 20, more preferably approximately 1 to 10 and most preferably several (1 or 2)) amino acids are added to the amino acid sequence; or those wherein at least 1 or 2 (preferably approximately 1 to 10, more preferably approximately 1 to 5 and most preferably several (1 or 2)) amino acids are substituted with other amino acids.

In the partial peptide of the invention, the C-terminal may be a carboxyl group (-COOH), a carboxylate ($-COO^-$), an amide ($-CONH_2$) or an ester (-COOR). Herein, R in the ester has the same significance as defined above. When the partial peptide of the invention contains a carboxyl group (or a carboxylate) at the site other than the C-terminus, the carboxyl group may be amidated or esterified and those amides or esters are also included in the partial peptide of the invention. As the esters in this case, for example, the C-terminal esters described above may be employed.

As in the protein of the invention described above, the partial peptide of the invention further includes those in which the amino group of the N-terminal methionine residue is protected by a protecting group, those in which the N-terminal residue is cleaved in vivo and the produced Gln is pyroglutamated, those in which substituents on the side chains of amino acids in the molecule are protected by appropriate protecting groups, conjugated peptides such as those, to which sugar chains are coupled, i.e., so-called glycopeptides, and the like.

As the salts of the peptide of the invention, the protein of the invention or its partial peptides, particularly preferred are physiologically acceptable acid addition salts. Examples of such salts employed are salts with, for example, inorganic acids (e.g., hydrochloric acid, phosphoric acid, hydrobromic acid or sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid or benzenesulfonic acid), and the like.

The peptide of the invention or the protein of the invention, or salts thereof may be manufactured by publicly known methods used to purify peptides/proteins from the human or non-human mammal cells or tissues described above, or may also be manufactured by culturing transformants containing DNAs encoding, e.g., the peptide of the invention later described or the protein of the invention having the amino acid sequence represented by SEQ ID NO: 1, SEQ ID NO: 44, SEQ ID NO: 51 or SEQ ID NO: 52. Alternatively, the peptide/protein of the invention may also be manufactured by the peptide/protein synthesis methods later described or by its modification. Furthermore, the protein or its salt containing the amino acid sequence represented by SEQ ID NO: 53 may be manufactured by a modification of the method described in Biochem. Biophys. Acta, 1491, 369-375, 2000. The protein or its salt containing the amino acid sequence represented by SEQ ID NO: 52 or SEQ ID NO: 54 may be manufactured by a modification of the method described in WO 98/46620.

Where the peptide/protein is manufactured from human or non-human mammal tissues or cells, the human or non-human mammal tissues or cells are homogenized, then the peptide/protein are extracted with an acid, etc., isolated and purified from the extract obtained by a combination of chromatography techniques such as reversed phase chromatography, ion exchange chromatography, and the like.

To synthesize the peptide of the invention or the protein of the invention or its partial peptides, or amides or salts thereof, commercially available resins that are normally used for the peptide/protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids in which α-amino groups and functional groups on the side chains are appropriately protected are condensed on the resin in the order of the sequence of the objective peptide/protein according to various condensation methods publicly known. At the end of the reaction, the peptide/protein is excised from the resin and at the same time, the protecting groups are removed. Then, intramolecular disulfide bond-forming reaction is performed in a highly diluted solution to obtain the objective peptide/protein or amides thereof.

For condensation of the protected amino acids described above, a variety of activation reagents available for the peptide/protein synthesis may be used, and carbodiimides are particularly preferably employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents used to activate the protected amino acids or condense with the resin may be chosen from solvents that are known to be usable for peptide/protein condensation reactions. For example, there may be employed acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxan, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the starting amino groups include Z, Boc, t-pentyloxycarbonyl, isobomyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower alkanoyl group, such as acetyl group, an aroyl group such as benzoyl group, and a group derived from carbonic acid such as benzyloxycarbonyl group and ethoxycarbonyl group. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, tertiary butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting amino acids include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the activated amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia, etc.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the peptide/protein, for example, the α-carboxyl group of the carboxy terminal amino acid is first protected by amidation; the peptide chain is then extended to amino group for a desired length. Thereafter, a peptide/protein in which only the protecting group of the N-terminal α-amino group has been eliminated from the peptide chain and a peptide/protein in which only the protecting group of the C-terminal carboxyl group has been eliminated are manufactured. The two peptides/proteins are condensed in a mixture of the solvents described above. The details of the condensation reaction are the same as described above. After the protected peptide/protein obtained by the condensation is purified, all the protecting groups are eliminated by the method described above to give the desired crude peptide/crude protein. This crude peptide/crude protein is purified by various known purification means. Lyophilization of the major fraction gives the amide of the desired peptide/protein.

To prepare the esterified peptide/protein, for example, the α-carboxyl group of the carboxy terminal amino acid is condensed with a desired alcohol to prepare the amino acid ester, which is followed by procedure similar to the preparation of the amidated peptide/protein above to give the desired esterified peptide/protein.

The peptide of the invention and the protein of the invention can be manufactured by publicly known methods for peptide synthesis. Also, the particle peptides of the protein of the invention or salts thereof can be manufactured by publicly known methods for peptide synthesis or by cleaving the protein of the invention with an appropriate peptidase.

For the methods of peptide synthesis, for example, either solid phase synthesis or liquid phase synthesis may be used. That is, the partial peptide or amino acids that can constitute the peptide of the invention or the protein of the invention can be condensed with the remaining part of the partial peptide of the invention. Where the product contains protecting groups, these protecting groups are removed to give the desired peptide. Publicly known methods for condensation and elimination of the protecting groups, are described in 1) to 5) below.

1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: *Peptide Gosei-no-Kiso to Jikken* (Basics and experiments of peptide synthesis), published by Maruzen Co. (1975)
4) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
5) Haruaki Yajima ed.: *Zoku Iyakuhin no Kaihatsu* (A sequel to Development of Pharmaceuticals), Vol. 14, Peptide Synthesis, published by Hirokawa Shoten

After completion of the reaction, the peptide of the invention or the partial peptide of the invention can be purified and isolated by a combination of conventional purification methods such as solvent extraction, distillation, column chromatography, liquid chromatography and recrystallization. When the peptide or partial peptide obtained by the above methods is in a free form, the peptide or partial peptide can be converted into an appropriate salt by a publicly known method; when the peptide or partial peptide is obtained in a salt form, it can be converted into a free form by a publicly known method.

The polynucleotide encoding the peptide of the invention or the protein of the invention may be any polynucleotide, so long as it contains the base sequence encoding the peptide of the invention or the protein of the invention described above, and preferably the polynucleotide is a DNA. Such a DNA may be any one of genomic DNA, genomic DNA library, cDNA derived from the cells or tissues described above, cDNA library derived from the cells or tissues described above and synthetic DNA. The vector to be used for the library may be any of bacteriophage, plasmid, cosmid, phagemid, and the like. In addition, the DNA can be amplified by reverse transcriptase polymerase chain reaction (hereinafter abbreviated as RT-PCR) with total RNA or mRNA fraction prepared from the cells or tissues described above.

Specifically, the DNA encoding the peptide containing the amino acid sequence represented by SEQ ID NO: 19 may be any one of, for example, a DNA containing the base sequence represented by SEQ ID NO: 20, or a DNA having a base sequence hybridizable to the base sequence represented by SEQ ID NO: 20 under high stringent conditions and encoding a peptide which has an activity substantially equivalent to the activity of the peptide of the invention (e.g., a binding activity to the protein of the invention, a signal transduction activity mediated by the protein of the invention, etc.).

The DNA containing the base sequence represented by SEQ ID NO: 20 includes a DNA containing the base sequence represented by SEQ ID NO: 20 or SEQ ID NO: 18, and the like.

Examples of the DNA that is hybridizable to the base sequence represented by SEQ ID NO: 20 under high stringent conditions include a DNA containing the base sequence having at least about 95% homology, preferably at least about 97% homology and more preferably at least about 99% homology, to the base sequence represented by SEQ ID NO: 20.

Specifically, the DNA encoding the peptide containing the amino acid sequence represented by SEQ ID NO: 39 may be any DNA, so long as it is a DNA containing the base sequence represented by SEQ ID NO: 40 or SEQ ID NO: 57, or a DNA having a DNA hybridizable to a DNA containing the base sequence represented by SEQ ID NO: 40 or SEQ ID NO: 57 under high stringent conditions and encoding a peptide which has an activity substantially equivalent to the activity of the peptide of the invention (a binding activity to the protein of the invention, a signal transduction activity mediated by the protein of the invention, etc.).

Examples of the DNA containing the base sequence represented by SEQ ID NO: 40 or SEQ ID NO: 57 include DNAs containing the base sequence represented by SEQ ID NO: 38, SEQ ID NO: 40, SEQ ID NO: 56 or SEQ ID NO: 57, and the like.

As the DNA hybridizable to a DNA containing the base sequence represented by SEQ ID NO: 40 or SEQ ID NO: 57 under high stringent conditions, there may be employed a DNA containing the base sequence having at least about 95% homology, preferably at least about 97% homology and more preferably at least about 99% homology, to the base sequence represented by SEQ ID NO: 40 or SEQ ID NO: 57, and the like.

The DNA encoding the protein containing the amino acid sequence represented by SEQ ID NO: 1 may be any DNA, so long as it is, e.g., a DNA containing the base sequence represented by SEQ ID NO: 2 or SEQ ID NO: 3, or a DNA having a DNA hybridizable to a DNA containing the base sequence represented by SEQ ID NO: 2 or SEQ ID NO: 3 under high stringent conditions and encoding a protein which has an activity substantially equivalent to the activity of the protein containing the amino acid sequence represented by SEQ ID NO: 1 (a ligand binding activity, a signal transduction activity, etc.).

As the DNA hybridizable to a DNA containing the base sequence represented by SEQ ID NO: 2 or SEQ ID NO: 3 under high stringent conditions, there may be employed a DNA containing the base sequence having at least about 90% homology, preferably at least about 95% homology and more preferably at least about 98% homology, to the base sequence represented by SEQ ID NO: 2 or SEQ ID NO: 3, and the like.

The DNA encoding the protein containing the amino acid sequence represented by SEQ ID NO: 44 may be any DNA, so long as it is, e.g., a DNA containing the base sequence represented by SEQ ID NO: 43, or a DNA having a DNA hybridizable to a DNA containing the base sequence represented by SEQ ID NO: 43 under high stringent conditions and encoding a protein which has an activity substantially equivalent to the activity of the protein of the invention (a binding activity to the peptide of the invention, a signal transduction activity, etc.).

As the DNA hybridizable to a DNA containing the base sequence represented by SEQ ID NO: 43 under high stringent conditions, there may be employed a DNA containing the base sequence having at least about 90% homology, preferably at least about 95% homology and more preferably at least about 98% homology, to the base sequence represented by SEQ ID NO: 43, and the like.

The DNA encoding the protein containing the amino acid sequence represented by SEQ ID NO: 51 may be any DNA, so long as it is, e.g., a DNA containing the base sequence represented by SEQ ID NO: 50, or a DNA having a DNA hybridizable to a DNA containing the base sequence represented by SEQ ID NO: 50 under high stringent conditions and encoding a protein which has an activity substantially equivalent to the activity of the protein of the invention (a binding activity to the peptide of the invention, a signal transduction activity, etc.).

As the DNA hybridizable to a DNA containing the base sequence represented by SEQ ID NO: 50 under high stringent conditions, there may be employed a DNA containing the base sequence having at least about 90% homology, preferably at least about 95% homology and more preferably at least about 98% homology, to the base sequence represented by SEQ ID NO: 50, and the like.

The DNA encoding the protein containing the amino acid sequence represented by SEQ ID NO: 52 may be any DNA, so long as it is, e.g., a DNA containing the base sequence represented by SEQ ID NO: 6, or a DNA having a DNA hybridizable to a DNA containing the base sequence represented by SEQ ID NO: 6 under high stringent conditions and encoding a protein which has an activity substantially equivalent to the activity of the protein containing the amino acid sequence represented by SEQ ID NO: 6 (a binding activity to the peptide of the invention, a signal transduction activity, etc.).

As the DNA hybridizable to a DNA containing the base sequence represented by SEQ ID NO: 6 under high stringent conditions, there may be employed a DNA containing the base sequence having at least about 90% homology, preferably at least about 95% homology and more preferably at least about 98% homology, to the base sequence represented by SEQ ID NO: 6, and the like.

The DNA encoding the protein containing the amino acid sequence represented by SEQ ID NO: 53 may be any DNA, so long as it is, e.g., a DNA containing the base sequence represented by SEQ ID NO: 63, or a DNA having a DNA hybridizable to a DNA containing the base sequence represented by SEQ ID NO: 63 under high stringent conditions and encoding a protein which has an activity substantially equivalent to the activity of the protein containing the amino acid sequence represented by SEQ ID NO: 63 (a binding activity to the peptide of the invention, a signal transduction activity, etc.).

As the DNA hybridizable to a DNA containing the base sequence represented by SEQ ID NO: 63 under high stringent conditions, there may be employed a DNA containing the base sequence having at least about 95% homology, preferably at least about 97% homology and more preferably at least about 98% homology, to the base sequence represented by SEQ ID NO: 63, and the like.

The DNA encoding the protein containing the amino acid sequence represented by SEQ ID NO: 54 may be any DNA, so long as it is, e.g., a DNA containing the base sequence represented by SEQ ID NO: 64, or a DNA having a DNA hybridizable to a DNA containing the base sequence represented by SEQ ID NO: 64 under high stringent conditions and encoding a protein which has an activity substantially equivalent to the activity of the protein containing the amino acid sequence represented by SEQ ID NO: 64 (a binding activity to the peptide of the invention, a signal transduction activity, etc.).

As the DNA hybridizable to a DNA containing the base sequence represented by SEQ ID NO: 64 under high stringent conditions, there may be employed a DNA containing the base sequence having at least about 95% homology, preferably at least about 97% homology and more preferably at least about 98% homology, to the base sequence represented by SEQ ID NO: 64, and the like.

The hybridization can be carried out by publicly known methods or by a modification thereof, for example, according to the method described in Molecular Cloning, 2nd. (J. Sambrook et al., Cold Spring Harbor Lab. Press, 1989), etc. When a commercially available library is used, hybridization may be carried out according to the instructions of the attached manufacturer's protocol. The hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.

More specifically, the DNA encoding the peptide containing the amino acid sequence represented by SEQ ID NO: 19 includes a DNA containing the base sequence represented by SEQ ID NO:20; the DNA encoding the peptide containing the amino acid sequence represented by SEQ ID NO: 17 includes a DNA containing the base sequence represented by SEQ ID NO: 18; the DNA encoding the peptide containing the amino acid sequence represented by SEQ ID NO: 39 includes a DNA containing the base sequence represented by SEQ ID NO: 40 or SEQ ID NO:57; the DNA encoding the peptide containing the amino acid sequence represented by SEQ ID NO: 37 includes a DNA containing the base sequence represented by SEQ ID NO: 38; and, the DNA encoding the peptide containing the amino acid sequence represented by SEQ ID NO: 55 includes a DNA containing the base sequence represented by SEQ ID NO: 56.

Also, the DNA encoding the protein containing the amino acid sequence represented by SEQ ID NO: 1 includes a DNA containing the base sequence represented by SEQ ID NO: 2 or SEQ ID NO: 3; the DNA encoding the protein containing the amino acid sequence represented by SEQ ID NO: 44 includes a DNA containing the base sequence represented by SEQ ID NO: 43; the DNA encoding the protein containing the amino acid sequence represented by SEQ ID NO: 51 includes a DNA containing the base sequence represented by SEQ ID NO: 50; the DNA encoding the protein containing the amino acid sequence represented by SEQ ID NO: 52 includes a DNA containing the base sequence represented by SEQ ID NO: 6; the DNA encoding the protein containing the amino acid sequence represented by SEQ ID NO: 53 includes a DNA containing the base sequence represented by SEQ ID NO: 63; and the DNA encoding the protein containing the amino acid sequence represented by SEQ ID NO: 54 includes a DNA containing the base sequence represented by SEQ ID NO: 64.

The nucleotide (oligonucleotide) comprising a base sequence complementary to the base sequence of a DNA encoding the peptide of the invention or a part thereof is intended to include not only a DNA encoding the peptide of the invention but also an RNA.

According to the present invention, antisense (oligo)nucleotides (nucleic acids) that can inhibit replication or expression of the gene of the peptide of the invention can be designed and synthesized, on the basis of base sequence information of a DNA encoding the cloned or sequenced peptide. Such (oligo)nucleotides (nucleic acids) can hybridize to RNA of the gene of the peptide of the invention and inhibit the synthesis or function of the RNA, or can regulate/control the expression of the gene of the peptide of the invention via interaction with RNAs associated with the peptide of the invention. (Oligo)nucleotides complementary to the specified sequences of RNAs associated with the peptide of the invention and (oligo)nucleotides that can specifically hybridize to RNAs associated with the peptide of the invention are useful for regulating/controlling expression of the gene of the peptide of the invention in vivo and in vitro, and are also useful for the treatment or diagnosis of diseases.

The term "correspond" is used to mean homologous or complementary to a specific sequence of nucleotides including genes, base sequences or nucleic acids. As between nucleotides, base sequences or nucleic acids and peptides, the term "corresponding" usually refers to amino acids of a peptide that is instructed to be derived from the sequence of nucleotides (nucleic acids) or its complements. The 5' end hairpin loop, 5' end 6-base-pair repeats, 5' end untranslated region, polypeptide translation initiation codon, protein coding region, ORF translation initiation codon, 3' untranslated region, 3' end palindrome region, and 3' end hairpin loop of the G protein-coupled receptor protein gene may be selected as preferred target regions, though any region may be a target within genes for the peptide of the invention.

The relationship between the targeted nucleic acids and the (oligo)nucleotides complementary to at least a portion of the target region, specifically the relationship between the target and the (oligo)nucleotides hybridizable to the target, is denoted to be in "an antisense". The antisense (oligo)nucleotides may be polydeoxynucleotides containing 2-deoxy-D-ribose, polydeoxynucleotides containing D-ribose, any other type of (oligo)nucleotides which are N-glycosides of a purine or pyrimidine base, other polymers containing non-nucleotide backbones (e.g., protein nucleic acids and synthetic sequence-specific nucleic acid polymers that are commercially available), other polymers containing nonstandard linkages (provided that the polymers contain nucleotides with such a configuration that allows base pairing or base stacking, as is found in DNAs or RNAs), etc. The antisense (oligo)nucleotides may be a double-stranded DNA, a single-stranded DNA, a double-stranded RNA, a single-stranded RNA and also a DNA:RNA hybrid, and further includes unmodified polynucleotides or unmodified oligonucleotides, those with publicly known types of modifications, for example, those with labels known in the art, those with caps, methylated (oligo)nucleotides, those with substitution of one or more naturally occurring nucleotides with their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (including nucleases, nuclease inhibitors, toxins, antibodies, signal peptides, poly-L-lysine, etc.), saccharides (e.g., monosaccharides, etc.), etc., those with intercalators (e.g., acridine, psoralen, etc.), those containing chelators (e.g., metals, radioactive metals, boron, oxidative metals, etc.), those containing alkylating agents, those with modified linkages (e.g., $\alpha$ anomeric nucleic acids, etc.). Herein, the terms "nucleoside", "nucleotide" and "nucleic acid" are used to refer to moieties that contain not only the purine and pyrimidine bases, but also other heterocyclic bases, which have been modified. Such modifications include methylated purines and pyrimidines, acylated purines and pyrimidines and other heterocyclic rings. Modified nucleotides and modified nucleotides also include modifications on the sugar moiety, for example, wherein one or more hydroxyl groups may optionally be replaced with a halogen, aliphatic groups, or may be converted into the corresponding functional groups such as ethers, amines, or the like.

The antisense nucleic acid of the invention is RNA, DNA or a modified nucleic acid. Specific examples of the modified nucleic acid are, but not limited to, sulfurized and thiophosphate derivatives of nucleic acids and those resistant to degradation of polynucleoside amides or oligonucleoside amides The antisense nucleic acids of the present invention can be modified preferably based on the following design, that is, by increasing the intracellular stability of the antisense nucleic acid, increasing the cellular permeability of the antisense nucleic acid, increasing the affinity of the nucleic acid to the target sense strand to a higher level, or minimizing the toxicity, if any, of the antisense nucleic acid.

Many such modifications are known in the art, as disclosed in J. Kawakami, et al., Pharm. Tech. Japan, Vol. 8, pp. 247, 1992; Vol. 8, pp. 395, 1992; S. T. Crooke et al. ed., Antisense Research and Applications, CRC Press, 1993; etc.

The antisense nucleic acid of the invention may contain altered or modified sugars, bases or linkages. The antisense nucleic acid may also be provided in a specialized form such as liposomes, microspheres or may be applied to gene therapy or may be provided in combination with attached moieties. Such attached moieties include polycations such as polylysine that act as charge neutralizers of the phosphate backbone, or hydrophobic moieties such as lipids (e.g., phospholipids, cholesterols, etc.) that potentiate the interaction with cell membranes or increase uptake of the nucleic acid. Preferred examples of the lipids to be attached are cholesterols or derivatives thereof (e.g., cholesteryl chloroformate, cholic acid, etc.). These moieties may be attached at the 3' or 5' ends of the nucleic acid and may be also attached through a base, sugar, or intramolecular nucleoside linkage. Other moieties may be capping groups specifically placed at the 3' or 5' ends of the nucleic acid to prevent degradation by nuclease such as exonuclease, RNase, etc Such capping groups include, but are not limited to, hydroxyl protecting groups known in the art, including glycols such as polyethylene glycol, tetraethylene glycol, and the like.

The inhibitory activity of the antisense nucleic acid can be examined using the transformant of the present invention, the gene expression system of the present invention in vitro or in vivo, or the translation system of proteins in vitro and in vivo. The nucleic acid can be applied to cells by a variety of publicly known methods.

The DNA encoding the partial peptide of the invention may be any DNA, so long as it contains the base sequence encoding the partial peptide of the invention described above. The DNA may also be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid, phagemid and the like. In addition, the DNA can be directly amplified by reverse transcriptase polymerase chain reaction with the mRNA fraction prepared from the cells or tissues described above.

Specifically, as the DNA encoding the partial peptide of the invention there are employed, for example, a DNA that has a part of the base sequence of the DNA containing the base sequence represented by SEQ ID NO: 2, SEQ ID NO:3, SEQ ID NO:43, SEQ ID NO:50, SEQ ID NO:6, SEQ ID NO:63 or SEQ ID NO: 64, or (ii) a DNA having a base sequence hybridizable to the base sequence represented by SEQ ID NO: 2, SEQ ID NO:3, SEQ ID NO:43, SEQ ID NO:50, SEQ ID NO:6, SEQ ID NO:63 or SEQ ID NO: 64 under high stringent conditions and containing a part of the base sequence of DNA encoding a protein having substantially the same activity (e.g., a ligand binding activity, a signal transduction activity, etc.) as that of the protein of the invention.

Examples of the DNA that is hybridizable to the base sequence represented by SEQ ID NO: 2, SEQ ID NO: 3, SEQ ID NO:43, SEQ ID NO:50, SEQ ID NO:6, SEQ ID NO:63 or SEQ ID NO: 64 include a DNA containing the base sequence having at least about 90% homology, preferably at least about 95% homology, and more preferably at least about 98% homology, to the base sequence represented by SEQ ID NO: 2, SEQ ID NO:3, SEQ ID NO :43, SEQ ID NO:50, SEQ ID NO:6, SEQ ID NO:63 or SEQ ID NO: 64.

For cloning of the DNA that completely encodes the peptide of the invention, the DNA may be either amplified by PCR using synthetic DNA primers containing a part of the base sequence of the peptide of the invention, or the DNA inserted into an appropriate vector can be selected by hybridization with a labeled DNA fragment or synthetic DNA that encodes a part or the entire region of the peptide of the invention. The hybridization can be carried out, for example, according to the method described in Molecular Cloning, 2nd. (J. Sambrook et al., Cold Spring Harbor Lab. Press, 1989). Where commercially available library is used, the hybridization may also be performed in accordance with the protocol described in the attached instructions.

Cloning of the DNA that completely encodes the protein of the invention or its partial peptides (hereinafter merely referred to as the protein of the invention) may be carried out as in the cloning of the DNA that completely encodes the peptide of the invention.

Conversion of the DNA base sequence can be effected by PCR or publicly known methods such as the ODA-LA PCR method, the Gapped duplex method or the Kunkel method, or modifications thereof, by using publicly known kits available as Mutan™-super Express Km (TaKaRa Shuzo Co., Ltd.) or Mutan™-K (TaKaRa Shuzo Co., Ltd.), etc.

The cloned DNA encoding the peptide/protein can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the peptide/protein of the invention can be manufactured, for example, by (a) excising the desired DNA fragment from the DNA encoding the peptide/protein of the invention, (b) and then ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, pcDNA3.1, pRc/CMV2, pRc/RSV (Invitrogen, Inc.), etc.

The promoter used in the present invention may be any promoter if it matches well with a host to be used for gene expression. In the case of using animal cells as the host, examples of the promoter include SRα promoter, SV40 promoter, HIV-LTR promoter, CMV promoter, HSV-TK promoter, etc.

Among them, CMV promoter, SR$\alpha$ promoter or the like is preferably used. Where the host is bacteria of the genus Escherichia, preferred examples of the promoter include trp promoter, lac promoter, recA promoter, $\lambda P_L$ promoter, lpp promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin promoter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as $Neo^r$, G418 resistance), etc. In particular, when dhfr gene is used as the selection marker together with CHO ($dhfr^-$) cell, the objective gene may be selected also on thymidine free media.

If necessary, a signal sequence that matches with a host is added to the N-terminal side of the protein of the invention. Examples of the signal sequence that can be used are Pho A signal sequence, OmpA signal sequence, etc. in the case of using bacteria of the genus Escherichia as the host; $\alpha$-amylase signal sequence, subtilisin signal sequence, etc. in the case of using bacteria of the genus Bacillus as the host; MF$\alpha$ signal sequence, SUC2 signal sequence, etc. in the case of using yeast as the host; and insulin signal sequence, $\alpha$-interferon signal sequence, antibody molecule signal sequence, etc. in the case of using animal cells as the host, respectively.

Using the vector containing the DNA encoding the peptide/protein of the invention thus constructed, transformants can be manufactured.

Examples of the host, which may be employed, are bacteria belonging to the genus Escherichia, bacteria belonging to the genus Bacillus, yeast, insect cells, insects, animal cells, and the like.

Specific examples of the bacteria belonging to the genus Escherichia include *Escherichia coli* K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include *Bacillus subtilis* MI114 [Gene, 24, 255 (1983)], 207-21 [Journal of Biochemistry, 95, 87 (1984)], etc.

Examples of yeast include *Saccharomyces cereviseae* AH22, $AH22R^-$, NA87-11A, DKD-5D, 20B-12, *Schizosaccharomyces pombe* NCYC1913, NCYC2036, *Pichia pastoris,* etc.

Examples of insect cells include, for the virus AcNPV, *Spodoptera frugiperda* cell (Sf cell), MG1 cell derived from mid-intestine of *Trichoplusia ni,* High Five™ cell derived from egg of *Trichoplusia ni,* cells derived from *Mamestra brassicae,* cells derived from *Estigmena acrea,* etc.; and for the virus BmNPV, *Bombyx mori N* cell (BmN cell), etc. is used. Examples of the Sf cell which can be used are Sf9 cell (ATCC CRL1711) and Sf21 cell (both cells are described in Vaughn, J. L. et al., *In vivo,* 13, 213-217 (1977).

As the insect, for example, a larva of *Bombyx mori* can be used (Maeda et al., Nature, 315, 592 (1985)).

Examples of animal cells include monkey cell COS-7, Vero, Chinese hamster cell CHO (hereinafter referred to as CHO cell), dhfr gene deficient Chinese hamster cell CHO (hereinafter simply referred to as CHO($dhfr^-$) cell), mouse L cell, mouse AtT-20, mouse myeloma cell, rat GH 3, human FL cell, etc.

Bacteria belonging to the genus Escherichia can be transformed, for example, by the method described in Proc. Natl. Acad. Sci. U.S.A., 69, 2110 (1972), Gene, 17, 107 (1982), etc. Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in *Saibo Kogaku* (Cell Engineering), extra issue 8, *Shin Saibo Kogaku Jikken Protocol* (New Cell Engineering Experimental Protocol), 263-267 (1995), published by Shujunsha, or Virology, 52, 456 (1973).

Thus, the transformant transformed with the expression vector containing the DNA encoding the G protein-coupled protein can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, etc. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently. Where the bacteria belonging to the genus Escherichia are used as the host, the transformant is usually cultivated at approximately 15 to 43°C for about 3 hours to about 24 hours. If necessary, the culture may further be aerated or agitated.

Where the bacteria belonging to the genus Bacillus are used as the host, the transformant is cultivated generally at approximately 30 to 40°C for about 6 hours to about 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to about 8. In general, the transformant is cultivated at about 20°C to about 35°C for about 24 hours to about 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture may be aerated or agitated.

Where animal cells are employed as the host, the transformant is cultivated in, for example, MEM medium containing about 5% to about 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 hours to about 60 hours and, if necessary, the culture may be aerated or agitated.

As described above, the peptide/protein of the invention can be produced in the cell or cell membrane, or outside the cell, of the transformant.

The peptide/protein of the invention can be separated and purified from the culture described above, e.g., by the following procedures.

When the peptide/protein of the invention is extracted from the culture or cells, after cultivation, the transformants or cells are collected by a publicly known method and suspended in an appropriate buffer. The transformants or cells are then disrupted by publicly known methods such as ultrasonication, a treatment with lysozyme and/or freeze-thaw cycling, followed by centrifugation, filtration, etc. Thus, the crude extract of the peptide/protein of the invention can be obtained. The buffer used for the procedures may contain a protein denaturant such as urea or guanidine hydrochloride, or a surfactant such as Triton X-100™, etc. When the peptide/protein of the invention is secreted in the culture broth, after completion of the cultivation, the supernatant can be separated from the transformants or cells and collected by publicly known methods.

The peptide/protein of the invention contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the peptide/protein thus obtained is in a free form, it can be converted into the salt by publicly known methods or modifications thereof. On the other hand, when the peptide/protein is obtained in the form of a salt, it can be converted into the free form or in the form of a different salt by publicly known methods or modifications thereof.

The peptide/protein produced by the recombinant can be treated, prior to or after the purification, with an appropriate protein modifying enzyme so that the peptide/protein of the invention can be appropriately modified to partially remove a peptide/polypeptide. Examples of the protein-modifying enzyme include trypsin, chymotrypsin, arginyl endopeptidase, protein kinase, glycosidase and the like.

The activity of the thus produced peptide of the invention can be determined by a binding test to the protein of the invention, an enzyme immunoassay using a specific antibody, or the like.

Also, the activity of the protein of the invention can be determined by a binding test to the peptide of the invention, an enzyme immunoassay using a specific antibody, or the like.

Antibodies to the peptide of the invention, the protein of the invention or its partial peptides, or salts thereof may be any of polyclonal and monoclonal antibodies, so long as they can recognize the peptide of the invention, the protein of the invention or its partial peptides, or salts thereof.

The antibodies to the peptide of the invention, the protein of the invention or its partial peptides, or salts thereof (in the specification sometimes simply referred to as the peptide/protein, etc. of the invention) can be manufactured according to publicly known methods for producing antibodies or antisera, using the peptide/protein, etc. of the invention as antigens.

[Preparation of monoclonal antibody]

(a) Preparation of monoclonal antibody-producing cells

The peptide/protein, etc. of the invention is administered to mammals either solely or together with carriers or diluents to the site where the antibody can be produced by the administration. In order to potentiate the antibody productivity upon the administration, complete Freund's adjuvants or incomplete Freund's adjuvants may be administered. The administration is usually carried out once every 2 to 6 weeks approximately 2 to 10 times in total. Examples of the applicable mammals are monkeys, rabbits, dogs, guinea pigs, mice, rats, sheep and goats, with the use of mice and rats being preferred.

In the preparation of monoclonal antibody-producing cells, a warm-blooded animal such as mouse, immunized with an antigen is selected, then spleen or lymph nodes are collected after 2 to 5 days from the final immunization and antibody-producing cells contained therein are fused with myeloma cells to give monoclonal antibody-producing hybridomas. Measurement of the antibody titer in antisera may be carried out, for example, by reacting a labeled form of the peptide/protein, etc. of the invention described later with the antiserum followed by assaying the binding activity of the labeling agent bound to the antibody. The fusion operation may be carried out, for example, using the method known by Koehler and Milstein [Nature, 256, 495 (1975)]. Examples of the fusion promoter are polyethylene glycol (PEG), Sendai virus, etc., among which PEG is preferably employed.

Examples of the myeloma cells are NS-1, P3U1, SP2/0, etc. In particular, P3U1 is preferably employed. A preferred ratio of the count of the antibody-producing cells (spleen cells) used to the count of myeloma cells is within a range of approximately 1:1 to 20:1. When PEG (preferably, PEG 1000 to PEG 6000) is added in a concentration of approximately 10 to 80% followed by culturing at about 20 to 40°C, preferably at about 30 to 37°C for about 1 to 10 minutes, efficient cell fusion can be carried out.

Various methods can be used for screening of a monoclonal antibody-producing hybridoma. Examples of such methods include a method which comprises adding the hybridoma supernatant to a solid phase (e.g., a microplate) adsorbed with the peptide/protein, etc. of the invention as an antigen directly or together with a carrier, adding an anti-immunoglobulin antibody (where mouse cells are used for the cell fusion, anti-mouse immunoglobulin antibody is used) labeled with a radioactive substance or an enzyme, or protein A and detecting the monoclonal antibody bound to the solid phase; a method which comprises adding the hybridoma culture supernatant to a solid phase adsorbed with an anti-immunoglobulin antibody or protein A, adding the peptide/protein, etc. of the invention labeled with a radioactive substance or an enzyme and detecting the monoclonal antibody bound to the solid phase; and the like.

The monoclonal antibody can be selected in accordance with publicly known methods or modifications thereof. In general, the selection can be effected in a medium for animal cells supplemented with HAT (hypoxanthine, aminopterin and thymidine). Any selection and growth media can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.), etc. can be used for the selection and growth medium. The cultivation is carried out generally at 20 to 40°C, preferably at about 37°C. The time for cultivation is normally for 5 days to 3 weeks, preferably 1 to 2 weeks. The cultivation is carried out generally in 5% $CO_2$. The antibody titer of the hybridoma culture supernatant can be determined as in the assay for the antibody titer in antisera described above.

(b) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or the specific purification method which comprises collecting an antibody alone with an activated adsorbent such as an antigen-binding solid phase, protein A or protein G and dissociating the binding to obtain the antibody].

[Preparation of polyclonal antibody]

The polyclonal antibody of the invention can be manufactured by publicly known methods or modifications thereof. For example, a complex of immunogen (antigen of the peptide/protein, etc. of the invention) and a carrier protein is formed and a mammal is immunized with the complex in a manner similar to the method described above for the manufacture of monoclonal antibody. The product containing the antibody to the peptide/protein, etc. is collected from the immunized animal. By separating and purifying the antibody, the polyclonal antibody can be manufactured.

In the complex of immunogen and carrier protein for immunizing mammals, the type of carrier protein and the mixing ratio of carrier to hapten may be any type and in any ratio, as long as the antibody is efficiently produced to the hapten immunized by crosslinking to the carrier. For example, a method, in which bovine serum albumin, bovine thyroglobulin or keyhole limpet hemocyanin is coupled to hapten in a carrier-to-hapten weight ratio of approximately 0.1 to 20, preferably approximately 1 to 5, is employed.

A variety of condensation agents can be used for the coupling of carrier to hapten. Glutaraldehyde, carbodiimide, maleimide activated ester and activated ester reagents containing thiol group or dithiopyridyl group, and the like are used for the coupling.

The condensation product is administered to warm-blooded animals either solely or together with carriers or diluents to the site wherein the antibody can be produced. In order to potentiate the antibody productivity upon the administration, complete Freund's adjuvant or incomplete Freund's adjuvant may be administered. The administration is usually made once every about 2 to 6 weeks approximately 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as used for the determination of serum antibody titer described above. The separation and purification of the polyclonal antibody can be carried out, following the method for the separation and purification of immunoglobulins performed as in the separation and purification of monoclonal antibodies described above.

The peptide of the invention, the DNA encoding the peptide of the invention (hereinafter sometimes referred to as the DNA of the invention) and the antibody to the peptide of the invention (hereinafter sometimes referred to as the antibody of the invention) are useful (i) as therapeutic/preventive agents for various diseases, with which the peptide of the invention is associated; (ii) for screening a compound or its salt that alters the binding property between the peptide of the invention and the protein of the invention; (iii) for quantification of the peptide of the invention or a salt thereof; (iv) as a gene diagnostic agent; (v) as a pharmaceutical comprising the antisense DNA; (vi) as a pharmaceutical and diagnostic agent comprising the antibody of the invention; (vii) for preparation of non-human animals bearing the DNA of the invention; (viii) for drug design based on comparison with structurally similar ligand receptors; etc.

In particular, by applying the receptor binding assay system using the expression system of the recombinant protein of the invention, compounds (e.g., ZAQ agonists, ZAQ antagonists, etc.) that alter the binding property of human- or non-human mammal-specific ligands to the protein of the invention can be screened, and the agonists or antagonists can be used as agents for the prevention/treatment of various diseases.

Hereinafter, the peptide of the invention, the DNA of the invention and the antibody of the invention will be specifically described, with reference to their use.

(1) Agents for the treatment/prevention of various diseases with which the peptide of the invention is associated

As described in EXAMPLES below, the peptide of the invention was found to be a ligand to the protein of the invention (G protein-coupled receptor), since the peptide is present in vivo as a humoral factor, activates the protein of the invention and increases the intracellular Ca ion level in the cell wherein the protein of the invention is expressed.

Also, the peptide of the invention is found to have about 63% homology to snake venom Mamba Intestinal Toxin 1 (hereinafter abbreviated as MIT1; SEQ ID NO: 21; Toxicon, 28, 847-856, 1990; FEBS Letters, 461, 183-188, 1999) on an amino acid level. Moreover, human type Bv8 mature peptide (FEBS Letters, 462, 177-181, 1999) is found to have about 92.6% homology to mouse type Bv8 mature peptide (FEBS Letters, 462, 177-181, 1999) or rat type Bv8 mature peptide on an amino acid level.

It was reported that MIT1 induced contraction in the ileum or distal colon, or relaxation in the proximal colon and its degree was as potent as comparable to 40 mM potassium chloride (FEBS Letters, 461, 183-188, 1999). However, the site or mechanism of its action was not unraveled. The inventors clarified that the protein of the invention also mediated to express the action of MIT1.

Furthermore, the inventors confirmed that human type Bv8 mature peptide has an intestinal tract contractile activity, as described in EXAMPLES below.

Moreover, human type Bv8 mature peptide activates MAP kinase and PI-3 kinase and has a neuroprotecting action (European Journal or Neuroscience, 13, 1694-1702, 2001).

In view of the foregoing, the peptide of the invention has an activity of controlling contraction of the intestinal tracts, etc. Accordingly, when the DNA, etc. of the invention is deficient, or when its expression level is abnormally reduced, various diseases such as digestive diseases (e.g., enteritis, diarrhea, constipation, malabsorption syndrome, etc.), central nervous diseases (e.g., Alzheimer's disease, Perkinson's syndrome, schizophrenia, etc.) are developed, preferably digestive diseases.

When a patient has a reduced level of, or deficient of the peptide of the invention in his or her body so that signal transduction is not fully or normally exerted in the cell wherein the protein of the invention is expressed, the peptide of the invention can provide its role sufficiently or properly for the patient, (a) by administering the DNA of the invention to the patient to express the peptide of the invention in the body, (b) by inserting the DNA of the invention into a cell to express the peptide of the invention and then transplanting the cell to the patient, or (c) by administering the peptide of the invention to the patient, etc.

When the DNA of the invention is used as the agent for the prevention/treatment described above, the DNA may be administered alone to human or other warm-blooded animal; or the DNA is inserted into an appropriate vector such as retrovirus vector, adenovirus vector, adenovirus-associated virus vector, etc. and then administered to human or other warm-blooded animal in a conventional manner. The DNA of the invention may also be administered as an intact DNA; or the DNA may be prepared into a pharmaceutical composition together with physiologically acceptable carriers such as adjuvants, etc. to assist its uptake and the pharmaceutical preparation may be administered by gene gun or through a catheter such as a catheter with a hydrogel.

When the peptide of the invention is used as the agent for the treatment/prevention described above, it is advantageous to use the peptide in a purity of at least 90%, preferably at least 95%, more preferably at least 98% and most preferably at least 99%.

The peptide of the invention can be used orally, for example, in the form of tablets which, if necessary, may be sugar coated, capsules, elixirs, microcapsules etc., or parenterally in the form of injectable preparations such as a sterile solution or a suspension, etc. in water or in other pharmaceutically acceptable liquid. These preparations can be manufactured, for example, by mixing the peptide of the invention with a physiologically acceptable known carrier, a flavoring agent, an excipient, a vehicle, an antiseptic agent, a stabilizer, a binder, etc. in a unit dosage form required in a generally accepted fashion that is applied to making pharmaceutical preparations. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin, alginic acid, etc., a lubricant such as magnesium stearate, a sweetening agent such as sucrose, lactose and saccharin, and a flavoring agent such as peppermint, akamono oil or cherry, etc. When the unit dosage is in the form of capsules, liquid carriers such as oils and fats may further be used together with the additives described above. A sterile composition for injection may be formulated according to a conventional manner used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol, etc.), a polyalcohol (e.g., propylene glycol, polyethylene glycol, etc.), a nonionic surfactant (e.g., polysorbate 80™, HCO-50, etc.), or the like. Examples of the oily medium include sesame oil, soybean oil, etc., which may also be used in combination with a dissolution aid such as benzyl benzoate, benzyl alcohol, etc. The oily medium may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

The vector in which the DNA of the invention is inserted may also be prepared into pharmaceutical preparations in a manner similar to the procedures above. Such preparations are generally used parenterally.

Since the thus obtained pharmaceutical preparation is safe and low toxic, the preparation can be administered to mammalian animal (e.g., human, rat, mouse, guinea pig, rabbit, sheep, swine, bovine, horse, cat, dog, monkey, etc.).

The dose of the peptide of the invention varies depending on target disease, subject to be administered, route for administration, etc.; for example, in oral administration of the peptide of the invention for the treatment of digestive diseases, the dose is normally about 1 mg to about 1000 mg, preferably about 10 to about 500 mg, and more preferably about 10 to about 200 mg per day for adult (as 60 kg body weight). In parenteral administration, a single dose varies depending on subject to be administered, target disease, etc. but it is advantageous for the treatment of digestive diseases to inject the active ingredient into the affected area at a daily dose of about 1 to about 1000 mg, preferably about 1 to about 200 mg, and more preferably about 10 to about 100 mg for adult (as 60 kg body weight). For other animal species, the corresponding dose as converted per 60 kg body weight can be administered.

(2) Screening of a compound or its salt that alters the binding property between the peptide of the invention and the protein of the invention

The method of screening a compound or its salt that alters the binding property between the peptide of the invention and the protein of the invention, which comprises using the peptide of the invention and the protein of the invention (including partial peptides of the protein of the invention), or the kit for screening a compound or its salt that alters the binding property between the peptide of the invention and the protein of the invention, which comprises using the peptide of the invention and the protein of the invention (hereinafter merely referred to as the screening method of the invention, or the screening kit of the invention) is described below in detail.
By using the protein of the invention, or by constructing the expression system of a recombinant form of the protein of the invention and using the binding assay system to the peptide of the invention through the expression system (ligand-receptor assay system), the compound or its salt that alters the binding property between the peptide of the invention and the protein of the invention (e.g., peptide, protein, a non-peptide compound, a synthetic compound, fermentation product, etc.) can be screened.
Such a compound includes a compound (ZAQ agonist) having the cell stimulating activity mediated by the protein of the invention (e.g., the activity that promotes or inhibits arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.), a compound having no cell stimulating activity (ZAQ antagonist), and the like. The term "alters the binding property between the peptide of the invention and the protein of the invention" is used to include both cases where binding of the peptide of the invention to the protein of the invention is inhibited and promoted.
Thus, the present invention provides the method of screening a compound or its salt that alters the binding property of the peptide of the invention to the peptide of the invention, which comprises comparing (i) the case wherein the peptide of the invention is brought in contact with the protein of the invention and (ii) the case wherein the peptide of the invention and a test compound are brought in contact with the protein of the invention.
According to the screening method of the invention, the method comprises assaying, for example, the binding amount of the peptide of the invention to the protein of the invention, the cell stimulating activity, or the like, (i) when the peptide of the invention is brought in contact with the protein of the invention described above and (ii) when the peptide of the invention and a test compound are brought in contact with the protein of the invention described above, and comparing (i) and (ii).
Specific examples of the screening method of the invention include:

(a) A method of screening a compound or its salt that alters the binding property between the peptide of the invention and the protein of the invention, which comprises measuring the binding amounts of the peptide of the invention to the protein of the invention when the peptide of the invention is brought in contact with the protein of the invention and when the peptide of the invention and a test compound are brought in contact with the protein of the invention; and comparing the binding amounts;

(b) A method of screening a compound or its salt that alters the binding property between the peptide of the invention and the protein of the invention, which comprises, when the peptide of the invention is brought in contact with a cell or its cell membrane fraction containing the protein of the invention and when the peptide of the invention and a test compound are brought in contact with a cell or its cell membrane fraction containing the protein of the invention, assaying binding amounts of the peptide of the invention to the cell or the membrane fraction; and comparing the binding amounts; and,

(c) The screening method according to (b) described above, wherein the peptide of the invention is the protein of the invention expressed on the cell membrane by culturing a transformant bearing a DNA encoding the protein of the invention;

(d) The receptor-binding assay system such as the screening method described in (a) to (c) above, wherein the peptide of the invention is a labeled ligand;

(e) A method of screening a compound or its salt that alters the binding property between the peptide of the invention and the protein of the invention, which comprises assaying cell stimulating activities mediated by the protein of the invention, when the peptide of the invention is brought in contact with a cell or its cell membrane fraction containing the protein of the invention and when the peptide of the invention and a test compound are brought in contact with a cell or its cell membrane fraction containing the protein of the invention; and comparing the activities;

(f) A method of screening a compound or its salt that alters the binding property between the peptide of the invention and the protein of the invention, which comprises assaying cell stimulating activities mediated by the protein of the invention, when the peptide of the invention is brought in contact with a cell or its cell membrane fraction containing the protein of the invention and when the peptide of the invention and a test compound are brought in contact with a cell or its cell membrane fraction containing the protein of the invention; and comparing the activities; and,

(g) The screening method according to (f) described above, wherein the protein of the invention is the protein of the invention expressed on the cell membrane by culturing a transformant bearing a DNA encoding the protein of the invention; etc.

The screening method of the invention will be specifically described below.

First, the protein of the invention used for the screening methods of the invention may be any of those containing the protein of the invention described above. However, since human-derived organs in particular are obtained only with extreme difficulty, the protein of the invention, etc. expressed in large quantities by use of recombinants are suitable.

To produce the protein of the invention, the aforesaid methods, etc. are applied.

When cells containing the protein of the invention or membrane fractions of these cells are employed in the screening methods of the invention, these cells or membrane fractions may be prepared following the procedures later described.

Where cells containing the protein of the invention are employed, the cells may be fixed using glutaraldehyde, formalin, etc. The fixation can be made by publicly known methods.

The cells containing the protein of the invention refer to host cells wherein the protein of the invention is expressed, and such host cells include Escherichia coli, Bacillus subtilis, yeast, insect cells, animal cells, etc. described above.

The cell membrane fraction is used to mean a fraction abundant in cell membrane obtained by cell disruption and subsequent fractionation by publicly known methods. The cell disruption methods include cell squashing using a Potter-Elvehjem homogenizer, disruption using a Waring blender or Polytron (manufactured by Kinematica Inc.), disruption by ultrasonication, disruption by cell spraying through thin nozzles under an increased pressure using a French press, and the like. Cell membrane fractionation is effected mainly by fractionation using a centrifugal force, such as fractional centrifugation, density gradient centrifugation, etc. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the protein of the invention expressed and membrane components such as cell-derived phospholipids, membrane proteins, etc.

The amount of the protein of the invention in the cells or cell membrane fractions containing the protein of the invention is preferably $10^3$ to $10^8$ molecules, more preferably $10^5$ to $10^7$ molecules, per cell. As the amount of expression increases, the ligand binding activity per unit of the membrane fraction (specific activity) increases so that not only the highly sensitive screening system can be constructed but also large quantities of samples can be assayed on the same lot.

To perform the screening methods such as the receptor-binding assay system, the cell stimulating assay system and the like, for example, a fraction of the protein of the invention and a labeled form of the peptide of the invention (e.g., a labeled form of the peptide of the invention), etc. are employed. For the fraction of the protein of the invention, a fraction from naturally occurring type of the protein of the invention or a fraction from recombinant type of the protein of the invention having an activity equivalent thereto, or the like, are desirable. Herein, the equivalent activity is used to mean an equivalent ligand binding activity, etc. As the labeled ligands, there may be used ligands labeled with, e.g., [$^3$H], [$^{125}$I], [$^{14}$C], [$^{32}$P], [$^{33}$P], [$^{35}$S], etc. In particular, a labeled form of the peptide of the invention prepared by publicly known methods using Bolton-Hunter reagent are available as well.

Specifically, screening of the compound that alters the binding property between the peptide of the invention and the protein of the invention can be performed by the following procedures. First, a receptor preparation is prepared by suspending cells containing the protein of the invention or their membrane fractions in a buffer appropriate for screening. Any buffer can be used so long as it does not interfere with ligand-protein binding, such buffer including a phosphate buffer, a Tris-HCl buffer, etc. having pH of 4 to 10 (desirably pH of 6 to 8). For the purpose of minimizing non-specific binding, a surfactant such as CHAPS, Tween-80™ (manufactured by Kao-Atlas Inc.), digitonin, deoxycholate, etc. may be added to the buffer. Further for the purpose of suppressing degradation of the protein of the invention or the peptide of the invention by a protease, a protease inhibitor such as PMSF, leupeptin, E-64 (manufactured by Peptide Institute, Inc.), pepstatin, etc. may also be added. A given quantity (5,000 cpm to 500,000 cpm) of a labeled form of the peptide of the invention is added to 0.01 ml to 10 ml of the receptor solution, and at the same time, $10^{-4}$ to $10^{-1}$ μM of a test compound is allowed to be co-present. To determine the amount of non-specific binding (NSB), a reaction tube containing a large excess of the peptide of the invention in an unlabeled form is also provided. The reaction is carried out at 0°C to 50°C, preferably about 4°C to 37°C for 20 minutes to 24 hours, preferably 30 minutes to 3 hours. After completion of the reaction, the reaction mixture is filtrated through glass fiber filter paper, etc. and washed with an appropriate volume of the same buffer. The residual radioactivity in the glass fiber filter paper is then measured by means of a liquid scintillation counter or a γ-counter. When the nonspecific binding (NSB) is subtracted from the count ($B_0$) when any antagonizing compound is absent and the thus obtained count ($B_0$- NSB) is made 100%, a test compound having the specific binding (B - NSB) of, e.g., 50% or less, can be selected as a candidate substance capable of competitive inhibition.

For assaying the binding of the protein of the invention to the peptide of the invention, BIAcore (manufactured by Amersham Pharmacia Biotech, Inc.) can also be employed. According to this technique, the peptide of the invention is immobilized onto a sensor chip by the amino coupling method following the protocol attached to the device. A buffer such as phosphate buffer, Tris buffer, etc., which contains the protein of the invention purified from cells containing the protein of the invention or from transformants containing a DNA encoding the protein of the invention or a membrane fraction containing the protein of the invention, or the purified protein of the invention or a membrane fraction containing the protein of the invention and a test compound, is passed over the sensor chip at a flow rate of 2 to 20 μl/min. By monitoring that the test compound co-present alters the change in surface plasmon resonance caused by binding of the peptide of the invention to the protein of the invention on the sensor chip, the compound that alters the binding of the protein of the invention to the peptide of the invention can be screened. According to this method, the alteration can be assayed as well, by the procedure which involves immobilizing the protein of the invention onto a sensor chip and passing over the sensor chip a buffer solution such as phosphate buffer, Tris buffer, etc., which contains the peptide of the invention or the peptide of the invention and a test compound. Examples of the test compound are the same as those described above.

To perform the screening methods of the cell stimulating assay system described above, the cell-stimulating activities mediated by the protein of the invention (e.g., the activity that promotes or suppresses arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.) may be assayed by publicly known methods, or using assay kits commercially available. Specifically, the cells containing the protein of the invention are first cultured on a multi-well plate, etc. Prior to screening, the medium is replaced with a fresh medium or with an appropriate non-cytotoxic buffer, and a test compound or the like is added thereto, followed by culturing for a given period of time. Subsequently, the cells are extracted or the supernatant is recovered and the resulting product is quantified by the respective methods. Where it is difficult to detect the production of an indicator substance for the cell stimulating activity (e.g., arachidonic acid, etc.) due to a degrading enzyme contained in the cells, an inhibitor against such a degrading enzyme may be added prior to the assay. For detecting activities such as the cAMP production suppressing activity, the baseline production in the cells is increased by forskolin or the like and the suppressing effect on the increased baseline production can be detected.

To perform the screening by assaying the cell stimulating activity, cells in which an appropriate form of the protein of the invention is expressed are required. As the cells wherein the protein of the invention is expressed, a recombinant type of the aforesaid cell line wherein the protein of the invention is expressed, etc. are desirable. The cells or transformants wherein the protein of the invention is expressed may be either stably expressed cells or temporarily expressed cells. The kind of animal cells used is the same as described above.

Examples of the test compound include peptides, proteins, non-peptide compounds, synthetic compounds, fermentation products, cell extracts, plant extracts, animal tissue extracts, and the like.

In more detail, the screening methods of the cell stimulating assay system described above are described in [1] to [12] below.

[1] When receptor-expressed cells are stimulated by a receptor agonist, G protein in the cells is activated and GTP is bound thereto. This phenomenon is observed as well in a membrane fraction of the receptor-expression cells. Usually, GTP is hydrolyzed and changes to GDP. When GTPγS is previously added to the reaction solution, GTPγS is bound to G protein as in GTP, but is not hydrolyzed so that the state of GTPγS bound to the G protein-containing cell membrane is maintained. When labeled GTPγS is used, the labeled GTPγS remained on the cell membrane is measured, whereby the stimulating activity of the receptor agonist in the receptor-expressed cells can be assayed.

Utilizing this reaction, the compound that alters the binding property between the peptide of the invention and the protein of the invention can be screened by assaying the stimulating activity of the peptide of the invention on the cells wherein the protein of the invention is expressed.

This method is carried out using the membrane fraction containing the protein of the invention. In this assay method, the substance showing the activity of promoting the binding of GTPγS to the membrane fraction containing the protein of the invention is an agonist.

Specifically, the compound that alters the binding property between the peptide of the invention and the protein of the invention can be screened by assaying the GTPγS binding promoting activities on the membrane fraction containing the protein of the invention in the presence of labeled GTPγS, when the peptide of the invention is brought in contact with the membrane fraction containing the protein of the invention and when the peptide of the invention and a test compound are brought in contact with the membrane fraction containing the protein of the invention; and comparing the activities therebetween.

In this method, the test compound showing the activity of suppressing the GTPγS binding promoting activity by the peptide of the invention against the membrane fraction containing the protein of the invention can be selected as a candidate substance capable of competitive inhibition.

On the other hand, an agonist can be screened as well by contacting a test compound alone with the membrane fraction containing the protein of the invention and assaying the GTPγS binding promoting activity on the membrane fraction containing the protein of the invention.

An example of the screening method is specifically described below.

The membrane fraction containing the protein of the invention, which is prepared by a modification of publicly known methods, is diluted with a buffer for membrane dilution (50 mM Tris, 5 mM $MgCl_2$, 150 mM NaCl, 1 μM GDP, 0.1% BSA, pH 7.4). A degree of dilution varies depending upon the amount of a receptor expressed. The dilution is dispensed by 0.2 ml each in Falcon 2053, to which the peptide of the invention or the peptide of the invention and a test compound is/are added, and [$^{35}$S]GTPγS is further added to the mixture in a final concentration of 200 pM. After maintaining at 25°C for an hour, 1.5 ml of ice-cooled wash buffer (50 mM Tris, 5 mM $MgCl_2$, 150 mM NaCl, 0.1% BSA, 0.05% CHAPS, pH 7.4) is added to the mixture followed by filtration through a glass fiber filter paper GF/F. After keeping at 65°C for 30 minutes, the mixture is dried and the radioactivity of [$^{35}$S] GTPγS bound to the membrane fraction remained on the filter paper is measured with a liquid scintillation counter. When the radioactivity in the experimental zone added with the peptide of the invention alone is defined as 100% and the radioactivity in the experimental zone not added with the peptide of the invention is defined as 0%, an effect of the test compound on the GTPγS binding promoting activity by the peptide of the invention is worked out. The test compound showing the GTPγS binding promoting activity of, for example, 50% or less can be selected as a candidate compound capable of competitive inhibition.

[2] In the cells wherein the protein of the invention is expressed, the intracellular cAMP production is suppressed by stimulation of the peptide of the invention. Utilizing this reaction, the compound that alters the binding property between the peptide of the invention and the protein of the invention can be screened by assaying the stimulating activity of the peptide of the invention on the cells wherein the protein of the invention is expressed.

Specifically, the compound that alters the binding property between the peptide of the invention and the protein of the invention can be screened by assaying intracellular cAMP production suppressing activities on the cells in the presence of a substance capable of increasing the amount of intracellular cAMP, when the peptide of the invention is brought in contact with the cells wherein the protein of the invention is expressed and when the peptide of the invention and a test compound are brought in contact with the cells wherein the protein of the invention is expressed; and comparing the activities therebetween.

As the substance capable of increasing the amount of intracellular cAMP, there are employed, e.g., forskolin, calcitonin, etc.

The amount of cAMP produced in the cells wherein the protein of the invention is expressed can be assayed by the RIA system using an anti-cAMP antibody, whose antibody is obtained from immunized mouse, rat, rabbit, goat, bovine, etc., and [$^{125}$I]-labeled cAMP (both commercially available) or by the EIA system using an anti-cAMP antibody and labeled cAMP in combination. Quantification by the SPA (Scintillation Proximity Assay) method is also available, using beads, which contain scintillants bearing anti-cAMP antibodies immobilized using protein A or antibodies to IgG, etc. of animal used to produce the anti-cAMP antibodies, and $^{125}$I-labeled cAMP (the kit manufactured by Amersham Pharmacia Biotech, Inc. is used).

In this method, the test compound showing the activity of inhibiting the cAMP production suppressing activity by the peptide of the invention against the cells wherein the protein of the invention is expressed can be selected as a candidate substance capable of competitive inhibition.

On the other hand, by monitoring the cAMP production suppressing activity when a test compound alone is brought in contact with the cells wherein the protein of the invention is expressed, the compound showing the agonist activity can be screened.

A specific example of the screening method is described below.

The cells wherein the protein of the invention is expressed (e.g., animal cells such as CHO cells, etc.) ((ZAQC-B1 cells; EXAMPLE 2 later described)) are inoculated on a 24-well plate in $5 \times 10^4$ cells/well followed by cultivation for 48 hours. The cells are washed with Hanks' balanced salt solution (pH 7.4) containing 0.2 mM 3-isobutyl-methylxanthine, 0.05% BSA and 20 mM HEPES (hereinafter simply referred to as a reaction buffer). Thereafter, 0.5 ml of the reaction buffer is added to the cells and the mixture is kept warm in the medium for 30 minutes. The reaction buffer is removed and 0.25 ml of a fresh reaction buffer is added to the cells. Then, 0.25 ml of a 2 μM forskolin-containing reaction buffer, in which 1 μM of the peptide of the invention or 1 μM of the peptide of the invention and a test compound is/are incorporated, is added to the cells, followed by reacting at 37°C for 24 minutes. The reaction is terminated by adding 100 μl of 20% perchloric acid. The reaction mixture is then put on ice for an hour to extract intracellular cAMP. The amount of cAMP in the extract is measured using a cAMP EIA kit (Amersham Pharmacia Biotech). Taking the amount of cAMP produced by forskolin stimulation as 100% and the amount of cAMP inhibited by addition of 1 μM of the peptide of the invention as 0%, an effect of the test compound on the cAMP production suppressing activity by the peptide of the invention is calculated. The test compound that inhibits the activity of the peptide of the invention to increase the cAMP producing activity, e.g., to 50% or more, can be selected as a candidate substance capable of competitive inhibition.

Further in the case of using the cells wherein the protein of the invention is expressed and which show the property of increasing the intracellular cAMP level through stimulation by the peptide of the invention, the compound that alters the binding property between the peptide of the invention and the protein of the invention can be screened by assaying intracellular cAMP production promoting activities on the cells, when the peptide of the invention is brought in contact with the cells wherein the protein of the invention is expressed and when the peptide of the invention and a test compound are brought in contact with the cells wherein the protein of the invention is expressed; and comparing the activities therebetween.

In this method, the test compound showing the activity of inhibiting the cAMP production promoting activity by the peptide of the invention against the cells wherein the protein of the invention is expressed can be selected as a candidate substance capable of competitive inhibition.

On the other hand, by monitoring the cAMP production promoting activity when a test compound alone is brought in contact with the cells wherein the protein of the invention is expressed, the compound showing the agonist activity can be screened.

To determine the cAMP production promoting activity, the amount of cAMP produced by adding the peptide of the invention or the peptide of the invention and a test compound to the cells (e.g., animal cells such as CHO cells, etc.) wherein the protein of the invention is expressed, without adding forskolin in the screening method described above, is quantified by the procedure described above.

[3] The compound that alters the binding property between the peptide of the invention and the protein of the invention can be screened by assaying the stimulating activity of the peptide of the invention on the cells, in which the protein of the invention is expressed, using CRE-reporter gene vector.

A DNA containing CRE (cAMP response element) is inserted into a vector upstream the reporter gene to acquire CRE-reporter gene vector. In the CRE-reporter gene vector-transfected cells wherein the protein of the invention is expressed, stimulation accompanied by increased cAMP induces expression of the reporter gene mediated by CRE and production of the gene product (protein) of the reporter gene subsequent thereto. That is, by assaying the enzyme activity of the reporter gene protein, a change in the amount of cAMP in the CRE-reporter gene vector-transfected cells can be detected.

Specifically, the compound that alters the binding property between the peptide of the invention and the protein of the invention can be screened by assaying intracellular cAMP production suppressing activities on the cells in the presence of a substance capable of increasing the amount of intracellular cAMP, when the peptide of the invention is brought in contact with the CRE-reporter gene vector-transfected cells wherein the protein of the invention is expressed and when the peptide of the invention and a test compound are brought in contact with the CRE-reporter gene vector-transfected cells wherein the protein of the invention is expressed; and comparing the activities therebetween.

As the substance capable of increasing the amount of intracellular cAMP, there are employed, e.g., forskolin, calcitonin, etc.

As the vector, there may be employed, e.g., PicaGene Basic Vector, PicaGene Enhancer Vector (Toyo Ink Mfg. Co., Ltd.), and the like. A DNA containing CRE is inserted into the vector described above at the multicloning site upstream the reporter gene, e.g., luciferase gene, which is made a CRE-reporter gene vector.

In this method, the test compound that restores the enzyme activity suppression of the reporter gene protein by the peptide of the invention can be selected as a candidate substance capable of competitive inhibition.

On the other hand, an agonist can be screened by assaying the suppression of a luminescence level increased by forskolin stimulation when a test compound alone is brought in contact with the cells wherein the protein of the invention is expressed.

Taking as an example in which luciferase is used as a reporter gene, a specific example of this screening method is described below.

The CRE-reporter gene (luciferase)-transfected cells wherein the protein of the invention is expressed are inoculated on a 24-well plate in $5 \times 10^3$ cells/well followed by cultivation for 48 hours. The cells are washed with Hanks' balanced salt solution (pH 7.4) containing 0.2 mM 3isobutyl-methylxanthine, 0.05% BSA and 20 mM HEPES (hereinafter merely referred to as a reaction buffer). Thereafter, 0.5 ml of the reaction buffer is added to the cells and the mixture is kept warm in the medium for 30 minutes. The reaction buffer is removed and 0.25 ml of a fresh reaction buffer is added to the cells. Then, 1 μM of the peptide of the invention or 1 μM of the peptide of the invention is/are added to 0.25 ml of the reaction buffer containing 2 μM forskolin, which is added to the cells. The reaction is then carried out at 37°C for 24 minutes. The cells are dissolved in a cell lysis agent for PicaGene (Toyo Ink Mfg. Co., Ltd.) and a luminescent substrate (Toyo Ink Mfg. Co., Ltd.) is added to the lysate. Luminescence by luciferase is measured with a luminometer, a liquid scintillation counter or a top counter. The levels of luminescence by luciferase are measured when only the peptide of the invention is added and when 1 μM of the peptide of the invention and a test compound are added, and compared therebetween.

The peptide of the invention suppresses an increase in luminescence by luciferase associated with forskolin stimulation. The compound that restores the suppression can be selected as a candidate substance capable of competitive inhibition.

As the reporter gene, there may be employed genes, e.g., alkaline phosphatase, chloramphenicol acetyltransferase, β-galactosidase, etc. The enzyme activities of these reporter gene proteins are assayed in accordance with methods publicly know, or using commercially available assay kits. The alkaline phosphatase activity can be assayed by using, e.g., Lumi-Phos 530 manufactured by Wako Pure Chemical Industries, Ltd.; the chloramphenicol acetyltransferase activity by using, e.g., FAST CAT chloramphenicol Acetyltransferase Assay Kit manufactured by Wako Pure Chemical Industries, Ltd.; and the β-galactosidase activity by using, e.g., Aurora Gal-XE manufactured by Wako Pure Chemical Industries, Ltd.

[4] The cells wherein the protein of the invention is expressed extracellularly release arachidonic acid metabolites by stimulation of the peptide of the invention. Utilizing this reaction, the compound that alters the binding property between the peptide of the invention and the protein of the invention can be screened by assaying the stimulating activity of the peptide of the invention on the cells wherein the protein of the invention is expressed.

By previously incorporating labeled arachidonic acid into the cells wherein the protein of the invention is expressed, the arachidonic acid metabolite-releasing activity can be assayed by measuring the labeled arachidonic acid metabolites released outside the cells.

Specifically, the compound that alters the binding property between the peptide of the invention and the protein of the invention can be screened by assaying arachidonic acid metabolite-releasing activities, when the peptide of the invention is brought in contact with the labeled arachidonic acid-containing cells wherein the protein of the invention is expressed and when the peptide of the invention and a test compound are brought in contact with the labeled arachidonic acid-containing cells wherein the protein of the invention is expressed; and comparing the activities therebetween.

In this method, the test compound that inhibits the arachidonic acid metabolite-releasing activity by the peptide of the invention can be selected as a candidate substance capable of competitive inhibition.

Furthermore, the compound showing the agonist activity can be screened as well by contacting a test compound alone with the cells wherein the protein of the invention is expressed and monitoring the arachidonic acid metabolite-releasing activity of the cells wherein the protein of the invention is expressed, in accordance with publicly known methods.

A specific example of this screening method is described below.

The cells wherein the protein of the invention is expressed are inoculated on a 24-well plate in $5 \times 10^4$ cells/well. After cultivation for 24 hours, [$^3$H] arachidonic acid is added to the cells in 0.25 μCi/well. Sixteen hours later, the cells are washed with Hanks' balanced salt solution (pH 7.4) containing 0.05% BSA and 20 mM HEPES (hereinafter simply referred to as a reaction buffer). To each well is added 500 μl of the reaction buffer containing the peptide of the invention in the final concentration of 10 μM, or the peptide of the invention in the final concentration of 10 μM and a test compound. After incubation at 37°C for 60 minutes, 400 μl of the reaction solution is charged in a scintillator and the amount of [$^3$H] arachidonic acid metabolites released in the reaction solution is measured using a scintillation counter.

When the amount of [$^3$H] arachidonic acid metabolites when 500 μl of the reaction buffer alone is added (neither the peptide of the invention nor the test compound is added) is taken as 0% and the amount of [$^3$H] arachidonic acid metabolites when the reaction buffer containing 10 μM of the peptide of the invention is added (no test compound is added) is taken as 100%, the amount of [$^3$H] arachidonic acid metabolites released where the test compound is added is calculated.

The compound showing the arachidonic acid metabolite-releasing activity of, e.g., 50% or less, can be selected as a candidate substance capable of competitive inhibition.

[5] When the cells wherein the protein of the invention is expressed are stimulated by the peptide of the invention, an intracellular Ca level increases. The compound that alters the binding property between the peptide of the invention and the protein of the invention can be screened by assaying the stimulating activity of the peptide of the invention, utilizing this reaction.

Specifically, the compound that alters the binding property between the peptide of the invention and the protein of the invention is screened by assaying intracellular calcium level increasing activities when the peptide of the invention is brought in contact with the cells wherein the protein of the invention is expressed and when the peptide of the invention and a test compound are brought in contact with the cells wherein the protein of the invention is expressed; and comparing the activities therebetween. The assay is carried out in accordance with methods publicly known.

In this method, the test compound that suppresses the intracellular calcium level increasing activity by the peptide of the invention can be selected as a candidate substance capable of competitive inhibition.

On the other hand, an agonist can be screened as well, by assaying an increase in fluorescence intensity when a test compound alone is added.

A specific example of the screening method is described below.

The cells wherein the protein of the invention is expressed are inoculated on a sterilized cover glass for microscopy. Two days after, the culture medium is replaced by HBSS in which 4 mM Fura-2 AM (Dojin Kagaku Kenkyusho) is suspended, followed by allowing to stand at room temperature for 2 hours and 30 minutes. After washing with HBSS, the cover glass is set on a cuvette and the peptide of the invention or the peptide of the invention and a test compound is/are added thereto. Using a fluorescence spectrophotometer, an increase in the ratio of fluorescence intensities at 505 nm is measured at the excited wavelengths of 340 nm and 380 nm, which are compared.

FLIPR (manufactured by Molecular Device Co.) may also be used. Fluo-3 AM (manufactured by Dojin Kagaku Kenkyusho) is added to a suspension of the cells wherein the protein of the invention is expressed, thereby to take Fluo-3 AM into the cells. After the supernatant is washed several times through centrifugation and the cells are inoculated on a 96-well plate. After setting in the FLIPR device, the peptide of the invention or the peptide of the invention and a test compound is/are added thereto. Using a fluorescence spectrophotometer, an increase in the ratio of fluorescence intensities is measured and compared therebetween, as in the case of Fura-2.

Furthermore, in the cells wherein the protein of the invention is expressed, when such a protein gene (e.g., aequorin, etc.) that emits light in association with an increase of intracellular Ca ions is previously co-expressed, the gene protein (e.g., aequorin, etc.) changes to Ca-bound aequorin by increasing the intracellular Ca ion level to emit light. Utilizing the light emission, the compound that alters the binding property between the peptide of the invention and the protein of the invention can be screened.

The cells wherein the protein of the invention is expressed and the gene of protein capable of emitting light by increasing the intracellular Ca ions is co-expressed, are inoculated on a 96-well plate. The peptide of the invention or the peptide of the invention and a test compound is/are added thereto and using a fluorescence spectrophotometer, an increase in the ratio of fluorescence intensities is measured and compared therebetween, as described above.

The test compound that suppresses the increase in fluorescence intensity by the peptide of the invention can be selected as a candidate substance capable of competitive inhibition.

A specific example of the screening method is described below.

The 24th clone of ETA (endothelin A receptor)-expressed CHO cells (hereinafter abbreviated as ETA24; see Journal of Pharmacology and Experimental Therapeutics, 279, 675-685, 1996) is used for control, and on an assay sample, the intracellular Ca ion level increasing activity is assayed in ZAQC-B1 cells (EXAMPLE 2 later described) and ETA24 cells using FLIPR (manufactured by Molecular Device, Inc.). Both ZAQC-B1 cells and ETA24 cells are used after subculturing these cells in DMEM supplemented with ZAQC-B1 cells and ETA24 cells as well as 10% dialyzed fetal bovine serum (hereinafter abbreviated as d FBS). ZAQC-B1 and ETA24 cells are suspended in a medium (10% d FBS-DMEM), respectively, in 15 x $10^4$ cells/ml. Using a dispenser, 200 µl each (3.0 x $10^4$ cells/200 µl/well) of the suspension is inoculated on a 96-well plate for FLIPR (black plate clear bottom, Coster, Inc.), followed by incubation at 37°C overnight in a 5% $CO_2$ incubator. The cells thus incubated were used (hereinafter referred to as the cell plate). Then, 20 ml of H/HBSS (Nissui Hanks 2 (9.8 g of Nissui Pharmaceutical Co., Ltd.), 0.35 g of sodium hydrogencarbonate, 4.77 g of HEPES; after adjusting the pH to 7.4 with a sodium hydroxide solution, the mixture is sterilized through a filter), 200 µl of 250 mM Probenecid and 200 µl of fetal bovine serum (FBS) are mixed. Furthermore, 2 vials (50 µg/vial) of Fluo 3-AM (Dojin Kagaku Kenkyusho) are dissolved in 40 µl of dimethylsulfoxide and 40 µl of 20% Pluronic acid (Molecular Probes, Inc.). The resulting solution is added to H/HBSS-Probenecid-FBS described above. After mixing them, the culture solution is removed and 100 µl each of the mixture is dispensed in each well of the cell plate using an eight-stranded pipette followed by incubation at 37°C for an hour in a 5% $CO_2$ incubator (dye loading). Then, 150 µl of H/HBSS containing 2.5 mM Probenecid and 0.1% CHAPS is added to the assay sample (each fraction) for dilution, and the dilution is transferred to a 96-well plate (V-Bottom plate, Coster Inc.) (hereinafter referred to as the sample plate). After completion of the dye loading onto the cell plate, the cell plate is washed 4 times with a wash buffer, which is obtained by adding 2.5 mM Probenecid to H/HBSS, using a plate washer (Molecular Devices, Inc.) to leave 100 µl of the wash buffer after washing. The cell plate and the sample plate are set in FLIPR to perform assay (50 µl of a sample is transferred from the sample plate to the cell plate with FLIPR).

[6] When a receptor agonist is added to receptor-expressing cells, the level of intracellular inositol triphosphate increases. By utilizing the intracellular inositol triphosphate producing activity in the cells wherein the protein of the invention is expressed, the compound that alters the binding property between the peptide of the invention and the protein of the invention can be screened.

Specifically, the compound that alters the binding property between the peptide of the invention and the protein of the invention is screened by assaying inositol triphosphate producing activities in the presence of labeled inositol, when the peptide of the invention is brought in contact with the cells wherein the protein of the invention is expressed and when the peptide of the invention and a test compound are brought in contact with the cells wherein the protein of the invention is expressed; and comparing the activities therebetween. The assay is carried out in accordance with methods publicly known.

In this method, the test compound that suppresses the inositol triphosphate producing activity can be selected as a candidate substance capable of competitive inhibition.

On the other hand, an agonist may also be screened by contacting a test compound alone with the cells wherein the protein of the invention is expressed and measuring the increase of inositol triphosphate production.

A specific example of the screening method is described below.

The cells wherein the protein of the invention is expressed are inoculated on a 24-well plate and cultured for a day. Then, the cells are cultured for a day in medium supplemented with myo-[2-$^3$H]inositol (2.5 μCi/well). The cells are thoroughly washed with radioactive inositol-free medium. After the peptide of the invention or the peptide of the invention and a test compound is/are added to the cells, 10% perchloric acid is added to terminate the reaction. The reaction mixture is neutralized with 1.5 M KOH and 60 mM HEPES solution and then passed through a column packed with 0.5 ml of AG1 x 8 resin (Bio-Rad). After washing with 5 mM sodium tetraborate ($Na_2B_4O_7$) and 60 mM ammonium formate, the radioactivity eluted with 1 M ammonium formate and 0.1 M formic acid is assayed with a liquid scintillation counter. When the radioactivity without adding the peptide of the invention is made 0% and the radioactivity when the peptide of the invention is added, is made 100%, an effect of the test compound on the binding of the peptide of the invention to the protein of the invention is calculated.

The test compound that reduces the inositol triphosphate producing activity, e.g., to 50% or less, can be selected as a candidate substance capable of competitive inhibition.

[7] The compound that alters the binding property between the peptide of the invention and the protein of the invention can be screened by assaying the stimulating activity of the peptide of the invention on the cells wherein the protein of the invention is expressed, using a TRE-reporter gene vector.

A DNA containing TRE (TPA response element) is inserted into a vector upstream the reporter gene to acquire a TRE-reporter gene vector. In the TRE-reporter gene vector-transfected cells wherein the protein of the invention is expressed, stimulation accompanied by an increase of the intracellular Ca level induces expression of TRE-mediated reporter gene and production of the reporter gene product (protein) subsequent thereto. That is, by assaying the enzyme activity of the reporter gene protein, a change in the amount of calcium in the TRE-reporter gene vector-transfected cells can be detected.

Specifically, the compound that alters the binding property between the peptide of the invention and the protein of the invention is screened by assaying activities of the reporter gene protein in the presence of labeled inositol, when the peptide of the invention is brought in contact with the TRE-reporter gene vector-transfected cells wherein the protein of the invention is expressed and when the peptide of the invention and a test compound are brought in contact with the TRE-reporter gene vector-transfected cells wherein the protein of the invention is expressed; and comparing the activities therebetween.

As the vector, there may be employed, e.g., PicaGene Basic Vector, PicaGene Enhancer Vector (Toyo Ink Mfg. Co., Ltd.), and the like. A DNA containing TRE is inserted into the vector described above at the multicloning site upstream the reporter gene, e.g., luciferase gene, which is made a TRE-reporter gene vector.

In this method, the test compound that suppresses the enzyme activity of reporter gene protein by the peptide of the invention can be selected as a candidate substance capable of competitive inhibition.

On the other hand, an agonist may also be screened by contacting a test compound alone with the TRE-reporter gene vector-transfected cells wherein the protein of the invention is expressed and measuring the increased amount of luminescence as in the peptide of the invention.

Taking as an example the embodiment wherein luciferase is used as the reporter gene, a specific example of this screening method is described below.

The TRE-reporter gene (luciferase)-transfected cells wherein the protein of the invention is expressed are inoculated on a 24-well plate in $5 \times 10^3$ cells/well followed by cultivation for 48 hours. After the cells are washed with Hanks' balanced salt solution (pH 7.4) containing 0.05% BSA and 20 mM HEPES, 10 nM of the peptide of the invention or 10 nM of the peptide of the invention and a test compound is/are added to the cells, followed by reacting at 37°C for 60 minutes. The cells are dissolved in a cell lysis agent for PicaGene (Toyo Ink Mfg. Co., Ltd.) and a luminescence substrate (Toyo Ink Mfg. Co., Ltd.) is added to the lysate. The luminescence by luciferase is measured by a luminometer, a liquid scintillation counter or a top counter. The amounts of luminescence by luciferase are measured when the peptide of the invention is added and when 10 nM of the peptide of the invention and a test compound are added, and compared therebetween.

The amount of luminescence by luciferase increases with elevation of intracellular calcium by the peptide of the invention. The compound that suppresses the increase can be selected as a candidate substance capable of competitive inhibition.

As the reporter gene, there may be employed genes, e.g., alkaline phosphatase, chloramphenicol acetyltransferase, β-galactosidase, etc. The enzyme activities of these reporter gene proteins are assayed in accordance with methods publicly known, or by using assay kits commercially available. The alkaline phosphatase activity can be assayed by using, e.g., Lumi-Phos 530 manufactured by Wako Pure Chemical Industries, Ltd.; the chloramphenicol acetyltransferase activity using, e.g., FAST CAT chloramphenicol Acetyltransferase Assay Kit manufactured by Wako Pure Chemical Industries, Ltd.; and the β-galactosidase activity using, e.g., Aurora Gal-XE manufactured by Wako Pure Chemical Industries, Ltd.

[8] The cells wherein the protein of the invention is expressed are stimulated by the peptide of the invention to activate MAP kinase, which leads to cell growth. Utilizing this reaction, the compound that alters the binding property between the peptide of the invention and the protein of the invention can be screened by assaying the stimulating activity of the peptide of the invention on the cells wherein the protein of the invention is expressed.

Specifically, the compound that alters the binding property between the peptide of the invention and the protein of the invention is screened by assaying cell growth, when the peptide of the invention is brought in contact with the cells wherein the protein of the invention is expressed and when the peptide of the invention and a test compound are brought in contact with the cells wherein the protein of the invention is expressed; and comparing the cell proliferation therebetween.

The growth of the cells wherein the protein of the invention is expressed may be determined by assaying, e.g., MAP kinase activity, thymidine uptake activity, cell counts, etc.

As a specific example with respect to the MAP kinase activity, the peptide of the invention or the peptide of the invention and a test compound is/are added to the cells wherein the protein of the invention is expressed, MAP kinase fractions are then obtained from cell lysates by immunoprecipitation using an anti-MAP kinase antibody, and MAP kinase activity is assayed by methods publicly known using, e.g., MAP Kinase Assay Kit manufactured by Wako Pure Chemical Industries, Ltd. and γ-[$^{32}$P]-ATP. Comparison is made in the activity.

With respect to the thymidine uptake activity, the activity is assayed by inoculating the cells wherein the protein of the invention is expressed on a 24-well plate, culturing, adding the peptide of the invention or the peptide of the invention and a test compound to the cells, further adding radioactively labeled thymidine (e.g., [methyl-$^3$H]-thymidine, etc.), causing cell lysis and then counting the radioactivity of the thymidine taken up into the cells with a liquid scintillation counter. Comparison is made in the activity.

With respect to the cell counting, the cells wherein the protein of the invention is expressed are inoculated on a 24-well plate and cultured, the peptide of the invention or the peptide of the invention and a test compound is/are added to the cells, and MTT (3-(4,5-dimethyl-2-thiazolyl)-2,5-diphenyl-2H-tetrazolium bromide) is further added thereto. After the cells are lysed with an aqueous isopropanol solution rendered acidic with hydrochloric acid, MTT formazan changed from MTT taken up into the cells is assayed by the absorption at 570 nm.

In this method, the test compound that suppresses the growth of the cells wherein the protein of the invention is expressed can be selected as a candidate substance capable of competitive inhibition.

On the other hand, an agonist may also be screened by contacting a test compound alone with the cells wherein the protein of the invention is expressed and assaying the cell growth activity as in the peptide of the invention.

A specific example of the screening method utilizing the thymidine uptake activity is described below.

The cells wherein the protein of the invention is expressed are inoculated on a 24-well plate in 5000 cells/well followed by incubation for one day. Next, the cells are incubated in a serum-free medium for 2 days to bring the cells under starvation. The peptide of the invention or the peptide of the invention and a test compound is/are added to the cells. After incubation for 24 hours, [methyl-$^3$H] thymidine is added in 0.015 MBq/well, followed by incubation for 6 hours. After the cells are washed with PBS, methanol is added to the cells. The mixture is allowed to stand for 10 minutes. Next, 5% trichloroacetic acid is added and the mixture is allowed to stand for 15 minutes. The immobilized cells are washed 4 times with distilled water. After cell lysis with 0.3 N sodium hydroxide solution, the radioactivity in the lysate is assayed with a liquid scintillation counter.

The compound that suppresses an increase of the radioactivity when the peptide of the invention is added, can be selected as a candidate substance capable of competitive inhibition.

[9] When the cells wherein the protein of the invention is expressed are stimulated by the peptide of the invention, the potassium channel is activated so that K ions present within the cells are effluxed extracellularly. Utilizing this reaction, the compound that alters the binding property between the peptide of the invention and the protein of the invention can be screened by assaying the stimulating activity of the peptide of the invention on the cells wherein the protein of the invention is expressed.

Rb ions (rubidium ions) in the related elements to K ions flow out of the cells through the potassium channel without being distinguished from K ions. Thus, radioactive isotope Rb ([$^{86}$Rb]) is previously incorporated in the cells wherein the protein of the invention is expressed, and the efflux of $^{86}$Rb that flows out in response to stimulation by the peptide of the invention (efflux activity) is determined thereby to assay the stimulating activity of the peptide of the invention on the cells wherein the protein of the invention is expressed.

Specifically, the compound that alters the binding property between the peptide of the invention and the protein of the invention is screened by assaying $^{86}$Rb efflux activities in the presence of $^{86}$Rb, when the peptide of the invention is brought in contact with the cells wherein the protein of the invention is expressed and when the peptide of the invention and a test compound are brought in contact with the cells wherein the protein of the invention is expressed;

and comparing the activities therebetween.

In this method, the test compound that suppresses the increase of the $^{86}$Rb efflux activity associated with stimulation by the peptide of the invention can be selected as a candidate substance capable of competitive inhibition.

On the other hand, an agonist may also be screened by contacting a test compound alone with the cells wherein the protein of the invention is expressed and measuring the increase of the $^{86}$Rb efflux activity as in the peptide of the invention.

A specific example of the screening method is described below.

The cells wherein the protein of the invention is expressed are inoculated on a 24-well plate and cultured for 2 days. Thereafter, the cells are kept warm for 2 hours in a medium containing 1 mCi/ml of $^{86}$RbCl. The medium is thoroughly washed to completely remove $^{86}$RbCl in the outer liquid. The peptide of the invention or the peptide of the invention and a test compound is/are added to the cells. After the outer liquid is recovered 30 minutes later, the radioactivity is measured with a $\gamma$ counter and compared.

The test compound that suppresses the [$^{86}$Rb] efflux activity in association with stimulation by the peptide of the invention can be selected as a candidate substance capable of competitive inhibition.

[10] In the cells wherein the protein of the invention is expressed, the extracellular pH changes in response to the peptide of the invention. Utilizing this reaction, the compound that alters the binding property between the peptide of the invention and the protein of the invention can be screened by assaying the stimulating activity of the peptide of the invention on the cells wherein the protein of the invention is expressed.

Specifically, the compound that alters the binding property between the peptide of the invention and the protein of the invention is screened by measuring changes in extracellular pH, when the peptide of the invention is brought in contact with the cells wherein the protein of the invention is expressed and when the peptide of the invention and a test compound are brought in contact with the cells wherein the protein of the invention is expressed; and comparing the changes therebetween.

The extracellular pH change is determined using, e.g., Cytosensor Device (Molecular Device, Inc.).

In this method, the test compound that suppresses the extracellular pH change by the peptide of the invention can be selected as a candidate substance capable of competitive inhibition.

On the other hand, an agonist may also be screened by contacting a test compound alone with the cells wherein the protein of the invention is expressed and measuring the extracellular pH change as in the peptide of the invention.

A specific example of the screening method is described below.

The cells wherein the protein of the invention is expressed are cultured overnight in a capsule for Cytosensor Device, which is set in a chamber of the device to reflux 0.1% BSA-containing RPMI 1640 medium (manufactured by Molecular Device, Inc.) until the extracellular pH becomes stable. After the pH becomes stable, a medium containing the peptide of the invention or the peptide of the invention and a test compound is refluxed onto the cells. The pH changes in the medium caused by reflux are measured and compared.

The compound that suppresses the extracellular pH change by the peptide of the invention can be selected as a candidate substance capable of competitive inhibition.

[11] In Saccharomyces Cerevisiae, sex pheromone receptor STe2 of haploid $\alpha$-mating type (MAT$\alpha$) is coupled to G protein Gpal to activate MAP kinase in response to sex pheromone $\alpha$-mating factor, whereby Far1 (cell-cycle arrest) and transcription activator Ste12 are activated. Ste12 induces expression of a wide variety of genes (e.g., FUS 1 which participates in conjugation). On the other hand, regulator Sst2 functions to inhibit the foregoing process. In this system, an attempt has been made to construct the assay system for the reaction of a receptor agonist with a receptor, which involves preparing a receptor gene-transfected yeast, activating the intracellular signal transduction system in yeast by stimulation with the receptor agonist and using the resulting growth, etc. as an indicator (Trends in Biotechnology, 15, 487-494, 1997). Utilizing this receptor gene-transfected yeast system, the compound that alters the binding property between the peptide of the invention and the protein of the invention can be screened.

A specific example is described below.

In MAT$\alpha$ yeast, the genes encoding Ste2 and Gpal are removed and instead, the gene for the protein of the invention and the genes encoding Ste2 and Gpal is introduced. The Far-coding gene is previously removed to cause no cell-cycle arrest and the gene encoding Sst is removed to increase the sensitivity in response to the peptide of the invention. Furthermore, FUS1-HIS3 gene, which is FUS1 ligated with histidine biosynthesis gene HIS3, is introduced. This genetic recombinant engineering may be carried out, e.g., by replacing the protein of the invention for somatostatin receptor type 2 (SSTR2) gene, in the method described in Molecular and Cellular Biology. 15 6188-6195, 1995.

The thus constructed transformant yeast is responsive to the peptide of the invention with a high sensitivity so that MAP kinase is activated to cause synthesis of histidine biosynthesis enzyme. Thus, the transformant becomes capable of growing in a histidine-deficient medium.

Accordingly, the compound that alters the binding property between the peptide of the invention and the protein of the invention can be screened by culturing the aforesaid the protein of the invention-expressed yeast (MAT$\alpha$

yeast wherein Ste2 gene and Gpa1 gene are removed, the protein gene of the invention and the Gpa-Gai2 fused protein-coding gene, Far gene and Sst gene are removed, and S1-HIS3 gene is transfected) in a histidine-deficient medium, contacting the peptide of the invention or the peptide of the invention and a test compound with the yeast, assaying growth of the yeast, and comparing the growth therebetween.

In this method, the test compound that suppresses growth of the yeast can be selected as a candidate substance capable of competitive inhibition.

On the other hand, an agonist may also be screened by contacting a test compound alone with the aforesaid yeast wherein the protein of the invention is expressed and assaying growth of the yeast as in the peptide of the invention.

A specific example of the screening method is described below.

The aforesaid yeast wherein the protein of the invention is expressed thus produced is cultured overnight in a complete synthesis liquid medium and then added to a histidine-free, dissolved agar medium in a concentration of 2 x $10^4$ cells/ml, followed by inoculation on a square Petri dish of 9 x 9 cm. After the agar is solidified, a sterilized filter paper impregnated with the peptide of the invention or the peptide of the invention and a test compound is put on the agar surface, which is incubated at 30°C for 3 days. To determine the effect of the test compound, growth of yeast around the filter paper is compared to the case wherein the sterilized filter paper impregnated only with the peptide of the invention peptide. Alternatively, the peptide of the invention is previously added to a histidine-free agar medium, a sterilized filter paper is impregnated with a test compound alone, and the yeast is incubated, under which observation may be made that growth of the yeast over the entire surface of Petri dish is affected at the periphery of the filter paper.

The compound that suppresses growth of the yeast can be selected as a candidate substance capable of competitive inhibition.

[12] When the protein gene RNA of the invention is injected into *Xenopus laevis* oocytes and stimulated by the peptide of the invention, the intracellular Ca ion level increases to cause a calcium-activated chloride current, which can be taken as fluctuation in membrane potential (the same applies also to the case where fluctuation occurs in K ion level gradient). Utilizing the above reaction caused by the peptide of the invention in *Xenopus laevis* oocytes wherein the protein of the invention is transfected, the compound that alters the binding property between the peptide of the invention and the protein of the invention can be screened by assaying the stimulating activity of the peptide of the invention on the cells wherein the protein of the invention is expressed.

Specifically, the compound that alters the binding property between the peptide of the invention and the protein of the invention can be screened by assaying changes in cell membrane potential, when the peptide of the invention is brought in contact with *Xenopus laevis* oocytes wherein the RNA of the gene encoding of the protein of the invention is transfected and when the peptide of the invention and a test compound are brought in contact with *Xenopus laevis* oocytes wherein the RNA of the gene encoding of the protein of the invention is transfected; and comparing the changes therebetween.

In this method, the test compound that suppresses the changes in cell membrane potential can be selected as a candidate substance capable of competitive inhibition.

On the other hand, an agonist may also be screened by contacting a test compound alone with *Xenopus laevis* oocytes wherein the RNA of the gene encoding of the protein of the invention is transfected and assaying changes in cell membrane potential as in the peptide of the invention.

A specific example of the screening method is described below.

Female *Xenopus laevis* is anesthetized by immersing in ice water and anatomized to withdraw oocytes. The oocyte clusters are treated with collagenase (0.5 mg/ml) dissolved in an MBS solution (88 mM NaCl, 1 mM KCl, 0.41 mM $CaCl_2$, 0.33 mM $Ca(NO_3)_2$, 0.82 mM $MgSO_4$, 2.4 mM $NaHCO_3$, 10 mM HEPES; pH 7.4) at 19°C for 1 to 6 hours at 150 rpm, until the oocytes are loosen. Washing is performed 3 times by replacing the outer liquid by the MBS solution followed by microinjection of the protein gene of the invention or poly A-added cRNA (50 ng/50 nl) with a micromanipulator.

The protein gene mRNA of the invention may be prepared from tissues or cells, or may be transcribed from plasmids *in vitro.* The oocytes are incubated in the MBS solution at 20°C for 3 days. The oocytes are placed in a hole of a voltage clamp device, which is continuously perfused with Ringer's solution, and impaled into the cells with glass microelectrodes for voltage clamp and glass microelectrodes for potential recording, in which (-) electrode is placed outside the oocytes. When the holding potential stabilizes, Ringer's solution containing the peptide of the invention or the peptide of the invention and a test compound is perfused to record a change in potential. An effect of the compound can be determined by comparing a change in cell membrane potential of the *Xenopus laevis* oocytes wherein wherein the RNA of the gene encoding of the protein of the invention is transfected, with the case wherein Ringer's solution containing the peptide of the invention alone is perfused.

The compound that suppresses the change in cell membrane potential can be selected as a candidate substance capable of competitive inhibition.

In the system described above, since the assay becomes easy when the variation in potential increases, poly A-added RNA of various G protein genes may be introduced. Also, the amount of luminescence, not a change in membrane potential, may be assayed by co-injecting poly A-added RNA of a gene for such a protein (e.g., aequorin, etc.) that emits light in the presence of Ca.

The kit for screening the compound or its salt that alters the binding property between the peptide of the invention and the protein of the invention comprises the protein of the invention, cells containing the protein of the invention or cell membrane fractions containing the protein of the invention, as well as the peptide of the invention.

Examples of the screening kits of the invention are as follow.

1. Reagents for screening

(i) Assay buffer and wash buffer

Hanks' balanced salt solution (manufactured by Gibco Co.) supplemented with 0.05% bovine serum albumin (manufactured by Sigma Co.).

The solution is sterilized by filtration through a 0.45 μm filter, and stored at 4°C or may be prepared at use.

(ii) Protein preparation of the invention

CHO cells wherein the protein of the invention is expressed are subcultured on a 12-well plate at a density of $5 \times 10^5$ cells/well and cultured at 37°C under 5% $CO_2$ and 95% air for 2 days.

(iii) Labeled ligand

The peptide of the invention labeled with [$^3$H], [$^{125}$I], [$^{14}$C], [$^{35}$S], etc. A solution of the peptide dissolved in an appropriate solvent or buffer is stored at 4°C or -20°C and upon use, diluted to 1 μM with the assay buffer.

(iv) Ligand reference solution

The peptide of the invention is dissolved in PBS containing 0.1 % bovine serum albumin (manufactured by Sigma Co.) in a volume of 1 mM, and the solution is stored at -20°C.

2. Assay method

(i) The cells wherein the protein of the invention is expressed are cultured on a 12-well culture plate. After washing twice with 1 ml of the assay buffer, 490 μl of the assay buffer is added to each well.

(ii) After 5 μl of a solution of test compound in $10^{-3}$ to $10^{-10}$ M is added, 5 μl of a labeled form of the peptide of the invention is added thereto. The reaction is carried out at room temperature for an hour. To examine the non-specific binding, 5 μl of the peptide of the invention of $10^{-3}$ M is previously added in place of the test compound.

(iii) The reaction solution is removed and the wells are washed 3 times with 1 ml of the wash buffer. The labeled peptide of the invention bound to the cells is dissolved in 0.2N NaOH-1% SDS, and mixed with 4 ml of liquid scintillator A (manufactured by Wako Pure Chemical Industries, Ltd.).

(iv) The radioactivity is measured using a liquid scintillation counter (manufactured by Beckman Co.), and the percent maximum binding (PMB) is calculated in accordance with the following equation.

$$PMB = [(B - NSB)/(B_0 - NSB)] \times 100$$

| | |
|---|---|
| PMB | Percent maximum binding |
| B | Value obtained in the presence of a test compound |
| NSB | Non-specific binding |
| $B_0$ | Maximum binding |

The compound or its salt, which is obtained using the screening methods or the screening kits of the invention, is a compound that alters the binding (inhibits or promotes the binding) of the peptide of the invention to the protein of the invention, and specifically, is a compound or its salt that has the cell stimulating activity mediated by the protein of the invention (a so-called an agonist to the protein of the invention (ZAQ agonist)); or a compound that does not have the stimulating activity such as the receptor-mediated cell stimulating activity (a so-called an antagonist to the protein of the invention (ZAQ antagonist)). The compounds may be peptides, proteins, non-peptide compounds, synthetic compounds, fermentation products, etc. These compounds may be novel or publicly known compounds.

To specifically determine if the compounds are agonists or antagonists to the protein of the invention described above, the following (A) or (B) is available.

(A) The binding assay recited in the screening methods *supra* is performed to obtain the compound that alters the binding property between the peptide of the invention and the protein of the invention (especially inhibits the binding) followed by assay for the compound to determine if the compound has the cell stimulating activity mediated by the protein of the invention as described above. The compound or its salts having the cell stimulating activity are agonists to the protein of the invention, whereas the compound or its salts having no such activity are antagonists to the protein of the invention.

(B)

(a) A test compound is brought in contact with cells containing the protein of the invention to assay the cell stimulating activity mediated by the protein of the invention. The compound or its salts having the cell stimulating activity are agonists to the protein of the invention.

(b) The cell stimulating activity mediated by the protein of the invention is assayed in the case that a compound (e.g., the peptide of the invention or an agonist to the protein of the invention, etc.) that activates the protein of the invention is brought in contact with the cells containing the protein of the invention and in the case that a compound that activates the protein of the invention and a test compound are brought in contact with the cells containing the protein of the invention, and comparison is made on the cell stimulating activity between the cases. The compound or its salts capable of reducing the cell stimulating activity by the compound that activates the protein of the invention are antagonists to the protein of the invention.

The agonists to the protein of the invention exhibit activities similar to the physiological activities that the peptide of the invention has, and are thus useful as safe and low toxic drugs as in the peptide of the invention.
To the contrary, the antagonists to the protein of the invention can suppress the physiological activities that the peptide of the invention has, and are useful as safe and low toxic drugs for suppressing the receptor activity.
Since the peptide of the invention possesses the activity of controlling contraction of the intestinal tracts, etc., the peptide of the invention can be used as pharmaceuticals for the treatment/prevention, etc. of digestive diseases (e.g., enteritis, diarrhea, constipation, malabsorption syndrome, etc.). Accordingly, in the compounds obtained using the screening methods or screening kits described above, the agonists to the protein of the invention (ZAQ agonists) can be used as therapeutic/preventive agents, etc. for digestive diseases (e.g., enteritis, diarrhea, constipation, malabsorption syndrome, etc.), central nervous diseases (e.g., Alzheimer's disease, Perkinson's syndrome, schizophrenia, etc.), etc., preferably as therapeutic/preventive agents for digestive diseases. Also, the antagonists to the protein of the invention (ZAQ antagonists) can be used as therapeutic/preventive agents, etc. for diseases (e.g., enteritis, diarrhea, constipation, malabsorption syndrome, etc.), central nervous diseases (e.g., Alzheimer's disease, Perkinson's syndrome, schizophrenia, etc.), etc., preferably as therapeutic/preventive agents for digestive diseases.
As the salts of compound obtained by using the screening methods or screening kits described above, there may be employed, for example, pharmaceutically acceptable salts. Examples of the salts include salts with inorganic bases, salts with organic bases, salts with inorganic acids, salts with organic acids, salts with basic or acidic amino acids, and the like.
Preferred examples of the salts with inorganic bases include alkali metal salts such as sodium salts, potassium salts, etc.; alkaline earth metal salts such as calcium salts, magnesium salts, etc.; aluminum salts, ammonium salts, and the like.
Preferred examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc.
Preferred examples of the salts with inorganic acids include salts with hydrochloric acid, hydrobromic acid, sulfuric acid, phosphoric acid, etc.
Preferred examples of the salts with organic acids include salts with formic acid, acetic acid, propionic acid, fumaric acid, oxalic acid, tartaric acid, maleic acid, citric acid, succinic acid, malic acid, methanesulfonic acid, benzenesulfonic acid, benzoic acid, etc.
Preferred examples of the salts with basic amino acids include salts with arginine, lysine, omithine, etc. Preferred examples of the salts with acidic amino acids include salts with aspartic acid, glutamic acid, etc.
Where the compound or its salts obtained by the screening methods or kits of the invention are used as the pharmaceuticals described above, such can be performed as in the case where the aforesaid peptide of the invention is provided.
When the compound or its salts obtained by the screening methods or kits of the invention are used as the above-mentioned pharmaceuticals, they can be formulated in a conventional manner. For example, they can be administered orally as tablets coated with sugar or with enteric coating if necessary, capsules, elixirs, microcapsules, etc., or parenterally in the form of injections such as sterile solutions or suspensions in water or in pharmaceutically acceptable solutions other than water. For example, the compound or its salts can be mixed with carriers, flavoring agents, excipients, vehicles, preservatives, stabilizers, binders, etc. in a unit dosage form generally accepted. The amount of active ingredients in these preparations is adjusted so as to obtain appropriate doses within specified ranges.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, a flavoring agent such as peppermint, akamono oil and cherry, etc. When the unit dosage is in the form of a capsule, liquid carriers such as oils and fats may further be used together with the additives described above. A sterile composition for injection may be formulated in a conventional manner used to make pharmaceutical preparations, e.g., by dissolving or suspending the active ingredients in a vehicle such as water for injection with a naturally occurring vegetable oil such as sesame oil and coconut oil, etc. to prepare the pharmaceutical preparations.

Examples of an aqueous medium for injection include physiological saline and an isotonic solution containing glucose and other auxiliary agents (e.g., D-sorbitol, D-mannitol, sodium chloride, etc.), etc. and may be used in combination with an appropriate dissolution aid such as an alcohol (e.g., ethanol), a polyalcohol (e.g., propylene glycol and polyethylene glycol), a nonionic surfactant (e.g., polysorbate 80(™) and HCO-50), etc. Examples of the oily medium include sesame oil, soybean oil, etc., which may also be used in combination with a dissolution aid such as benzyl benzoate, benzyl alcohol, etc.

These compounds may further be formulated together with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

Since the pharmaceutical preparation thus obtained is safe and low toxic, it can be administered to a mammal (e.g., human, mouse, rat, guinea pig, rabbit, sheep, swine, bovine, cat, dog, monkey, chimpanzee, etc.).

The dose of the compound or its salts obtained using the screening methods or screening kits of the invention varies depending on conditions, etc.; for example, in oral administration, the dose is normally about 0.1 to about 1000 mg, preferably about 1.0 to about 300 mg, more preferably about 3.0 to about 50 mg per day for adult (as 60 kg body weight). In parenteral administration, the single dose varies depending on subject to be administered, target organ, conditions, method for administration, etc.; e.g., in the form of an injection, it is advantageous to administer the ZAQ antagonist intravenously at a daily dose of about 0.01 to about 30 mg, preferably about 0.1 to about 20 mg, more preferably about 0.1 to about 10 mg, to adult patient (as 60 kg body weight) with a digestive disease. For other animal species, the corresponding dose as converted per 60 kg weight can be administered.

(3) Quantification of the peptide of the invention or its salts

The antibody of the invention can specifically recognize the peptide of the invention. Therefore, the antibody can be used to quantify the peptide of the invention in a test fluid, especially for quantification by the sandwich immunoassay, etc.

That is, the invention provides, for example, the following methods of quantification:

(i) A method of quantifying the peptide of the invention in a test fluid, which comprises competitively reacting the antibody of the invention with the test fluid and a labeled form of the peptide of the invention, and measuring the ratio of the labeled peptide of the invention bound to the antibody; and,

(ii) A method of quantifying the peptide of the invention in a test fluid, which comprises reacting the test fluid with the antibody of the invention immobilized on a carrier and a labeled form of the antibody of the invention simultaneously or sequentially, and measuring the activity of the label on the immobilized carrier.

Using a monoclonal antibody to the peptide of the invention (hereinafter sometimes referred to as the monoclonal antibody of the invention), the peptide of the invention can be assayed and can further be detected by tissue staining or the like. For these purposes, the antibody molecule itself may be used, or $F(ab')_2$, Fab' or Fab fractions of the antibody molecule may be used as well.

The methods of quantifying Assay the peptide of the invention using the antibody of the invention are not to be limited particularly. Any method can be used, so long as the amount of antibody, antigen, or antibody-antigen complex in response to the amount of antigen (e.g., the amount of the peptide of the invention) in a test fluid can be detected by chemical or physical means and can be calculated from a standard curve prepared from standard solutions containing known amounts of the antigen. For example, nephrometry, competitive method, immunometric method, and sandwich method are advantageously used, among which the sandwich method described below is particularly preferable in terms of sensitivity and specificity.

As labeling agents for the methods using labeled substances, there are employed, for example, radioisotopes, enzymes, fluorescent substances, luminescent substances, etc. As the radioisotopes, there are employed, for example, [$^{125}$I], [$^{131}$I], [$^{3}$H], [$^{14}$C], etc. As the enzymes described above, stable enzymes with a high specific activity are preferred; for example, β-galactosidase, β-glucosidase, alkaline phosphatase, peroxidase, malate dehydrogenase and the like are used. Examples of the fluorescent substance used are fluorescamine, fluorescein isothiocyanate and the like. As the luminescent substances, there are employed, for example, luminol, luminol derivatives, luciferin, lucigenin and the like. Furthermore, the biotin-avidin system may also be used for binding an antibody or antigen to the label.

For immobilization of the antigen or antibody, physical adsorption may be used. Chemical binding methods conventionally used for insolubilization or immobilization of peptides, enzymes, etc. may be used as well. For the carriers, examples include insoluble polysaccharides such as agarose, dextran, cellulose, etc.; synthetic resin such as polystyrene, polyacrylamide, silicone, etc., or glass, etc.

In the sandwich method, the immobilized monoclonal antibody of the invention is reacted with a test fluid (primary reaction), then with a labeled form of another monoclonal antibody of the invention (secondary reaction), and the activity of the labeling agent on the immobilizing carrier is assayed, whereby the amount of the peptide of the invention in the test fluid can be quantified. The order of the primary and secondary reactions may be reversed, and the reactions may be performed simultaneously or with some time intervals. The methods of labeling and immobilization can be performed by modifications of those methods described above. In the immunoassay by the sandwich method, the antibody used for immobilized or labeled antibody is not necessarily from one species, but a mixture of two or more species of antibodies may be used to increase the measurement sensitivity.

In the methods of assaying the peptide of the invention by the sandwich method, antibodies that bind to different sites of the peptide of the invention are preferably used as the monoclonal antibodies of the invention for the primary and secondary reactions. That is, in the antibodies used for the primary and secondary reactions, for example, when the antibody used in the secondary reaction recognizes the C-terminal region of the peptide of the invention, it is preferable to use the antibody capable of recognizing the region other than the C-terminal region for the primary reaction, e.g., the antibody capable of recognizing the N-terminal region.

The monoclonal antibody of the invention can be used for the assay systems other than the sandwich method, for example, the competitive method, immunometric method, nephrometry, etc.

In the competitive method, an antigen in a test fluid and a labeled antigen are competitively reacted with an antibody, and the unreacted labeled antigen (F) and the labeled antigen bound to the antibody (B) are separated (B/F separation). The amount of the labeling agent in B or F is measured, and the amount of the antigen in the test fluid is quantified. This reaction method includes a liquid phase method using a soluble antibody as an antibody, polyethylene glycol for B/F separation and a secondary antibody, etc. to the soluble antibody, and an immobilized method either using an immobilized antibody as the primary antibody, or using a soluble antibody as the primary antibody and an immobilized antibody as the secondary antibody.

In the immunometric method, an antigen in a test fluid and an immobilized antigen are competitively reacted with a definite amount of labeled antibody, the immobilized phase is separated from the liquid phase, or an antigen in a test fluid is reacted with an excess amount of labeled antibody, the immobilized antigen is then added to bind the unreacted labeled antibody to the immobilized phase, and the immobilized phase is separated from the liquid phase. Then, the amount of the labeling agent in either phase is measured to quantify the antigen in the test fluid.

In the nephrometry, insoluble precipitates produced after the antigen-antibody reaction in gel or solution, are quantified. Even when the amount of an antigen in a test fluid is small and only a small amount of precipitates is obtained, laser nephrometry using scattering of laser can be advantageously employed.

For applying these immunological assay methods to the quantification methods of the invention, any particular conditions or procedures are not required. The assay systems for the peptide of the invention may be constructed by adding ordinary technical consideration in the art to conventional conditions and procedures in the respective methods. For the details of these general technical means, reference can be made to the following reviews and texts.

For example, reference can be made on Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immonoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing), etc.

As described above, the peptide of the invention can be quantified with high sensitivity, by using the antibody of the invention.

Furthermore, by quantifying the level of the peptide of the invention using the antibody of the present invention, (1) when an increased level of the peptide of the invention is detected, it can be diagnosed that one suffers from, for example, digestive diseases (e.g., enteritis, diarrhea, constipation, malabsorption syndrome, etc.), central nervous diseases (e.g., Alzheimer's disease, Perkinson's syndrome, schizophrenia, etc.), etc., preferably suffers from digestive diseases, etc., or it is highly likely that one would suffer from these disease in the future. Also, (2) when a decreased level of the peptide of the invention is detected, it can be diagnosed that one suffers from, for example, digestive diseases (e.g., enteritis, diarrhea, constipation, malabsorption syndrome, etc.), central nervous diseases (e.g., Alzheimer's disease, Perkinson's syndrome, schizophrenia, etc.), etc., preferably suffers from digestive diseases, etc., or it is highly likely that one would suffer from these disease in the future.

Besides, the antibody of the invention may be used for detecting the peptide of the invention present in test samples such as body fluids, tissues, etc. The antibody may also be used for preparation of antibody columns used to purify the peptide of the invention, for detection of the peptide of the invention in each fraction upon purification, for analysis of the behavior of the peptide of the invention in test cells; etc.

(4) Gene diagnostic agent

By using the DNA of the invention, e.g., as a probe, an abnormality (gene abnormality) of the DNA or mRNA encoding the peptide of the invention in human or other warm-blooded animal (e.g., rat, mouse, guinea pig, rabbit, chicken, sheep, swine, bovine, horse, cat, dog, monkey, etc.) can be detected. Therefore, the DNA of the invention is useful as a gene diagnostic agent for damages to the DNA or mRNA, its mutation or decreased expression, or increased expression or overexpression of the DNA or mRNA.

The gene diagnosis described above using the DNA of the invention can be performed by, for example, publicly known Northern hybridization or PCR-SSCP assay (Genomics, 5 874-879 (1989); Proceedings of the National Academy of Sciences of the United States of America, 86, 2766-2770 (1989)), DNA microarray, etc.

For example, when reduced expression is detected by northern hybridization or DNA microarray, or when DNA mutation is detected by PCR-SS or DNA microarray, it can be diagnosed that it is highly likely to suffer from digestive diseases (e.g., enteritis, diarrhea, constipation, malabsorption syndrome, etc.), central nervous diseases (e.g., Alzheimer's disease, Perkinson's syndrome, schizophrenia, etc.), etc., preferably to suffer from digestive diseases, etc.

(5) Pharmaceuticals comprising antisense DNA

Antisense DNA that binds to the DNA of the invention complementarily and suppresses the DNA expression can suppress in vivo the functions of the peptide of the invention or the DNA of the invention, and can be used as the agent for the treatment/prevention of diseases associated with, e.g., overexpression of the peptide of the invention (for example, digestive diseases (e.g., enteritis, diarrhea, constipation, malabsorption syndrome, etc.), central nervous diseases (e.g., Alzheimer's disease, Perkinson's syndrome, schizophrenia, etc.), etc., preferably digestive diseases, etc.).

The antisense DNA described above may be employed as the above therapeutic/prophylactic agents similarly to the therapeutic/prophylactic agent for various diseases comprising the DNA of the invention described above.

For example, the antisense DNA is administered solely, or the antisense DNA is inserted into an appropriate vector such as retrovirus vector, adenovirus vector, adenovirus-associated virus vector, etc., which is then administered in a conventional manner. The antisense DNA may be administered as it stands, or may be prepared into a dosage form together with a physiologically acceptable carrier to increase its uptake and administered by gene gun or through a catheter such as a catheter with a hydrogel.

In addition, the antisense DNA may also be employed as an oligonucleotide probe for diagnosis to examine the presence of the DNA of the invention in tissues or cells and the conditions of its expression.

The present invention further provides:

(i) A double-stranded RNA containing a part of the RNA encoding the peptide of the invention;
(ii) A pharmaceutical comprising the double-stranded RNA;
(iii) A ribozyme containing a part of the RNA encoding the peptide of the invention; and,
(iv) A pharmaceutical comprising the ribozyme.

As in the antisense nucleotide described above, the double-stranded RNA (RNAi; RNA interference method), ribozyme, etc. can suppress in vivo the expression of polynucleotide (e. g., DNA) encoding the peptide of the invention and the functions of the peptide or DNA of the invention and thus, can be used as preventive/therapeutic agents for diseases, for example, digestive diseases (e.g., enteritis, diarrhea, constipation, malabsorption syndrome, etc.), central nervous diseases (e.g., Alzheimer's disease, Perkinson's syndrome, schizophrenia, etc.), preferably for digestive diseases, etc.

The double-stranded RNA can be manufactured by designing the same based on the sequence of the polynucleotide of the invention, by publicly known methods (e.g., Nature, 411, 494, 2001) with a modification.

The ribozyme can be manufactured by designing the same based on the sequence of the polynucleotide of the invention, by a modification of publicly known methods (e.g., TRENDS in Molecular Medicine, 7, 221, 2001). For example, the ribozyme designing the same based on the sequence of the polynucleotide of the invention, can be manufactured by ligating a publicly known ribozyme to a part of the RNA encoding the peptide of the invention. The part of the RNA encoding the peptide of the invention includes a contiguous part (RNA fragment) to the cleavage site on the RNA of the invention, which can be cleaved by a publicly known ribozyme.

Where the double-stranded RNA or ribozyme described above is used as the prophylactic/therapeutic agent described above, the RNA or ribozyme may be prepared into pharmaceutical preparations, as in the antisense polynucleotide, which are provided for administration.

(6) Pharmaceuticals and diagnostic agents comprising the antibody of the invention

The antibody of the invention which possesses the effect of neutralizing the activities of the peptide of the invention can be used as pharmaceuticals for the treatment/prevention of diseases associated with, e.g., overexpression of the peptide of the invention (for example, digestive diseases (e.g., enteritis, diarrhea, constipation, malabsorption syndrome, etc.), central nervous diseases (e.g., Alzheimer's disease, Perkinson's syndrome, schizophrenia, etc.), or the like), or as diagnostic agents of diseases associated with, e.g., overexpression of the peptide of the invention (for example, digestive diseases (e.g., enteritis, diarrhea, constipation, malabsorption syndrome, etc.)).

The therapeutic/prophylactic agent for the treatment/prevention of the diseases described above, which contains the antibody of the invention, may be administered orally or parenterally to human or non-human mammals (e.g., rat, rabbit, sheep, swine, bovine, cat, dog, monkey, etc.) as a liquid preparation in its original form, or as a pharmaceutical composition in an appropriate drug form. The dose varies depending on subject to be administered, target disease, conditions, route for administration, etc.; for example, when it is used for the treatment/prevention of digestive diseases, the antibody of the invention is intravenously administered to adult normally in a single dose of about 0.01 mg to about 20 mg/kg body weight, preferably about 1.0 to about 10 mg/kg body weight, and more preferably about 0.1 to about 5 mg per day, once to about 5 times a day, preferably once to about 3 times. In parenteral administration of other routes as well as in oral administration, a dose similar to those given above can be administered. Where conditions are particularly severe, the dose may be increased depending on the conditions.

The antibody of the invention may be administered in itself or as an appropriate pharmaceutical composition. The pharmaceutical composition used for the administration described above contains a pharmacologically acceptable carrier with the aforesaid or salts thereof, a diluent or excipient. Such a composition is provided in the preparation suitable for oral or parenteral administration.

That is, examples of the composition for oral administration include solid or liquid preparations, specifically, tablets (including sugar-coated tablets and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

As the compositions for parenteral administration, there are employed, for example, injections, suppositories, etc. and the injections include the form of intravenous, subcutaneous, transcutaneous, intramuscular, drip injections, etc. Such injections are prepared by publicly known methods, e.g., by dissolving, suspending or emulsifying the aforesaid antibody or its salts in a sterile aqueous or oily liquid medium. Examples of the aqueous medium for injection used include physiological saline, isotonic solutions containing glucose and other adjuvant, etc. Appropriate dissolution aids, for example, alcohol (e.g., ethanol), polyalcohol (e.g., propylene glycol, polyethylene glycol), nonionic surfactant [e.g., polysorbate 80™, HCO-50 (polyoxyethylene (50 mol) adduct of hydrogenated castor oil)] may be used in combination. For the oily solution, for example, sesame oil, soybean oil and the like are used, and dissolution aids such as benzyl benzoate and benzyl alcohol may be used in combination. The thus-prepared liquid for injection is normally filled in an appropriate ampoule. The suppository used for rectal administration is prepared by mixing the aforesaid antibody or its salts with conventional suppository base.

The oral or parenteral pharmaceutical composition described above is advantageously prepared in a unit dosage form suitable for the dose of the active ingredient. Examples of such unit dosage form include tablets, pills, capsules, injections (ampoules), suppositories, etc. It is preferred that the antibody described above is contained generally in a dose of about 5 to about 500 mg per unit dosage form, about 5 to about 100 mg especially for injections and about 10 to about 250 mg for other preparations.

Each composition described above may further contain other active components, unless their formulation with the antibody causes any adverse interaction.

(7) Preparation of non-human animal bearing the DNA of the invention

Transgenic non-human animal capable of expressing the protein, etc. of the invention can be prepared using the DNA of the invention. Examples of the non-human animal include mammals (e.g., rats, mice, rabbits, sheep, swine, bovine, cats, dogs, monkeys, etc.) and the like (hereinafter merely referred to as animal). In particular, mice, rabbits, etc. are preferable.

To transfect the DNA of the invention into a target animal, it is generally advantageous to employ the DNA as a gene construct ligated downstream the promoter capable of expressing the DNA in an animal cell. For example, when the rabbit-derived DNA of the invention is transfected, the gene construct, in which the DNA is ligated downstream various promoters capable of expressing the DNA of the invention derived from an animal that is highly homologous to the DNA of the invention, is microinjected to, e.g., rabbit fertilized ova. Thus, the DNA-transfected animal capable of producing a high level of the protein, etc. of the invention can be prepared. As the promoters, there may be used, e.g., a virus-derived promoter and a ubiquitous expression promoter such as metallothionein, etc. Preferably, NGF gene promoters, enolase gene promoter, etc., which are specifically expressed in the brain, are used.

The transfection of the DNA of the invention to the fertilized egg cell stage secures the presence of the DNA in all germ and somatic cells in the target animal. The presence of the protein, etc. of the invention in the germ cells in the DNA-transfected animal means that all germ and somatic cells contain the receptor protein of the invention in all progenies of the animal. The progenies of the animal that took over the gene contain the protein, etc. of the invention in all germ and somatic cells.

The animal, to which the DNA of the invention has been transfected, can be subjected to mating and breeding for generations under common breeding circumstance, as the DNA-carrying animal, after confirming that the gene can be stably retained. Moreover, male and female animals bearing the desired DNA are mated to give a homozygote having the transfected gene in both homologous chromosomes and then the male and female animals are mated so that such breeding for generations that progenies contain the DNA can be performed.

The animal, to which the DNA of the invention has been transfected, may be used to analyze the functions of the peptide of the invention, since the protein, etc. of the invention is highly expressed. That is, the animal, to which he DNA of the invention has been transfected, may be used as the cell sources for tissue culture. The protein, etc. of the invention can be analyzed by, for example, direct analysis of the DNA or RNA in the tissues from the DNA-transfected mouse of the invention, or by analysis of the tissues containing the peptide of the invention expressed from the gene. Cells from tissues containing the protein, etc. of the invention are cultured by the standard tissue culture technique and using these cells, the function of the cells from the tissues that are generally difficult to culture, for example, cells derived from the brain and peripheral tissues can be studied. Using these cells, it is also possible to select pharmaceuticals, for example, that potentiate the function of various tissues. Where a highly expressing cell line is available, the protein, etc. of the invention may also be isolated and purified from the cell line.

(8) Knockout animal

The present invention provides a non-human mammal embryonic stem cell wherein the DNA of the invention is inactivated and a non-human mammal deficient in expressing the DNA of the invention.

That is, the present invention provides:

1) A non-human mammal embryonic stem cell in which the DNA of the invention is inactivated;
2) The embryonic stem cell according to 1), wherein the DNA is inactivated by introducing a reporter gene (e.g., β-galactosidase gene derived from *Escherichia coli);*
3) The embryonic stem cell according to 1), which is resistant to neomycin;
4) The embryonic stem cell according to 1), wherein the non-human mammal is a rodent;
5) The embryonic stem cell according to 4), wherein the rodent is mouse;
6) A non-human mammal deficient in expressing the DNA of the invention, wherein the DNA of the invention is inactivated;
7) The non-human mammal according to 6), wherein the DNA is inactivated by introducing a reporter gene (e.g., β-galactosidase derived from *Escherichia coli)* therein and the reporter gene is capable of being expressed under control of a promoter to the DNA of the invention;
8) The non-human mammal according to 6), which is a rodent;
9) The non-human mammal according to 8), wherein the rodent is mouse; and,
10) A method of screening a compound or its salt that promotes or inhibits the promoter activity on the DNA of the invention, which comprises administering a test compound to the mammal of 7) and detecting the expression of the reporter gene.

The non-human mammalian embryonic stem cell, in which the DNA of the invention is inactivated, refers to a non-human mammalian embryonic stem cell that suppresses the ability of the non-human mammal to express the DNA by artificially mutating the DNA of the invention possessed by the non-human mammal, or the DNA has no substantial ability to express the protein of the invention (hereinafter sometimes referred to as the knockout DNA of the invention) by substantially inactivating the activities of the peptide of the invention encoded by the DNA (hereinafter abbreviated as ES cell).

As the non-human mammal, the same examples as described above apply.

Techniques for artificially mutating the DNA of the invention include deletion of a part or all of the DNA sequence and insertion of or substitution with other DNA, e.g., by genetic engineering. By these variations, the knockout DNA of the invention may be prepared, for example, by displacing the reading frame of a codon or by disrupting the function of a promoter or exon.

Specifically, the non-human mammalian embryonic stem cell, in which the DNA of the invention is inactivated (hereinafter merely referred to as the DNA-inactivated ES cell of the invention or the knockout ES cell of the present invention), can be obtained, for example, by the following procedures: the DNA of the invention that the target non-human mammal has is isolated; a DNA strand (hereinafter simply referred to as targeting vector) having a DNA sequence so constructed as to eventually destroy the function of exon by inserting into the DNA at the exon site a chemical resistant gene represented by a neomycin resistant gene or a hygromycin resistant gene, a reporter gene represented by lacZ (β-galactosidase gene) or cat (chloramphenicol acetyltransferase gene), etc. thereby to destroy the function of exon, or by inserting into the DNA at the intron site between exons the DNA sequence which terminates gene transcription (e.g., polyA-added signal, etc.) thereby to disable the synthesis of complete messenger RNA, is transfected to a chromosome of the animal by, e.g., homologous recombination; the thus obtained ES cells are analyzed by the Southern hybridization using as a probe a DNA sequence on or contiguous to the DNA of the invention, or by PCR using as primers a DNA sequence on the targeting vector and another DNA sequence contiguous to the DNA of the invention, which is other than the DNA sequence used to prepare the targeting vector; thus, the knockout ES cell of the invention is selected.

As the parent ES cells to inactivate the DNA of the invention by homologous recombination, etc., there may be employed the strain already established as described above, or may be newly established in accordance with a modification of the publicly known method by Evans and Kaufman. For example, in the case of mouse ES cells, ES cells of the 129 strain are currently used in general. However, since the immunological background is obscure, those established using C57BL/6 mouse or $BDF_1$ mouse ($F_1$ between C57BL/6 and DBA/2), wherein the low ovum collection per C57BL/6 mouse or C57BL/6 has been improved by crossing with DBA/2, may also be preferably used instead, for purposes of obtaining a pure line of ES cells with the clear immunological genetic background; etc. The $BDF_1$ mouse is advantageous in that, when a pathologic model mouse is generated using ES cells obtained therefrom, the genetic background can be changed to that of the C57BL/6 mouse by back-crossing with the C57BL/6 mouse, since the $BDF_1$ mouse has the C57BL/6 mouse for background, in addition to advantages that ovum availability per animal is high and ova are robust.

In establishing the ES cells, blastocytes of 3.5 days after fertilization are generally used. A large number of early stage embryos can be acquired more efficiently, by collecting the embryos of the 8-cell stage and using the same for culturing to reach the blastocyte stage.

Although the ES cells used may be of either sex, male ES cells are generally more convenient for generation of a germ cell line chimera and are thus preferred. It is desirable to identify sexes as soon as possible also in order to save painstaking culture time.

An example of the method for sex identification of the ES cell includes a method in which a gene in the sex determining region on the Y-chromosome is amplified by PCR and detected. When this method is used, ES cells (about 50 cells) corresponding to almost 1 colony are sufficient, whereas karyotype analysis required about $10^6$ cells hitherto; therefore, the first selection of ES cells at the early stage of culture can be based on sex identification, and male cells can be selected early, which saves a significant amount of time at the early stage of culture.

The second selection can be achieved by, for example, confirmation of chromosome number by the G-banding method, etc. It is usually desirable that the chromosome number of the acquired ES cells is 100% of the normal number. However, when it is difficult to obtain the cells having the normal number of chromosomes due to physical operation etc. in cell establishment, it is desirable that the ES cells are again cloned to normal cells (e.g., in mouse, cells having the number of 2n = 40) after the gene of the ES cells is knocked out.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably in 5% carbon dioxide and 95% air, or 5% oxygen, 5% carbon dioxide and 90% air) in the presence of LIF (1-10000 U/ml) on appropriate feeder cells such as STO fibroblasts, treated with a trypsin/EDTA solution (normally 0.001 to 0.5% trypsin/0.1 to 5 mM EDTA, preferably about 0.1% trypsin/1 mM EDTA) at the time of passage to obtain separate single cells, which are then seeded on freshly prepared feeder cells.

This passage is normally conducted every 1 to 3 days; at the passage, cells are observed and when cells are found to be morphologically abnormal in culture, if any, it is desirable that the cells should be abandoned.

By allowing ES cells to reach a high density in mono-layers or to form cell aggregates in suspension under appropriate conditions, it is possible to differentiate them to various cell types, for example, parietal and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the invention, which are obtainable from the differentiated ES cells of the invention, are useful for studying the functions of the peptide of the invention *in vitro* cytologically.

The non-human mammal deficient in expression of the DNA of the invention can be identified from a normal animal by measuring the amount of mRNA in the subject animal by publicly known methods, and indirectly comparing the expression levels.

As the non-human mammal, the same examples described above apply.

With respect to the non-human mammal deficient in expression of the DNA of the invention, the DNA of the invention can be knocked out by transfecting the targeting vector prepared as described above to mouse embryonic stem cells or mouse oocytes, and conducting homologous recombination, in which the DNA sequence of the targeting vector wherein the DNA of the invention is inactivated by the transfection is replaced by the DNA of the invention on the chromosome of mouse embryonic stem cells or mouse oocytes.

The cells wherein the DNA of the invention is knocked out can be identified by the Southern hybridization analysis using as a probe a DNA sequence on the DNA of the invention or its contiguous region, or by PCR analysis using as primers a DNA sequence on the targeting vector and another DNA sequence which is not included in the DNA of the invention derived from mouse, which is used as the targeting vector. When non-human mammalian embryonic stem cells are used, the cell line wherein the DNA of the invention is inactivated is cloned by homologous recombination; the resulting cloned cell line is injected to, e.g., non-human mammalian embryos or blastocytes, at an appropriate stage such as the 8-cell stage. The resulting chimeric embryos are transplanted to the uterus of the pseudopregnant non-human mammal. The resulting animal is a chimeric animal composed of both cells having the normal locus of the DNA of the invention and those having the artificially mutated locus of the DNA of the invention.

When some germ cells of the chimeric animal have the mutated locus of the DNA of the invention, an individual, in which all tissues are composed of cells having the artificially mutated locus of the DNA of the invention, can be selected from a series of offsprings obtained by crossing between such a chimeric animal and a normal animal, e.g., by coat color identification, etc. The individuals thus obtained are normally deficient in heterozygous expression of the peptide of the invention. The individuals deficient in homozygous expression of the peptide of the invention can be obtained from offsprings of the intercross between the heterozygotes.

When an oocyte is used, a DNA solution may be injected, e.g., to the prenucleus by microinjection thereby to obtain a transgenic non-human mammal having a targeting vector introduced into its chromosome. From such transgenic non-human mammals, those having a mutation at the locus of the DNA of the invention can be obtained by selection based on homologous recombination.

As described above, individuals wherein the DNA of the invention is knocked out permit passage rearing under ordinary rearing conditions, after it is confirmed that the DNA has been knocked out also in the animal individuals obtained by their crossing.

Furthermore, the genital system may be obtained and maintained by conventional methods. That is, by crossing male and female animals each having the inactivated DNA, homozygote animals having the inactivated DNA on both homologous chromosomes can be obtained. The homozygotes thus obtained may be reared so that one normal individual and a plurality of homozygotes are produced from a mother animal to efficiently obtain such homozygotes. By crossing male and female heterozygotes, homozygotes and heterozygotes having the inactivated DNA are proliferated and passaged.

The non-human mammalian embryonic stem cells, in which the DNA of the invention is inactivated, are very useful for preparing a non-human mammal deficient in expression of the DNA of the invention.

Since the non-human mammal, in which the DNA of the invention fails to express, lacks various biological activities that can be induced by the peptide of the invention, such an animal can be a disease model suspected of inactivated biological activities of the peptide of the invention and thus, is useful for effective studies to investigate causes for and therapy for these diseases.

(7a) Method of screening compounds having therapeutic/prophylactic effects for diseases caused by deficiency, damages, etc. of the DNA of the invention

The non-human mammal deficient in expression of the DNA of the invention can be used for screening of compounds having therapeutic/prophylactic effects for diseases caused by deficiency, damages, etc. of the DNA of the invention.

That is, the present invention provides a method of screening a compound or its salts having therapeutic/prophylactic effects for diseases caused by deficiency, damages, etc. of the DNA of the invention, characterized by administering a test compound to the non-human mammal deficient in expression of the DNA of the invention, and observing/assaying a change occurred in the animal.

As the non-human mammal deficient in expression of the DNA of the invention used for the screening method, the same examples as given hereinabove apply.

Examples of the test compounds include peptides, proteins, non-peptide compounds, synthetic compounds, fermentation products, cell extracts, vegetable extracts, animal tissue extracts, blood plasma, etc. and these compounds may be novel compounds or publicly known compounds.

Specifically, the non-human mammal deficient in the expression of the DNA of the invention is treated with a test compound, comparison is made with an intact animal for control and a change in each organ, tissue, disease conditions, etc. of the animal is used as an indicator to assess the therapeutic/prophylactic effects of the test compound.

For treating a test animal with a test compound, for example, oral administration, intravenous injection, etc. are applied and the treatment is appropriately selected depending upon conditions of the test animal, properties of the test compound, etc. Furthermore, the amount of a test compound administered can be appropriately selected depending on administration method, nature of the test compound, or the like.

For example, in the case of screening a compound having a therapeutic/prophylactic effect for digestive diseases (e.g., enteritis, diarrhea, constipation, malabsorption syndrome, etc.), central nervous diseases (e.g., Alzheimer's disease, Perkinson's syndrome, schizophrenia, etc.) or the like, the compound is administered to a non-human mammal deficient in expression of the DNA of the invention, and a change in the amount of evacuation induced by restraint stress on the animal is measured with passage of time.

The compound obtained using the above screening method is a compound selected from the test compounds described above and exhibits a prophylactic/therapeutic effect for the diseases caused by deficiencies, damages, etc. of the peptide of the invention. Therefore, the compound can be used as a safe and low toxic drug for the prevention/treatment, etc. for these diseases. Furthermore, compounds derived from such a compound obtained by the screening above may be used as well.

The compound obtained by the screening method above may form salts. As the salts of the compound, there may be used salts with physiologically acceptable acids (e.g., inorganic acids or organic acids) or bases (e.g., alkali metal salts), preferably physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.), and the like.

A pharmaceutical comprising the compound or salts thereof obtained by the screening methods above may be manufactured in a manner similar to that of the pharmaceutical comprising the peptide of the invention described above.

Since the pharmaceutical preparation thus obtained is safe and low toxic, it can be administered to human or non-human mammals (e.g., rat, mouse, guinea pig, rabbit, sheep, swine, bovine, horse, cat, dog, monkey, etc.).

The dose of the compound or its salt may vary depending on target disease, subject to be administered, route for administration, etc. When the compound is orally administered, the compound is administered to adult patient (as 60 kg body weight) with a digestive disease generally in a daily dose of approximately 0.1 to 100 mg, preferably approximately 1.0 to 50 mg and more preferably approximately 1.0 to 20 mg. In parenteral administration, a single dose of the compound may vary depending on subject to be administered, target disease, etc. When the compound is administered to adult patient (as 60 kg) with a digestive disease in the form of injection, it is advantageous to administer the compound intravenously in the form of injection, generally in a daily dose of approximately 0.01 to 30 mg, preferably approximately 0.1 to 20 mg, and more preferably approximately 0.1 to 10 mg. For other animal species, the corresponding dose as converted per 60 kg weight can be administered.

(7b) Method of screening a compound that promotes or inhibits the activity of a promoter to the DNA of the invention

The present invention provides a method of screening a compound or its salt that promotes or inhibits the activity of a promoter to the DNA of the invention, which comprises administering a test compound to a non-human mammal deficient in expression of the DNA of the invention and detecting expression of the reporter gene.

In the screening method described above, there are particularly used, among the aforesaid non-human mammals deficient in expression of the DNA of the invention, those selected from the non-human mammal deficient in expression of the DNA of the invention, in which the DNA of the invention is inactivated by introducing a reporter gene and the reporter gene can be expressed under control of a promoter to the DNA of the invention.

The same examples given above for the test compound apply to the test compound as well.

As the reporter gene, the same specific examples given above are employed, and β-galactosidase gene (lacZ), soluble alkaline phosphatase gene, luciferase gene, etc. are preferred.

In the non-human mammal deficient in expression of the DNA of the invention wherein the DNA of the invention is substituted with a reporter gene, the reporter gene is present under control of the promoter to the DNA of the invention. Thus, the activity of the promoter can be detected by tracing the expression of a substance encoded by the reporter gene.

For example, when a part of the DNA region encoding the peptide of the invention is substituted with, e.g., β-galactosidase gene (lacZ) derived from *Escherichia coli,* β-galactosidase is expressed in the tissue where the peptide of the invention should originally be expressed, in place of the peptide of the invention. Thus, the expression state of the peptide of the invention can be readily observed in an animal in vivo, by staining with a reagent, e.g., 5-bromo-4-chloro-3 -indolyl-β-D-galactopyranoside (X-gal), which is a substrate for β-galactosidase. Specifically, a mouse deficient in the peptide of the invention, or its tissue section is fixed with glutaraldehyde, etc. After washing with phosphate buffered saline (PBS), the system is reacted with a staining solution containing X-gal at room temperature or at about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening method above are compounds selected from the test compounds described above, which promote or inhibit the promoter activity on the DNA of the invention.

The compound obtained by the screening method may be in the form of salts. The salts of the compound are salts with physiologically acceptable acids (e.g., inorganic acids, etc.) or bases (e.g., organic acids, etc.) or the lie, and physiologically acceptable acid addition salts are preferred. Examples of such salts are salts with inorganic acids (e.g., hydrochloric acid, phosphoric acid, hydrobromic acid, sulfuric acid, etc), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc), and the like.

Since the compounds or salts thereof that promote the promoter activity on the DNA of the invention can promote the expression of the peptide of the invention, or can promote the function of the protein, they are useful as pharmaceuticals for the prevention/treatment, etc. of digestive diseases (e.g., enteritis, diarrhea, constipation, malabsorption syndrome, etc.), central nervous diseases (e.g., Alzheimer's disease, Perkinson's syndrome, schizophrenia, etc.), preferably for digestive diseases, etc.

Since the compounds or salts thereof that inhibit the promoter activity on the DNA of the invention can inhibit the expression of the peptide of the invention, or can inhibit the function of the protein, they are useful as pharmaceuticals for the prevention/treatment, etc. of digestive diseases (e.g., enteritis, diarrhea, constipation, malabsorption syndrome, etc.), central nervous diseases (e.g., Alzheimer's disease, Perkinson's syndrome, schizophrenia, etc.), preferably for digestive diseases, etc.

In addition, compounds derived from the compounds obtained by the screening above may be employed as well.

The pharmaceuticals comprising the compounds or salts thereof obtained by the screening method above may be manufactured in a manner similar to those comprising the peptide of the invention described above.

Since the pharmaceutical preparation thus obtained is safe and low toxic, it can be administered to human or mammals (e.g., rat, mouse, guinea pig, rabbit, sheep, swine, bovine, horse, cat, dog, monkey, etc.).

The dose of the compound or salts thereof varies depending on target disease, subject to be administered, route for administration, etc.; for example, when the compound that promotes the promoter activity on the DNA of the invention is orally administered, they may be administered to adult patient (as 60 kg body weight) with a digestive disease normally in a daily dose of about 0.1 to about 1000 mg, preferably about 1.0 to about 50 mg and more preferably about 1.0 to about 20 mg. In parenteral administration, a single dose of the compound may vary depending upon subject to be administered, target disease, etc.; when the compound that promotes the promoter activity on the DNA of the invention is administered to adult patient (as 60 kg) with a digestive disease in the form of injectable preparation, it is advantageous to administer the compound intravenously in a daily dose of about 0.01 to about 30 mg, preferably about 0.1 to about 20 mg, more preferably about 0.1 to about 10 mg. For other animal species, the corresponding dose as converted per 60 kg can be administered.

On the other hand, for example, when the compound that inhibits the promoter activity on the DNA of the invention is orally administered, the compound is administered to adult patient (as 60 kg body weight) with a digestive disease in a daily dose of approximately 0.1 to 100 mg, preferably approximately 1.0 to 50 mg, and more preferably approximately 1.0 to 20 mg. In parenteral administration, a single dose of the compound may vary depending on subject to be administered, target disease, etc.; when the compound that inhibits the promoter activity on the DNA of the invention is administered to adult patient (as 60 kg) with a digestive disease in the form of injectable preparation, it is advantageous to administer the compound intravenously in a daily dose of approximately 0.01 to 30 mg, preferably approximately 0.1 to 20 mg, more preferably approximately 0.1 to 10 mg. For other animal species, the corresponding dose as converted per 60 kg weight can be administered.

As stated above, the non-human mammal deficient in expression of the DNA of the invention is extremely useful for screening the compound or its salt that promotes or inhibits the activity of a promoter on the DNA of the invention and can greatly contribute to the elucidation of causes for various diseases suspected of deficiency in expression of the DNA of the invention and for the development of prophylactic/therapeutic agent for these diseases.
Furthermore, a so-called transgenic animal (gene transfected animal) can be prepared by using a DNA containing the promoter region of the DNA of the invention, ligating genes encoding various proteins downstream and injecting the same into animal oocytes. It is then possible to synthesize the polypeptide specifically and study its activity in vivo. When an appropriate reporter gene is ligated to the promoter site described above and a cell line that expresses the gene is established, the resulting system can be utilized as the survey system for a low molecular compound having the action of specifically promoting or inhibiting the in vivo productivity of the peptide per se of the invention.
In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA | deoxyribonucleic acid |
| cDNA | complementary deoxyribonucleic acid |
| A | adenine |
| T | thymine |
| G | guanine |
| C | cytosine |
| Y | thymine or cytosine |
| N | thymine, cytosine, adenine or guanine |
| R | adenine or guanine |
| M | cytosine or adenine |
| W | thymine or adenine |
| S | cytosine or guanine |
| RNA | ribonucleic acid |
| mRNA | messenger ribonucleic acid |
| dATP | deoxyadenosine triphosphate |
| dTTP | deoxythymidine triphosphate |
| dGTP | deoxyguanosine triphosphate |
| dCTP | deoxycytidine triphosphate |
| ATP | adenosine triphosphate |
| Gly or G | glycine |
| Ala or A | alanine |
| Val or V | valine |
| Leu or L | leucine |
| Ile or I | isoleucine |
| Ser or S | serine |
| Thr or T | threonine |
| Cys or C | cysteine |
| Met or M | methionine |
| Glu or E | glutamic acid |
| Asp or D | aspartic acid |

(continued)

| | |
|---|---|
| Lys or K | lysine |
| Arg or R | arginine |
| His or H | histidine |
| Phe or F | phenylalanine |
| Tyr or Y | tyrosine |
| Trp or W | tryptophan |
| Pro or P | proline |
| Asn or N | asparagine |
| Gln or Q | glutamine |
| pGlu | pyroglutamic acid |
| Xaa | unidentified amino acid residue |

Also, substituents, protecting groups and reagents, etc. frequently used in this specification are presented by the codes below.

| | |
|---|---|
| Me | methyl group |
| Et | ethyl group |
| Bu | butyl group |
| Ph | phenyl group |
| Ac | acetyl group |
| TC | thiazolidine-4(R)-carboxamido group |
| Bom | benzyloxymethyl |
| Bzl | benzyl |
| Z | benzyloxycarbonyl |
| Br-Z | 2-bromobenzyloxycarbonyl |
| Cl-Z | 2-chlorobenzyloxycarbonyl |
| $Cl_2Bzl$ | 2,6-dichlorobenzyl |
| Boc | t-butoxycarbonyl |
| HOBt | 1-hydroxybenztriazole |
| HOOBt | 3,4-dihydro-3-hydroxy-4-oxo-1,2,3-benzotriazine |
| PAM | phenylacetamidomethyl |
| Tos | p-toluenesulfonyl |
| Fmoc | N-9-fluorenyl methoxycarbonyl |
| DNP | dinitrophenol |
| Bum | t-butoxymethyl |
| Trt | trityl |
| Bom | benzyloxymethyl |
| Z | benzyloxycarbonyl |
| MeBzl | 4-methylbenzyl |
| DCC | N,N'-dicyclohexylcarbodiimido |

(continued)

| | |
|---|---|
| HONB | 1-hydroxy-5-norbornene-2,3-dicarboxyimido |
| NMP | N-methylpyrrolidone |
| HONB | N-hydroxy-5-norbornene-2,3-dicarboxyimido |
| NMP | N-methylpyrrolidone |
| TFA | trifluoroacetic acid |
| CHAPS | 3-[(3-cholamidopropyl)dimethylammonio]-1-propanesulfonate |
| PMSF | phenylmethylsulfonyl fluoride |
| GDP | guanosine-5'-diphosphate |
| Fura-2AM | pentacetoxymethyl 1-[6-amino-2-(5-carboxy-2-oxazolyl)-5-benzofuranyloxy]-2-(2-amino-5-methylphen |
| | oxy)ethane-N,N,N',N'-tetraacetate |
| Fluo-3AM | pentacetoxymethyl 1-[2-amino-5-(2,7-dichloro-6-hydroxy-3-oxy-9-xanthenyl)phenoxy]-2-(2-amino-5-m ethylphenoxy)ethane-N,N,N',N'-tetraacetate |
| HEPES | 2-[4-(2-hydroxyethyl)-1-piperazinyl]ethanesulfonic acid |
| EDTA | ethylenediaminetetraacetic acid |
| SDS | sodium dodecyl sulfate |
| BSA | bovine serum albumin |
| HBSS | Hanks' balanced salt solution |
| EIA | enzymeimmunoassay |

The sequence identification numbers in the sequence listing of the specification indicates the following sequence.

[SEQ ID NO: 1]

This represents the amino acid sequence of human brain-derived protein ZAQ.

[SEQ ID NO: 2]

This represents the base sequence of the DNA encoding human brain-derived protein ZAQ (ZAQC).

[SEQ ID NO: 3]

This represents the base sequence of the DNA encoding human brain-derived protein ZAQ (ZAQT).

[SEQ ID NO: 4]

This represents the base sequence of Primer 1 used in EXAMPLE 3 later described.

[SEQ ID NO: 5]

This represents the base sequence of Primer 2 used in EXAMPLE 3 later described.

[SEQ ID NO: 6]

This represents the base sequence of the DNA encoding human type I5E receptor protein.

[SEQ ID NO: 7]

This represents the base sequence of primer hBv8-F1 used in EXAMPLE 3 later described.

[SEQ ID NO: 8]

This represents the base sequence of primer hBv8-R1 used in EXAMPLE 3 later described.

[SEQ ID NO: 9]

This represents the base sequence of the DNA fragment obtained in EXAMPLE 3 later described.

[SEQ ID NO: 10]

This represents the base sequence of primer hBv8-R2 used in EXAMPLE 3 later described.

[SEQ ID NO: 11]

This represents the 5' end base sequence encoding human type Bv8 peptide obtained in EXAMPLE 3 later described.

[SEQ ID NO: 12]

This represents the base sequence of primer hBv8-WF used in EXAMPLE 3 later described.

[SEQ ID NO: 13]

This represents the base sequence of primer hBv8-WR used in EXAMPLE 3 later described.

[SEQ ID NO: 14]

This represents the base sequence of primer hBv8-CF used in EXAMPLE 3 later described.

[SEQ ID NO: 15]

This represents the base sequence of primer hBv8-SR used in EXAMPLE 3 later described.

[SEQ ID NO: 16]

This represents the base sequence of the DNA fragment obtained in EXAMPLE 3 later described.

[SEQ ID NO: 17]

This represents the amino acid sequence of human type Bv8 precursor peptide.

[SEQ ID NO: 18]

This represents the base sequence of the DNA encoding human type Bv8 precursor peptide.

[SEQ ID NO: 19]

This represents the amino acid sequence of human type Bv8 mature peptide.

[SEQ ID NO: 20]

This represents the base sequence of the DNA encoding human type Bv8 mature peptide.

[SEQ ID NO: 21]

This represents the amino acid sequence of snake venom MIT1.

[SEQ ID NO: 22]

This represents the amino acid sequence of the C-terminal Lys-deficient human type Bv8 mature peptide.

[SEQ ID NO: 23]

This represents the base sequence of primer BF2 used in EXAMPLE 5 later described.

[SEQ ID NO: 24]

This represents the base sequence of primer BR1 used in EXAMPLE 5 later described.

[SEQ ID NO: 25]

This represents the base sequence of the DNA fragment obtained in EXAMPLE 5 later described.

[SEQ ID NO: 26]

This represents the base sequence of primer RB5-1 used in EXAMPLE 5 later described.

[SEQ ID NO: 27]

This represents the base sequence of primer RB5-3 used in EXAMPLE 5 later described.

[SEQ ID NO: 28]

This represents the 5' end base sequence encoding rat type Bv8 obtained in EXAMPLE 5 later described.

[SEQ ID NO: 29]

This represents the base sequence of primer RB3-1 used in EXAMPLE 5 later described.

[SEQ ID NO: 30]

This represents the base sequence of primer RB3-2 used in EXAMPLE 5 later described.

[SEQ ID NO: 31]

This represents the 3' end base sequence encoding rat type BV8 obtained in EXAMPLE 5 later described.

[SEQ ID NO: 32]

This represents the base sequence of primer RBv8-WF1 used in EXAMPLE 5 later described.

[SEQ ID NO: 33]

This represents the base sequence of primer RBv8-WF2 used in EXAMPLE 5 later described.

[SEQ ID NO: 34]

This represents the base sequence of primer RBv8-WR1 used in EXAMPLE 5 later described.

[SEQ ID NO: 35]

This represents the base sequence of primer RBv8-WR2 used in EXAMPLE 5 later described.

[SEQ ID NO: 36]

This represents the base sequence of the DNA fragment obtained in EXAMPLE 5 later described.

[SEQ ID NO: 37]

This represents the amino acid sequence of rat type Bv8 precursor peptide.

[SEQ ID NO: 38]

This represents the base sequence of the DNA encoding rat type Bv8 precursor peptide.

[SEQ ID NO: 39]

This represents the amino acid sequences of rat type Bv8 mature peptide and mouse type Bv8 mature peptide.

[SEQ ID NO: 40]

This represents the base sequence of the DNA encoding rat type Bv8 mature peptide.

[SEQ ID NO: 41]

This represents the base sequence of the primer used in EXAMPLE 6 later described.

[SEQ ID NO: 42]

This represents the base sequence of the primer used in EXAMPLE 6 later described.

[SEQ ID NO: 43]

This represents the base sequence of the cDNA encoding novel G protein-coupled receptor protein (rZAQ1).

[SEQ ID NO: 44]

This represents the amino acid sequence of novel G protein-coupled receptor protein (rZAQ1).

[SEQ ID NO: 45]

This represents the base sequence of Probe 1 used in EXAMPLE 7 later described.

[SEQ ID NO: 46]

This represents the base sequence of Probe 2 used in EXAMPLE 7 later described.

[SEQ ID NO: 47]

This represents the base sequence of Primer 1 used in EXAMPLE 7 later described.

[SEQ ID NO: 48]

This represents the base sequence of Primer 2 used in EXAMPLE 7 later described.

[SEQ ID NO: 49]

This represents the base sequence of Primer 3 used in EXAMPLE 7 later described.

[SEQ ID NO: 50]

This represents the base sequence of the cDNA encoding novel G protein-coupled receptor protein (rZAQ2).

[SEQ ID NO: 51]

This represents the amino acid sequence of novel G protein-coupled receptor protein (rZAQ2).

[SEQ ID NO: 52]

This represents the amino acid sequence of human type I5E receptor protein.

[SEQ ID NO: 53]

This represents the amino acid sequence of mouse-derived G protein-coupled receptor protein (GPR73).

[SEQ ID NO: 54]

This represents the amino acid sequence of mouse-derived G protein-coupled receptor protein (mI5E).

[SEQ ID NO: 55]

This represents the amino acid sequence of mouse type Bv8 precursor peptide.

[SEQ ID NO: 56]

This represents the base sequence of the DNA encoding mouse type Bv8 precursor peptide.

[SEQ ID NO: 57]

This represents the base sequence of the DNA encoding mouse type Bv8 mature peptide.

[SEQ ID NO: 58]

This represents the base sequence of Primer 3 used in EXAMPLE 1 described later.

[SEQ ID NO: 59]

This represents the base sequence of Primer 4 used in EXAMPLE 1 described later.

[SEQ ID NO: 60]

This represents the base sequence of ZAQprobe used in EXAMPLE 1 described later.

[SEQ ID NO: 61]

This represents the base sequence of primer ZAQC Sal used in EXAMPLE 1 described later.

[SEQ ID NO: 62]

This represents the base sequence of primer ZAQC Spe used in EXAMPLE 4 described later.

[SEQ ID NO: 63]

This represents the base sequence encoding mouse-derived G protein-coupled receptor protein (GPR73).

[SEQ ID NO: 64]

This represents the base sequence encoding mouse-derived G protein-coupled receptor protein (mI5E).

[SEQ ID NO: 65]

This represents the base sequence of DNA fragment #1 used in REFERENCE EXAMPLE 1.

[SEQ ID NO: 66]

This represents the base sequence of DNA fragment #2 used in REFERENCE EXAMPLE 1.

[SEQ ID NO: 67]

This represents the base sequence of DNA fragment #3 used in REFERENCE EXAMPLE 1.

[SEQ ID NO: 68]

This represents the base sequence of DNA fragment #4 used in REFERENCE EXAMPLE 1.

[SEQ ID NO: 69]

This represents the base sequence of DNA fragment #5 used in REFERENCE EXAMPLE 1.

[SEQ ID NO: 70]

This represents the base sequence of DNA fragment #6 used in REFERENCE EXAMPLE 1.

[SEQ ID NO: 71]

This represents the base sequence of the synthetic DNA encoding human type Bv8 represented by SEQ ID NO: 30.

Transformant *Escherichia coli* DH5α/pCR2.1-ZAQC obtained in EXAMPLE 1 later described has been deposited with the National Institute of Advanced Industrial Science and Technology, International Patent Organism Depositary (former Ministry of International Trade and Industry, Agency of Industrial Science and Technology, National Institute of Bioscience and Human Technology (NIBH)) located at Central 6, 1-1-1 Higashi, Tsukuba, Ibaraki, Japan (postal code 305-8566) under the Accession Number FERM BP-6855 since August 23, 1999, and with the Institute for Fermentation, Osaka (IFO) located at 2-17-85, Juso-Honmachi, Yodogawa-ku, Osaka-shi, Osaka, Japan (postal code 532-8686) under the Accession Number IFO 16301 since August 4, 1999.

Transformant *Escherichia coli* DH5α/pCR2.1-ZAQT obtained in EXAMPLE 1 later described has been deposited with the National Institute of Advanced Industrial Science and Technology, International Patent Organism Depositary (former Ministry of International Trade and Industry, Agency of Industrial Science and Technology, National Institute of Bioscience and Human Technology (NIBH)) located at Central 6, 1-1-1 Higashi, Tsukuba, Ibaraki, Japan (postal code 305-8566) under the Accession Number FERM BP-6856 since August 23, 1999, and with the Institute for Fermentation, Osaka (IFO) under the Accession Number IFO 16302 since August 4, 1999.

Transformant *Escherichia coli* TOP10/pRBv obtained in EXAMPLE 5 later described has been deposited with the National Institute of Advanced Industrial Science and Technology, International Patent Organism Depositary (former Ministry of International Trade and Industry, Agency of Industrial Science and Technology, National Institute of Bioscience and Human Technology (NIBH)) located at Central 6, 1-1-1 Higashi, Tsukuba, Ibaraki, Japan (postal code 305-8566) under the Accession Number FERM BP-7427 since January 11, 2001, and with the Institute for Fermentation, Osaka (IFO) under the Accession Number IFO 16522 since December 22, 2000.

Transformant *Escherichia coli* DH5α/pCR2.1-rZAQ1 obtained in EXAMPLE 6 later described has been deposited with the National Institute of Advanced Industrial Science and Technology, International Patent Organism Depositary (former Ministry of International Trade and Industry, Agency of Industrial Science and Technology, National Institute of Bioscience and Human Technology (NIBH)) located at Central 6, 1-1-1 Higashi, Tsukuba, Ibaraki, Japan (postal code 305-8566) under the Accession Number FERM BP-7275 since August 21, 2000, and with the Institute for Fermentation, Osaka (IFO) under the Accession Number IFO 16459 since August 1, 2000.

Transformant *Escherichia coli* DH10B/pCMV-rZAQ2 obtained in EXAMPLE 7 later described has been deposited with the National Institute of Advanced Industrial Science and Technology, International Patent Organism Depositary (former Ministry of International Trade and Industry, Agency of Industrial Science and Technology, National Institute of Bioscience and Human Technology (NIBH)) located at Central 6, 1-1-1 Higashi, Tsukuba, Ibaraki, Japan (postal code 305-8566) under the Accession Number FERM BP-7276 since August 21, 2000, and with the Institute for Fermentation, Osaka (IFO) under the Accession Number IFO 16460 since August 1, 2000.

Transformant *Escherichia coli* MM294(DE3)/pTCh2ZAQ obtained in REFERENCE EXAMPLE 1 later described has been deposited with the National Institute of Advanced Industrial Science and Technology, International Patent Organism Depositary (former Ministry of International Trade and Industry, Agency of Industrial Science and Technology, National Institute of Bioscience and Human Technology (NIBH)) located at Central 6, 1-1-1 Higashi, Tsukuba, Ibaraki, Japan (postal code 305-8566) under the Accession Number FERM BP-7572 since April 27, 2001, and with the Institute for Fermentation, Osaka (IFO) under the Accession Number IFO 16587 since March 15, 2001.

EXAMPLES

Hereinafter, the present invention will be described in more detail with reference to EXAMPLES and REFERENCE EXAMPLES but is not deemed to limit the scope of the invention. The gene manipulation procedures using *Escherichia coli* were performed in accordance with the methods described in the Molecular Cloning.

EXAMPLE 1

Cloning of cDNA encoding G protein-coupled receptor protein ZAQ and its base sequencing as well as analysis of expression distribution of ZAQ

(1) Cloning of cDNA encoding G protein-coupled receptor protein ZAQ and its base sequencing

Using human pituitary cDNA (CLONTECH, Inc.) as a template, PCR was carried out by using two primers, i. e., Primer 1 (5'-GTC GAC ATG GAG ACC ACC ATG GGG TTC ATG G-3'; SEQ ID NO: 4) and Primer 2 (5'-ACT AGT TTA TTT TAG TCT GAT GCA GTC CAC CTC TTC-3'; SEQ ID NO: 5). In the reaction above, the composition of the reaction solution was that 1/10 volume of the cDNA described above was used as a template, 1/50 volume of Advantage2 Polymerase Mix (CLONTECH, Inc.), 0.2 μM each of Primers 1 and 2 and 200 μM of dNTPs were added thereto, and the buffer bundled to the enzyme was further added to make the solution volume 25 μl. For the PCR, the reaction at 94°C for 2 minutes was followed by repeating 3 cycles of the reaction at 94°C for 20 seconds and 72°C for 100 seconds, 3 cycles of the reaction at 94°C for 20 seconds and 68°C for 100 seconds and 38 cycles of the reaction at 94°C for 20 seconds, 64°C for 20 seconds and 68°C for 100 seconds, and finally extension was carried out at 68°C for 7 minutes. After PCR, the reaction product was subcloned to plasmid vector pCR2.1 (Invitrogen, Inc.) following the instructions of TA Cloning Kit (Invitrogen, Inc.). The subcloned product was transfected to Escherichia coli DH5α followed by selecting clones bearing cDNA in ampicillin-containing LB agar medium. Thereafter, the sequences of individual clones were analyzed to acquire two cDNA sequences ZAQC (SEQ ID NO: 2) and ZAQT (SEQ ID NO: 3) encoding novel G protein-coupled receptor protein. Since both of the proteins having the amino acid sequences deduced from the cDNAs had the same sequence (SEQ ID NO: 1), they were named ZAQ; the transformant containing the DNA represented by SEQ ID NO: 2 was named Escherichia coli DH5α/pCR2.1-ZAQC and the transformant containing the DNA represented by SEQ ID NO: 3 was named Escherichia coli DH5α/pCR2.1-ZAQT.

(2) Analysis of expression distribution of ZAQ by Taqman PCR

Search of the primers and probe used for Taqman PCR were conducted on Primer Express ver.1.0 (PE Biosystems Japan), and Primer 3 (5'-TCATGTTGCTCCACTGGAAGG-3' (SEQ ID NO: 58)), Primer 4 (5'-CCAATTGTCTTGAGGTCCAGG-3' (SEQ ID NO: 29)) and ZAQprobe (5'-TTCTTACAATGGCGGTAAGTCCAGTGCAG - 3' (SEQ ID NO: 60)) were selected. FAM (6-carboxyfluorescein) was added as a reporter dye for the probe.

The PCR fragment, which was obtained by amplification using pAK-ZAQC as a template and using primers ZAQC Sal (5'-GTCGACATGGAGACCACCATGGGGTTCATGG-3' (SEQ ID NO: 61)) and ZAQC Spe (5'-ACTAGTTTATTTTAGTCTGATGCAGTCCACCTCTTC-3' (SEQ ID NO: 62)), was purified by CHROMA SPIN200 (CLONTECH Laboratories, Inc. (CA, USA)), then adjusted to $10^0$ - $10^6$ copies/μl and used as a standard DNA. For the cDNA sources of the respective tissues, Human Multiple Tissue cDNA Panel I and Panel II (CLONTECH Laboratories, Inc.) were employed. A given quantity of Taqman Universal PCR Master Mix (PE Biosystems Japan) described in the attached brochure was added to the primers, probe and template, followed by PCR and analysis using ABI PRISM 7700 Sequence Detection System (PE Biosystems Japan).

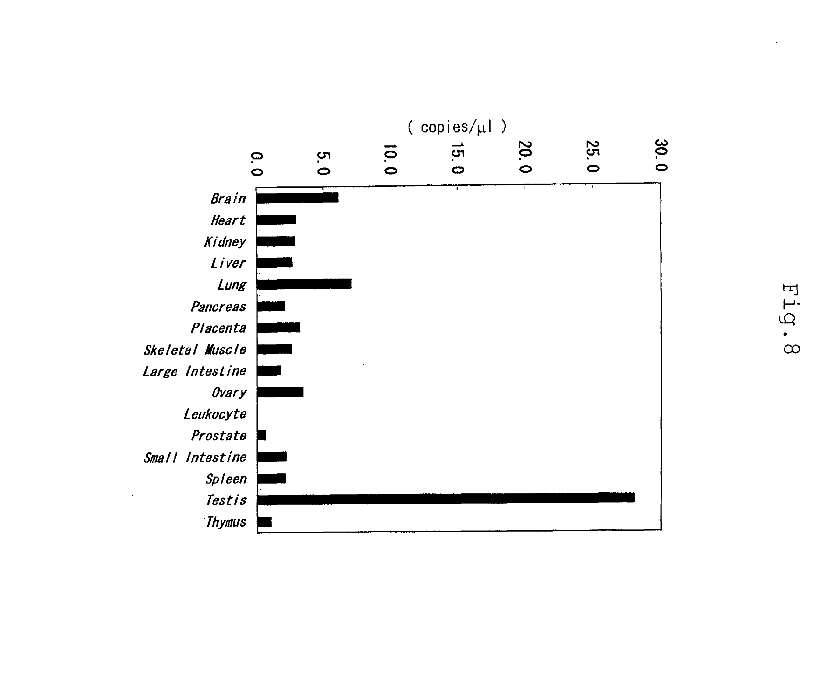

The results are shown in FIG. 8 and TABLE 1. Expression of ZAQ was observed mainly at the testis, and then at the sites such as lung, brain, etc.

TABLE 1

| Tissue | ZAQ (copy number/μl) |
|---|---|
| Brain | 6.1 |
| Heart | 2.9 |
| Kidney | 2.8 |
| Liver | 2.6 |
| Lung | 7.0 |
| Pancreas | 2.1 |
| Placenta | 3.2 |
| Skeletal Muscle | 2.6 |
| Colon | 1.8 |
| Ovary | 3.4 |
| Leukocyte | 0.0 |
| Prostate | 0.7 |
| Small Intestine | 2.2 |
| Spleen | 2.1 |
| Testis | 28.0 |
| Thymus | 1.1 |

EXAMPLE 2

Preparation of the receptor stable expression CHO cells

(2-1) Preparation of the ZAQ stable expression cell line

The ZAQ stable expression cell line was prepared as follows. That is, one clone of DH5α/pCR2.1-ZAQC obtained in EXAMPLE I was shake-cultured in an ampicillin-containing LB medium to obtain plasmid pCR2.1-ZAQC. The plasmid was digested with restriction enzymes Sal I and Spe I to excise the insert encoding ZAQC. Using Ligation Express Kit (CLONTECH Laboratories, Inc. (CA,USA)), pAKKO 1.11 H (Biochemica et Biophysica Acta, 1219 (1994) 251-259) similarly treated with restriction enzymes Sal I and Spe I was ligated with the insert, which was transfected to Escherichia coli DH10B by electroporation. The construct of the plasmid the resulting clone had was identified by restriction enzyme treatment and sequence analysis. The plasmid correctly constructed was used as plasmid pAK-ZAQC for expression of CHO cells.

A transformant was acquired by transfecting the plasmid pAK-ZAQC to CHO/dhfr⁻ cells (American Type Culture Collection) using CellPhect Transfection kit (Amersham Pharmacia Biotech, Inc.). First, 120 μl of Buffer A (bundled to CellPhect Transfection Kit) was added to 4 μg of the plasmid DNA dissolved in 120 μl of distilled water. The mixture was stirred and then allowed to stand for 10 minutes. Thereafter, 240 μl of Buffer B (bundled to CellPhect Transfection Kit) was added to the mixture, which was vigorously stirred to form DNA-calcium phosphate complex containing the DNA. Then, $5 \times 10^5$ cells of CHO/dhfr⁻ were inoculated on a 60 mm Petri dish. After culturing for 1 day in Ham's F-12 medium (Nissui Seiyaku Co., Ltd.) supplemented with 10% fetal bovine serum (BIO WHITTAKER, Inc.) at 37°C in 5% carbon dioxide gas, 480 μl of a suspension of the DNA-calcium phosphate complex was dropwise added onto the cells in the Petri dish. After culturing at 37°C in 5% carbon dioxide gas for 6 hours, the cells were washed twice with serum-free Ham's F-12 medium, and 1.2 ml of a buffer (140 mM NaCl, 25 mM HEPES, 1.4 mM $Na_2HPO_4$, pH 7.1) containing 15% glycerol was added onto the cells in the Petri dish and treated for 2 minutes. After washing twice again with serum-free Ham's F-12 medium, the cells were cultured overnight in Ham's F-12 medium containing 10% fetal bovine serum at 37°C in 5% carbon dioxide gas. The cells were dispersed by trypsin treatment and recovered from the dish. On a 6-well plate, $2 \times 10^4$ each of the cells were inoculated and culture was initiated at 37°C under 5% carbon dioxide gas in Dulbecco's modified Eagle medium (DMEM) (Nissui Seiyaku Co., Ltd.) supplemented with dialyzed 10% fetal bovine serum (JRH BIOSCIENCES, Inc.), 1 mM MEM nonessential amino acid solution (Dainippon Pharmaceutical Co., Ltd.),

100 units/ml Penicillin and 100 μg/ml Streptomycin. Since the plasmid-introduced transfected CHO cells grew in the medium but the non-transfected cells became gradually extinct, the medium was exchanged on days 1 and 2 after the initiation of culture to remove the dead cells. About 21 colonies of the transfected CHO cells, which grew in 10 to 14 days after the initiation of culture, were selected. After RNAs were recovered from the respective cells selected using commercially available RNA isolation kits, ZAQ-expressing CHO cell B-1 clone (hereinafter merely referred to as ZAQC-B 1 cells), which expressed ZAQ on a high level, was selected subsequently by following RT-PCR publicly known.
(2-2) Preparation of the ISE stable expression cell line

Human ISE receptor cDNA (SEQ ID NO: 6) was cloned by PCR publicly known and incorporated into pAKKO 1.11 expression vector. The expression vector was transfected to CHO/dhfr⁻ cells (American Type Culture Collection) by the method described in (2-1). About 20 colonies of the transfected CHO cells, which grew in 8 to 10 days after the initiation of culture, were selected. The selected cells were inoculated on a 96-well plate in 3 x $10^4$ cells/well, and the reactivity on snake venom MIT1 (SEQ ID NO: 21) was examined by the test method described in (2-3) described hereinafter. No. 4 clone of the I5E-expressing CHO cells showing a good reactivity was selected (hereinafter abbreviated as I5E-4 cells).

(2-3) Assay for the receptor activating activity

(2-3) Assay for the receptor activating activity

The ZAQC-B1 obtained in (2-1) above and the ISE-4 cells obtained in (2-2) above were suspended in a medium (10% d FBS-DMEM), respectively, in 15 x $10^4$cells/ml and an aliquot of 200 μl was inoculated on a 96-well plate for FLIPR (Black plate clear bottom, Coster, Inc.) using a dispenser (3.0 x $10^4$ cells/200 μl/well). After culturing overnight in a 5% $CO_2$ incubator at 37°C, it was provided for use (hereinafter which was made a cell plate). Twenty milliliters of H/HBSS (9.8 g of Nissui Hanks 2 (Nissui Seiyaku Co., Ltd.), 0.35 g of sodium hydrogencarbonate and 4.77 g of HEPES; the pH was adjusted to 7.4 with 6 M sodium hydroxide solution followed by filter sterilization), 200 μl of 250 mM Probenecid and 200 μl of fetal bovine serum (FBS) were mixed. Also, 2 vials (50 μg) of Fluo 3-AM (Dojin Kagaku Kenkyusho) were dissolved in 40 μl of dimethylsulfoxide and 40 μl of 20% Pluronic acid (Molecular Probes Co., Ltd.). The solution was added to H/HBSS-Probenecid-FBS described above. After thoroughly mixing them, an aliquot of 100 μl was added to the medium-removed cell plate using an 8-channel pipette, followed by incubation in a 5% $CO_2$ incubator at 37°C for an hour (dye loading). Snake venom MIT1 was diluted by adding thereto 2.5 mM Probenecid and 150 μl of H/HBSS containing 0.2% BSA. The dilution was transferred to a 96-well plate for FLIPR (V-Bottom plate, Coster, Inc.) (which was made a sample plate hereinafter). After completion of the dye loading onto the cell plate, the cell plate is washed 4 times with a wash buffer, which was obtained by adding 2.5 mM Probenecid to H/HBSS, using a plate washer (Molecular Devices, Inc.) to leave 100 μl of the wash buffer after washing. The cell plate and the sample plate were set in FLIPR to perform assay (50 μl of the sample was transferred from the sample plate to the cell plate with FLIPR). By assaying a change in fluorescence intensity with passage of time, the intracellular Ca ion level increasing activity was measured.

EXAMPLE 3

Preparation of human type Bv8 peptide

(3-1) Cloning of cDNA of human type Bv8 peptide

Using human testis Marathon-Ready cDNA (CLONTECH, Inc.) as a template, primer hBv8-F1 (SEQ ID NO: 7) and primer hBv8-R1 (SEQ ID NO: 8) were prepared and PCR was carried out as described below.

hBv8-F1: 5'-CTACTTCTGCTGCTGCCGCTGCTGTT-3' (SEQ ID NO: 7)

hBv8-R1: 5'-TTGGAAAGTTGAGGAAGCAAGAGCATTT-3' (SEQ ID NO: 8)

The reaction solution for PCR was prepared by mixing 1 μl of 50X Advantage 2 Polymerase Mix (CLONTECH, Inc.), 5 μl of 10x Advantage 2 PCR buffer bundled (400 mM Tricine-KOH, 150 mM KOAc, 35 mM $Mg(OAc)_2$, 37.5 μg/ml BSA, 0.05% Tween-20 and 0.05% Nonidet-P40), 4 μl of dNTP mixture (2.5 mM each, TaKaRa Shuzo Co., Ltd.), 1 μl of 10 μM primer hBv8-F1 and primer hBv8-R1, 5 μl of template cDNA and 33 μl of distilled water. After the early denaturation at 95°C for 1 minute, the reaction conditions were set to repeat 35 cycles of the reaction at 95°C for 30 seconds and 68°C for 1 minute, followed by final extention at 68°C for 1 minute.

Using TOPO TA Cloning Kit (Invitrogen, Inc.), the DNA fragment obtained was cloned in accordance with the method described in the manual attached. The base sequence of the cloned DNA was decoded using ABI377DNA sequencer to obtain the sequence represented by SEQ ID NO:9.

Using human testis Marathon-Ready cDNA (CLONTECH, Inc.) as a template, primer hBv8-R2 (SEQ ID NO: 10) was prepared and the 5' RACE experiment was carried out as described below.

hBv8-R2: 5'-TGTCTCCCAGTTTGCCCATAGGTGTGC-3' (SEQ ID NO: 10)

The reaction solution for PCR in the 5' RACE was prepared by mixing 1 μl of 50X Advantage-GC 2 Polymerase Mix (CLONTECH, Inc.), 10 μl of 5x Advantage-GC 2 PCR buffer bundled (200 mM Tricine-KOH, 75 mM KOAc, 17.5 mM $Mg(OAc)_2$, 25% Dimethyl Sulfoxide, 18.75 μg/ml BSA, 0.025%Tween-20 and 0.025% Nonidet-P40), 4 μl of dNTP mixture (2.5 mM each, TaKaRa Shuzo Co., Ltd.), 1 μl of 10 μM primer hBv8-R1, 1 μl of 10 μM primer AP1 (primer AP1 was the one bundled to Marathon-Ready cDNA Kit by CLONTECH, Inc.), 5 μl of cDNA (CLONTECH, Inc., human testis Marathon-Ready cDNA) and 28 μl of distilled water. After the early denaturation at 94°C for 1 minute, the reaction conditions were set to repeat 10 cycles of the reaction at 94°C for 30 seconds and 72°C for 3 minutes, 5 cycles of the reaction at 94°C for 30 seconds and 70°C for 3 minutes and 25 cycles of the reaction at 94°C for 30 seconds and 68°C for 3 minutes.

Subsequently, nested PCR was carried out using the reaction solution for PCR. The reaction solution was prepared by mixing 1 μl of 50X Advantage-GC 2 Polymerase Mix (CLONTECH, Inc.), 10 μl of 5x Advantage-GC 2 PCR buffer bundled (200 mM Tricine-KOH, 75 mM KOAc, 17.5 mM $Mg(OAc)_2$, 25% Dimethyl Sulfoxide, 18.75 μg/ml BSA, 0.025%Tween-20 and 0.025% Nonidet-P40), 4 μl of dNTP mixture (2.5 mM each, TaKaRa Shuzo Co., Ltd.), 1 μl of 10 μM primer hBv8-R2, 1 μl of 10 μM primer AP2 (primer AP2 was the one bundled to Marathon-Ready cDNA Kit from CLONTECH, Inc.), 5 μl of template DNA (50-fold dilution of the PCR reaction solution) and 28 μl of distilled water. After the early denaturation at 94°C for 1 minute, the reaction conditions were set to repeat 35 cycles of the reaction at 94°C for 30 seconds and 68°C for 3 minutes.

Using TOPO TA Cloning Kit (Invitrogen, Inc.), the DNA fragment obtained was cloned in accordance with the method described in the manual attached. The base sequence of the cloned DNA was decoded using ABI377DNA sequencer to obtain the 5' end sequence (SEQ ID NO: 11).

Based on information of SEQ ID NO: 9 and SEQ ID NO: 11, primer hBv8-WF (SEQ ID NO: 12), primer hBv8-WR (SEQ ID NO: 13), primer hBv8-CF (SEQ ID NO: 14) and primer hBv8-SR (SEQ ID NO: 15) were prepared, and PCR was carried out as described below.

hBv8-WF: 5'-CCATGAGGAGCCTGTGCTGCGCC-3' (SEQ ID NO: 12)

hBv8-WR: 5'-CTATTCACATTTGGTTTCTACTC-3' (SEQ ID NO: 13)

hBv8-CF: 5'-GTCGACCACCATGAGGAGCCTGTGCTGCG-3' (SEQ ID NO: 14)

hBv8-SR: 5'-ACTAGTCGATTACTTTTGGGCTAAAC-3' (SEQ ID NO: 15)

The reaction solution for PCR was prepared by mixing 1 μl of PfuTurbo DNA polymerase (Stratagene, Inc.), 5 μl of 10x PCR buffer bundled, 4 μl of 2.5 mM dNTP mixture, 2.5 μl each of 10 μM primer hBv8-WF and primer hBv8-WR, 5μl each of template DNA (human testis Marathon-Ready cDNA CLONTECH, Inc.) as a template and 30 μl of distilled water. After the early denaturation at 95°C for 1 minute, the reaction conditions were set to repeat 35 cycles of the reaction at 95°C for 1 minute, 65°C for 1 minute and 72°C for 1 minute, followed by extension at 72°C for 5 minutes.

Subsequently, nested PCR was carried out using the reaction solution in the PCR as a template. The reaction solution for PCR was prepared by mixing 1 μl of PfuTurbo DNA polymerase (Stratagene, Inc.), 5 μl of 10x PCR buffer bundled, 4 μl of 2.5 mM dNTP mixture, 2.5 μl each of 10 μM primer hBv8-CF and primer hBv8-SR, 1 μl of template DNA and 34 μl of distilled water. After the early denaturation at 95°C for 1 minute, the reaction conditions were set to repeat 35 cycles of the reaction at 95°C for 1 minute, 65°C for 1 minute and 72°C for 1 minute, followed by final extension at 72°C for 5 minutes.

Using TOPO TA Cloning Kit (Invitrogen, Inc.), the DNA fragment obtained was cloned in accordance with the method described in the manual attached. The base sequence of the cloned DNA was decoded using ABI377DNA sequencer, which revealed that the DNA had the base sequence of 346 bp represented by SEQ ID NO: 16. The plasmid bearing the DNA having the base sequence represented by SEQ ID NO: 16 was named pHBv. Escherichia coli was transformed by pHBv and the transformant was named Escherichia coli TOP10/pHBv.

Analysis of the base sequence of this DNA fragment revealed that the DNA fragment represented by SEQ ID NO: 17 contained the DNA (SEQ ID NO: 18) encoding human type Bv8 precursor peptide (108 amino acid residues).

Also, the base sequence represented by SEQ ID NO: 18 has a typical signal sequence. It was revealed that the DNA having the base sequence represented by SEQ ID NO: 18 contained DNA of 243 base pairs (SEQ ID NO: 20) encoding human type Bv8 mature peptide (81 amino acid residues) represented by SEQ ID NO: 19.

(3-2) Establishment of human type Bv8 peptide expression CHO cells

From the plasmid pHBv described in (3-1) above, the insert cDNA was excised using restriction enzymes Sal I and Spe I and incorporated into pAKKO 1.11 H expression vector. The plasmid was transfected to CHO/dhFr cells (American Type Culture Collection) using CellPhect Transfection kit (Amersham Pharmacia Biotech, Inc.) to acquire a transformant. First, 120 μl of Buffer A (bundled to CellPhect Transfection Kit) was added to 10 μg of the plasmid DNA dissolved in 300 μl of distilled water. The mixture was stirred and then allowed to stand for 10 minutes. Thereafter, 240 μl of Buffer B (bundled to CellPhect Transfection Kit) was added to the mixture, which was vigorously stirred to form DNA-calcium phosphate complex containing the DNA. Then, 4 x $10^5$ cells of CHO/dhfr$^-$ were inoculated on a 60 mm Petri dish. After culturing for 1 day in Ham's F-12 medium (Nissui Seiyaku Co., Ltd.) supplemented with 10% fetal bovine serum (BIO WHITTAKER, Inc.) and nonessential amino acid solution for MEM at 37°C in 5% carbon dioxide gas, the complex was dropwise added onto the cells in the Petri dish. After culturing at 37°C in 5% carbon dioxide gas for 7 hours, the cells were washed twice with serum-free Ham's F-12 medium, and 3 ml of 15% glycerol was added onto the cells in the Petri dish and treated for 2 minutes. After washing twice again with serum-free Ham's F-12 medium, the cells were cultured at 37°C in Ham's F-12 medium containing 10% fetal bovine serum for 15 hours in 5% carbon dioxide gas. The cells were dispersed by trypsin treatment and recovered from the dish. On a 6-well plate, 1.25 x $10^4$ each of the cells were inoculated and culture was initiated at 37°C in 5% carbon dioxide gas in Dulbecco's Modified Eagle Medium (DMEM) (Nissui Seiyaku Co., Ltd.) supplemented with dialyzed 10% fetal bovine serum (JRH BIOSCIENCES, Inc.), 1 mM MEM nonessential amino acid solution, 100 units/ml Penicillin and 100 μg/ml Streptomycin. Since the plasmid-introduced transfected CHO cells grew in the medium but the non-transfected cells became gradually extinct, the medium was exchanged every 2 other days after the initiation of culture to remove the dead cells. About 29 colonies of the transfected CHO cells, which grew in 10 to 14 days after the initiation of culture, were selected. After the cells became confluent, the medium was replaced by Dulbecco's modified Eagle medium (Phenol Red-free, GIBCO BRL, Inc.) containing 10% fetal bovine serum (JRH BIOSCIENCES, Inc.), 1 mM MEM nonessential amino acid solution, 100 units/ml Penicillin and 100 μg/ml Streptomycin, followed by incubation for 3 days. Then, the culture supernatant was recovered and the ZAQ activating activity was assayed in the ZAQ expression CHO cells in terms of calcium assay by FLIPR, following the method described in (2-3) above. By this assay, ZAQL-2 expression CHO cell clone No. 2 (hereinafter simply referred to as ZAQL-2/CHO No.4), which expressed human type Bv8, was selected.

(3-3) Preparation of the serum-free culture supernatant of human type Bv8 peptide expression CHO cells

ZAQL-2/CHO No.4 was cultured in Dulbecco's Modified Eagle Medium (DMEM) (Nissui Seiyaku Co., Ltd.) supplemented with dialyzed 10% fetal bovine serum (JRH BIOSCIENCES, Inc.), 1 mM MEM nonessential amino acid solution, 100 units/ml Penicillin and 100 μg/ml Streptomycin, whereby 4 Single Trays (Nunc, Inc.) became confluent, and treated with trypsin to disperse the same. The cells were then recovered. The cells on one Single Tray above were suspended in 1.5 L of the medium described above, and inoculated on Cell Factories 10 (Nunc, Inc.). The cells on four Cell Factories 10 were cultured at 37°C in 5% carbon dioxide gas for 3 days. After the culture supernatant was removed, the cells on one Cell Factories 10 was washed with 1 L of H/HBSS described above. After H/HBSS was removed, serum-free medium (Dulbecco's Modified Eagle Medium containing 1 mM MEM nonessential amino acid solution, 100 units/ml Penicillin and 100 μg/ml Streptomycin) was added in 2 L per one Cell Factories 10, followed by culturing for further 2 days. The culture supernatant recovered was centrifuged at 1,000 rpm for 10 minutes, using Hitachi High-Speed Centrifuge, and then filtered through gauze to obtain the clear supernatant. Acetic acid was added to the supernatant in a final concentration of 1 M, which was provided for the following procedures.

(3-4) Rough fractionation of the serum-free culture supernatant of human type Bv8 peptide expression CHO cells by octadodecyl reversed phase chromatography

PrepC18 (Waters, Inc.) packed with octadecyl group-immobilized silica gel was swollen and packed in a glass column (BioRad Co., Ltd., inner diameter of 5cm, height of 10 cm). Thereafter, the extract prepared in (3) above was loaded onto the column, which had been equilibrated with 1 M acetic acid. The column was then washed with 800 ml of 1 M acetic acid. Next, 1000 ml of 60% acetonitrile/0.1% trifluoroacetic acid was passed through the column to elute the desired crude peptide component. After the eluate obtained was concentrated, the concentrate was lyophilized using a freeze dryer (12EL; VirTis, Inc.).

(3-5) Separation by reversed phase high performance liquid chromatography using Wakosil-II 5C18HG Prep

Wakosil-II 5C18HG Prep (Wako Pure Chemical Industries, Ltd., 20 mm x 250 mm) column for reversed phase high performance liquid chromatography was equilibrated by passing therethrough 91.7 vol% of Solution A (0.1% trifluoroacetic acid/distilled water)/8.3 vol% of Solution B (0.1% trifluoroacetic acid/60% acetonitrile) at 40°C in a flow rate of 5 ml/min. The lyophilized product obtained in (3-4) described above was subjected to chromatographic operation. That is, the lyophilized product was dissolved by adding 36 ml of 1 M acetic acid thereto. After 1/3 volume of the solution was loaded onto the column, the concentration of Solution B was elevated at a flow rate of 5 ml/min. with a linear gradient to 66.7 vol% of Solution A/33.3% of Solution B over 1 minute and then to 16.7 vol% of Solution A/83.3% of Solution B over 120 minutes. The eluate was fractionated by 5 ml each, while assigning numbers to the fractions. This operation was performed 3 times to treat the lyophilized product of (3-4) described above. From the fractions fractionated, 3 μl each was taken and mixed with 150 μl of 0.2% BSA, which was lyophilized with a freeze dryer (12EL; VirTis, Inc.). To the lyophilized product, 50 μl of assay buffer [obtained by adding 2.5 mM Probenecid and 0.1% CHAPS to 9.8 g of H/HBSS (Nissui Hanks 2 (Nissui Seiyaku Co., Ltd.), 0.35 g of sodium hydrogencarbonate and 4.77 g of HEPES; the pH was adjusted to 7.4 with sodium hydroxide solution followed by filter sterilization treatment)] was added to dissolve the product. Using 150 μl of the solution, the ZAQ receptor activating activity was assayed by a modification of the method described in (2-3) above. The results revealed that the component having the desired ZAQ receptor activating activity was eluted maily in Fraction Nos. 73-74.

(3-6) Separation by ion exchange high performance liquid chromatography using TSKgel CM-2SW

TSKgel CM-2SW column for ion exchange high performance liquid chromatography (TOSO Co., Ltd., 4.6 mm x 250 mm) was equilibrated at 25°C by passing therethrough 100 vol% of Solution A (4 M ammonium formate : distilled water : acetonitrile = 1 : 299 : 100)/0 vol% of Solution B (4 M ammonium formate: distilled water : acetonitrile = 1 : 2 : 1) at a flow rate of 1 ml/min. The fraction Nos. 73-74 obtained in (3-5) described above was lyophilized and dissolved in 4 ml of Solution A. After the solution was loaded onto TSKgel CM-2SW column, the concentration was elevated with a linear gradient to 25 vol% of Solution A/75 vol% of Solution B over 120 minutes at a flow rate of 2 ml/min. to recover the eluate. The eluate was fractionated by 2 ml each, while assigning numbers to the fractions. From the fractions fractionated, 2 μl each was taken and mixed with 100 μl of 0.2% BSA, which was lyophilized with a freeze dryer (12EL; VirTis, Inc.). To the lyophilized product, 100 μl of the assay buffer described above was added to dissolve the product, and the solution was diluted to 100-fold with the same buffer. The ZAQ receptor activating activity was assayed by a modification of the method described in (2-3) above. The results revealed that the component having the desired ZAQ receptor activating activity was eluted maily in Fraction Nos. 83-85.

(3-7) Purification by reversed phase high performance liquid chromatography using TSKgel ODS-80Ts

TSKgel ODS-80Ts column (Toso Co., Ltd., 4.6 mm x 100 mm) for reversed phase high performance liquid chromatography was equilibrated at 40°C by passing therethrough 91.7 vol% of Solution A (0.1% trifluoroacetic acid/distilled water)/8.3 vol% of Solution B (0.1% trifluoroacetic acid/60% acetonitrile) at a flow rate of 1 ml/min. The fraction Nos. 83-85 obtained in (3-6) described above were subjected to chromatographic operation. That is, the lyophilized product was dissolved by adding 2 ml of 1 M acetic acid thereto. After the solution was loaded onto the column, the concentration of Solution B was elevated with a linear gradient at a flow rate of 1 ml/min. to 75 vol% of Solution A/25% of Solution B over 1 minute and then to 25 vol% of Solution A/75% of Solution B over 60 minutes, thereby to recover the eluate. The eluate was fractionated by 0.5 ml each, while assigning numbers to the fractions. From the fraction Nos. 121-130, 1 μl each was taken and diluted to 100-fold with the assay buffer described above, which was assayed for the ZAQ receptor activating activity by a modification of the method described in (2-3) above. It was revealed that the component having the desired ZAQ receptor activating activity was eluted in Fraction Nos. 123-125.

(3-8) Purification by reversed phase high performance liquid chromatography using TSKgel Super-Phenyl

TSKgel Super-Phenyl column (Toso Co., Ltd., 4.6 mm x 100 mm) for reversed phase high performance liquid chromatography was equilibrated at 40°C by passing therethrough 91.7 vol% of Solution A (0.1% trifluoroacetic acid/distilled water)/8.3 vol% of Solution B (0.1% trifluoroacetic acid/60% acetonitrile) at a flow rate of 1 ml/min. The fraction Nos. 123-125 obtained in (3-7) described above were subjected to chromatographic operation. That is, after the fraction solution was directly loaded to adsorb onto the column, the concentration of Solution B was elevated with a linear gradient at a flow rate of 1 ml/min. to 75 vol% of Solution A/25% of Solution B over 1 minute and then to 25 vol% of Solution A/75% of Solution B over 60 minutes, thereby to recover the eluate. The eluate was fractionated by 0.5 ml each, while assigning numbers to the fractions. From the fraction Nos. 91-100, 1 µl each was taken and diluted to 100-fold with the assay buffer described above, which was assayed for the ZAQ receptor activating activity by a modification of the method described in (2-3) above. The results revealed that the component having the desired ZAQ receptor activating activity was eluted in Fraction Nos. 97-98.

(3-9) Re-purification by reversed phase high performance liquid chromatography using TSKgel Super-Phenyl

TSKgel Super-Phenyl column (Toso Co., Ltd., 4.6 mm x 100 mm) for reversed phase high performance liquid chromatography was equilibrated at 40°C by passing therethrough 91.7 vol% of Solution A (0.1% trifluoroacetic acid/distilled water)/8.3 vol% of Solution B (0.1% trifluoroacetic acid/60% acetonitrile) at a flow rate of 1 ml/min. The fraction Nos. 97-98 obtained in (3-8) described above were subjected to chromatographic operation. That is, after the fraction solution was directly loaded to adsorb onto the column, the concentration of Solution B was elevated with a linear gradient at a flow rate of 1 ml/min. to 75 vol% of Solution A/25% of Solution B over 1 minute and then to 25 vol% of Solution A/75% of Solution B over 60 minutes, thereby to recover the eluate. As a result, the component having the desired ZAQ receptor activating activity was eluted as a single peak.

(3-10) Structural analysis of the purified human type Bv8 peptide

The structure of the main component for ZAQ activation obtained in (3-9) described above was determined by the following method. The purified human type Bv8 peptide was lyophilized with a freeze dryer (12EL; VirTis, Inc.). The lyophilized peptide obtained was dissolved in DMSO (dimethylsulfoxide). A part of the solution was provided for amino acid sequencing from the N terminus, using a protein sequencer (Perkin-Elmer, Inc., PE Biosystems Procise 491cLC). As a result, the N-terminal amino acid sequence, which coincided with the amino acid sequence of the predicted human type Bv8 mature peptide represented by SEQ ID NO: 19, was obtained. Also, mass spectrometry was performed by electron spray ionization using Finnigan LCQ LC/MS device; the molecular weight was calculated to be 8664.34. This measurement value was smaller by 128.2 than the calculated value 8792.54 of human type Bv8 mature peptide (SEQ ID NO: 19). It was thus confirmed that the peptide (SEQ ID NO:22), wherein C-terminal Lys residue was removed further from the mature peptide, could be obtained.

EXAMPLE 4

Assay for the reactivity of human type Bv8 peptide

The ZAQ and I5E receptor activating activity of the purified human type Bv8 peptide obtained in EXAMPLE 3 was assayed by a modification of the method described in (2-3) above.

As a result, human type Bv8 peptide induced the intracellular calcium level concentration-dependently in the ZAQ receptor expression CHO cells and I5E receptor expression CHO cells. Furthermore, snake venom MIT1 exhibited the I5E receptor activating activity from the level lower by about 10 times than that of human type Bv8 peptide. The results are shown in FIG. 9 and FIG. 10.

EXAMPLE 5

Cloning of cDNA of rat type Bv8 peptide

Using rat testis Marathon-Ready cDNA (CLONTECH, Inc.) as a template, degenerate primer BF2 (SEQ ID NO: 23) and primer BR1 (SEQ ID NO: 24) were prepared, and PCR was carried out as described below.

BF2: 5'-GCTTGYGACAAGGACTCYCA-3' (SEQ ID NO: 23)

BR1: 5'-GTTYCTACTYCAGAGYGAT-3' (SEQ ID NO: 24)

The reaction solution for PCR was prepared by mixing 0.4 μl of 50X Advantage 2 Polymerase Mix (CLONTECH, Inc.), 2 μl of 10x Advantage 2 PCR buffer bundled (400 mM Tricine-KOH, 150 mM KOAc, 35 mM $Mg(OAc)_2$, 37.5 μg/ml BSA, 0.05%Tween-20 and 0.05% Nonidet-P40), 1.6 μl of dNTP mixture (2.5 mM each, TaKaRa Shuzo Co., Ltd.), 0.4 μl each of 10 μM primer BF2 and primer BR1, 2 μl of cDNA and 13.2 μl of distilled water. After the early denaturation at 95°C for 1 minute, the reaction conditions were set to repeat 40 cycles of the reaction at 95°C for 30 seconds, 55°C for 1 minute and 68°C for 1 minute, followed by final extension at 68°C for 5 minutes.

Using TOPO TA Cloning Kit (Invitrogen, Inc.), the DNA fragment obtained was cloned in accordance with the method described in the manual attached. The base sequence of the cloned DNA was decoded using ABI377DNA sequencer to acquire the partial sequence represented by SEQ ID NO: 25.

Based on information of this sequence, primer RB5-1 (SEQ ID NO: 26) and primer RB5-3 (SEQ ID NO: 27) were prepared, and the 5' RACE experiment was performed as described below.

RB5-1: 5'-GTGCATCCTCCGCCCCCAAAATGGAA-3' (SEQ ID NO: 26)

RB5-3: 5'-GACAGCGCAGCACATTCCTCCTCCACAC-3' (SEQ ID NO: 27)

The reaction solution for PCR in the 5' RACE was prepared by mixing 1 μl of 50X Advantage 2 Polymerase Mix (CLONTECH, Inc.), 5 μl of 10x Advantage 2 PCR buffer (400 mM Tricine-KOH, 150 mM KOAc, 35 mM $Mg(OAc)_2$, 37.5 μg/ml BSA, 0.05%Tween-20 and 0.05% Nonidet-P40), 4 μl of dNTP mixture (2.5 mM each, TaKaRa Shuzo Co., Ltd.), 1 μl of 10 μM primer RB5-1, 1 μl of 10 μM primer AP1(primer AP was the one bundled to Marathon-Ready cDNA Kit available from CLONTECH, Inc.), 5 μl of cDNA (CLONTECH, Inc., rat testis Marathon-Ready cDNA) and 33 μl of distilled water. After the early denaturation at 94°C for 1 minute, the reaction conditions were set to repeat 5 cycles of the reaction at 94°C for 30 seconds and 72°C for 3 minutes, 5 cycles of the reaction at 94°C for 30 seconds and 70°C for 3 minutes, and 25 cycles of the reaction at 94°C for 30 seconds and 68°C for 3 minutes.

Subsequently, nested PCR was performed using the reaction solution for the PCR as a template. The reaction solution was prepared by mixing 1 μl of 50X Advantage 2 Polymerase Mix (CLONTECH, Inc.), 5 μl of 10x Advantage 2 PCR buffer bundled (400 mM Tricine-KOH, 150 mM KOAc, 35 mM $Mg(OAc)_2$, 37.5 μg/ml BSA, 0.05%Tween-20 and 0.05% Nonidet-P40), 4 μl of dNTP mixture (2.5 mM each, TaKaRa Shuzo Co., Ltd.), 1 μl of 10 μMプライマー]-RB5-3, 1 μl of 10 μM primer AP2 (primer AP2 was the one bundled to Marathon-Ready cDNA Kit from CLONTECH, Inc.), 5 μl of template cDNA (50-fold dilution of the PCR reaction solution) and 33 μl of distilled water. After the early denaturation at 94°C for 1 minute, the reaction conditions were set to repeat 35 cycles of the reaction at 94°C for 30 seconds and 68°C for 3 minutes.

Using TOPO TA Cloning Kit (Invitrogen, Inc.), the DNA fragment obtained was cloned in accordance with the method described in the manual attached. The base sequence of the cloned DNA was decoded using ABI377DNA sequencer to obtain the 5' end sequence (SEQ ID NO: 28).

Based on information of SEQ ID NO: 25, primer RB3-1 (SEQ ID NO: 29) and primer RB3-2 (SEQ ID NO: 30) were prepared, and the 3' RACE experiment was performed as described below.

RB3-1: 5'-GAGACAGCTGCCACCCCCTGACTCGGAA-3' (SEQ ID NO: 29)

RB3-2: 5'-GGCGGAGGATGCACCACACTTGTCCCTG-3' (SEQ ID NO: 30)

The reaction solution for PCR in the 3' RACE was prepared by mixing 1 μl of 50X Advantage 2 Polymerase Mix (CLONTECH, Inc.), 5 μl of 10x Advantage 2 PCR buffer bundled (400 mM Tricine-KOH, 150 mM KOAc, 35 mM $Mg(OAc)_2$, 37.5 μg/ml BSA, 0.05%Tween-20 and 0.05% Nonidet-P40), 4 μl of dNTP mixture (2.5 mM each, TaKaRa Shuzo Co., Ltd.), 1 μl of 10 μM primer RB3-1, 1 μl of 10 μM primer AP1 (primer AP was the one bundled to Marathon-Ready cDNA Kit from CLONTECH, Inc.), 5 μl of cDNA(CLONTECH, Inc., rat testis Marathon-Ready cDNA) and 33 μl of distilled water. After the early denaturation at 94°C for 1 minute, the reaction conditions were set to repeat 5 cycles of the reaction at 94°C for 30 seconds and 72°C for 3 minutes, 5 cycles of the reaction at 94°C for 30 seconds and 70°C for 3 minutes and 25 cycles of the reaction at 94°C for 30 seconds and 68°C for 3 minutes.

Subsequently, nested PCR was performed using the reaction solution for the PCR. The reaction solution was prepared by mixing 1 µl of 50X Advantage 2 Polymerase Mix (CLONTECH, Inc.), 5 µl of 10x Advantage 2 PCR buffer (400 mM Tricine-KOH, 150 mM KOAc, 35 mM $Mg(OAc)_2$, 37.5 µg/ml BSA, 0.05%Tween-20 and 0.05% Nonidet-P40), 4 µl of dNTP mixture (2.5 mM each, TaKaRa Shuzo Co., Ltd.), 1 µl of 10 µM primer RB3-2, 1 µl of 10 µM primer AP2 (primer AP2 was the one bundled to Marathon-Ready cDNA Kit from CLONTECH, Inc.), 5 µl of template cDNA (50-fold dilution of the PCR reaction solution) and 33 µl of distilled water. After the early denaturation at 94°C for 1 minute, the reaction conditions were set to repeat 35 cycles of the reaction at 94°C for 30 seconds and 68°C for 3 minutes.

Using TOPO TA Cloning Kit (Invitrogen, Inc.), the DNA fragment obtained was cloned in accordance with the method described in the manual attached. The base sequence of the cloned DNA was decoded using ABI377DNA sequencer to obtain the 3' end sequence (SEQ ID NO: 31).

Using rat brain Marathon-Ready cDNA (CLONTECH, Inc.) as a template, primer RBv8-WF1 (SEQ ID NO: 32), primer RBv8-WF2 (SEQ ID NO: 33), primer RBv8-WR1 (SEQ ID NO: 34) and primer RBv8-WR2 (SEQ ID NO: 35) were prepared based on information of the 5' RACE and 3' RACE, and PCR was performed as described below.

RBv8-WF1: 5'-TAACCGCCACCGCCTCCT-3' (SEQ ID NO: 32)

RBv8-WF2: 5'-GGGACGCCATGGAGGAC-3' (SEQ ID NO: 33)

RBv8-WR1: 5'-CGAGACTTGACAGACATTGTTCAGTG-3' (SEQ ID NO: 34)

RBv8-WR2: 5'-TTTCCAGCTCCTGCTTCAGA-3' (SEQ ID NO: 35)

The reaction solution for PCR was prepared by mixing 0.6 µl of PfuTurbo DNA polymerase(Stratagene, Inc.), 3 µl of 10x PCR buffer bundled, 2.4 µl of 2.5 mM dNTP mixture, 1.5 µl each of 10 µM primer RBv8-WF1 and primer RBv8-WR1, 3 µl of template DNA and 18 µl of distilled water. After the early denaturation at 95°C for 1 minute, the reaction conditions were set to repeat 35 cycles of the reaction at 95°C for 30 seconds, 55°C for 30 seconds and 72°C for 1 minute, followed by final extension at 72°C for 5 minutes.

Subsequently, the reaction solution in the PCR was diluted to 50-fold with distilled water, and nested PCR was performed using the dilution as a template. The reaction solution for PCR was prepared by mixing 0.6 µl of PfuTurbo DNA polymerase (Stratagene, Inc.), 3 µl of 10x PCR buffer bundled, 2.4 µl of 2.5 mM dNTP mixture, 1.5 µl each of 10 µM primer RBv8-WF2 and primer RBv8-WR2, 3 µl of template DNA and 18 µl of distilled water. After the early denaturation at 95°C for 1 minute, the reaction conditions were set to repeat 35 cycles of the reaction at 95°C for 30 seconds, 55°C for 30 seconds and 72°C for 1 minute, followed by final extension at 72°C for 5 minutes.

Using Zero Blunt TOPO PCR Cloning Kit (Invitrogen, Inc.), the DNA fragment obtained was cloned in accordance with the method described in the manual attached. The base sequence of the cloned DNA was decoded using ABI377DNA sequencer and found to have the base sequence of 356 bp represented by SEQ ID NO: 36. The plasmid having the base sequence represented by SEQ ID NO: 36 was named pRBv. Escherichia coli was transformed by plasmid pRBv, which transformant was named Escherichia coli TOP10/pRBv.

As a result of analysis of the base sequence of this DNA fragment, it was revealed that the DNA fragment represented by SEQ ID NO: 36 contained the DNA (SEQ ID NO: 38) encoding rat type Bv8 precursor peptide (107 amino acid residues) represented by SEQ ID NO: 37.

Also, the base sequence represented by SEQ ID NO: 38 contained a typical signal sequence, and it was revealed that the DNA having the base sequence represented by SEQ ID NO: 38 contained the DNA (SEQ ID NO: 40) composed of 234 base pairs, encoding rat type Bv8 mature peptide (81 amino acid residues) represented by SEQ ID NO: 39.

EXAMPLE 6

Cloning of cDNA encoding rat brain cDNA-derived, novel G protein-coupled receptor protein (rZAQ1) and its base sequencing

Using rat whole brain cDNA library (CLONTECH, Inc.) as a template, PCR was carried out by using two primers (SEQ ID NO: 41 and SEQ ID NO: 42). In the reaction, the composition of the reaction solution was that 1/10 volume of the aforesaid cDNA was used as a template, and 1/50 volume of Advantage-2 cDNA polymerase Mix (CLON-

TECH, Inc.), 0.2 μM each of the primers and 200 μM of dNTPs were added thereto, and the buffer bundled to the enzyme was added to make the solution volume 25 μl. The PCR was carried out, (i) after maintaining at 94°C for 2 minutes, by repeating (ii) 3 cycles of 94°C for 20 seconds and 72°C for 1 minute and 30 seconds, (iii) 3 cycles of 94°C for 20 seconds and 68°C for 1 minute and 30 seconds, (iv) 36 cycles of 94°C for 20 seconds, 62°C for 20 seconds and 68°C for 1 minute, followed by final extension at 68°C for 7 minutes. After the PCR, the reaction product was subcloned to plasmid vector pCR2.1-TOPO (Invitrogen, Inc.) following the instructions of TOPO-TA Cloning Kit (Invitrogen, Inc.). The subcloned product was transfected to Escherichia coli DH5α and clones having the cDNA were selected in ampicillin-containing LB agar medium. As a result of analysis on the sequences of the respective clones, the cDNA (SEQ ID NO: 43) encoding novel G protein-coupled receptor protein was acquired. In the amino acid sequence (SEQ ID NO: 44) deduced from the base sequence of cDNA, 83.7% homology to the amino acid sequence represented by SEQ ID NO: 1 was observed. Novel G protein-coupled receptor protein containing the amino acid sequence was named rZAQ1. Also, the transformant (Escherichia coli) containing a DNA having the base sequence represented by SEQ ID NO: 43 was named Escherichia coli DH5α/pCR2.1-rZAQ1.

## EXAMPLE 7

### Cloning of cDNA encoding rat brain cDNA-derived, novel G protein-coupled receptor protein (rZAQ2) and its base sequencing

The clone encoding rZAQ2 was acquired by the gene trapper method. That is, after Probes 1 and 2 (SEQ ID NO: 45 and SEQ ID NO: 46) were biotinylated, the probes were hybridized to rat whole brain cDNA library (GIBCO-BRL, Inc.), which was rendered single-stranded. Using Primers 1 and 2 (SEQ ID NO: 47 and SEQ ID NO: 48), the single-stranded gene obtained was annealed to double strands. This gene was electroporated to Escherichia coli DH10B and transformants were obtained using ampicillin resistance as an indicator. In addition, the clone encoding the targeting base sequence was selected by colony PCR using Probe 1 (SEQ ID NO: 45) and Primer 3 (SEQ ID NO: 49). The amino acid sequence (SEQ ID NO: 51) deduced from the base sequence (SEQ ID NO: 50) of ORF (open reading frame), which was predicted from the base sequence of this clone, showed homology of 80.6% to rZAQ 1. Novel G protein-coupled receptor protein having the amino acid sequence was named rZAQ2. Also, the transformant (Escherichia coli) acquired by the gene trapper method was named Escherichia coli DH10B/pCMV-rZAQ2.

## EXAMPLE 8

### Experiment on contraction using specimen removed from guinea pig ileum

Guinea pigs (std: Hartley, 7-8 weeks old, male, weighing about 450 g) weres bled to death by cutting the carotid artery and laparotomized to remove the ileum. The ileum was placed on a glass Petri dish charged with Tyrode solution (composition: 138 mM NaCl, 2.7 mM KCl, 1.8 mM $CaCl_2$, 0.5 mM $MgCl_2$, 1.1 mM $NaH_2PO_4$, 11.9 mM $NaHCO_3$ and 5.6 mM glucose), which was bubbled with 95% $O_2$-5% $CO_2$ gas. After connective tissues or fat pieces adhered to the ileum were removed with scissors for surgery and tweezers, the ileum was cut into a size of about 1.5 cm long, which was used as specimen. The specimen was suspended in an organ bath (20 ml) filled with the Tyrode solution maintained at 37°C and a load of 0.5 g was applied over 30 minutes or longer to stabilize the specimen. Contraction response of the ileum specimen was measured using AMPLIFIER CASE 7747 of NEC Sanei. After determination of the maximum contraction by administration of 1 μM acetylcholine, purified human type Bv8 peptide obtained by the method of REFERENCE EXAMPLE 1 with a modification or snake venom MIT1 was accumulatively administered to assay the contraction response. For dilution of human type Bv8 and MIT1, physiological saline containing 0.05% bovine serum albumin was employed.

The results are shown in FIG. 11.

The contraction response induced by human type Bv8 and MIT1 was shown by percentage, taking the contraction induced by 1 μM acetylcholine as 100%. The $EC_{50}$ values of human type Bv8 and MIT1 calculated from the dose-response curve were 0.57 nM and 0.40 nM, respectively.

REFERENCE EXAMPLE 1

Production of human type Bv8 peptide (SEQ ID NO: 19) in Escherichia coli

(1) Construction of human type Bv8 peptide expression plasmid in Escherichia coli

(a) Preparation of structural gene of human type Bv8

Using 6 DNA fragments #1 through #6 (SEQ ID NO: 65 to SEQ ID NO: 70), the structural gene of human type Bv8 was prepared.

(b) Phosphorylation of DNA oligomers

Each of four DNA oligomers (#2 to #5), excluding the above-described # 1 and #6, which should form the 5' end, was reacted at 37°C for an hour in 25 μl of a phosphorylation reaction solution [10 μg of DNA oligomer, 50mM Tris-HCl, pH7.6, 10mM $MgCl_2$, 1mM sperimidine, 10 mM dithiothreitol (hereinafter abbreviated as DTT), 0.1 mg/ml bovine serum albumin (hereinafter abbreviated as BSA), 1 mM ATP, 10 units of T4 polynucleotide kinase (TaKaRa Shuzo Co., Ltd.)], whereby the 5' end of each oligomer was phosphorylated. After treatment with phenol, 2-fold volume of ethanol was added. The mixture was cooled to -70°C and centrifuged to precipitate DNA.

(c) Ligation of DNA fragments

The DNA fragments obtained in (b) above were combined with #1 and #6 described above to make the volume 120 μl. After maintaining at 90°C for 10 minutes, the mixture was gradually chilled to room temperature for annealing. Thereafter, ligation was carried out using TaKaRa DNA Ligation Kit ver.2 (TaKaRa Shuzo Co., Ltd.). To 30 μl of the annealing solution, 30 μl of Solution II bundled to the kit was added followed by thoroughly mixing them. Then, 60 μl of Solution I bundled to the kit was added to the mixture. The mixture was reacted at 37°C for an hour for ligation followed by phenol treatment. The aqueous phase was recovered and 2-fold volume of ethanol was added thereto. After cooling to -70°C, the mixture was centrifuged to precipitate the DNA. The DNA fragment thus obtained was phosphorylated using T4 polynucleotide kinase (TaKaRa Shuzo Co., Ltd.). The product was provided for the subsequent step (d).

(d) Construction of human type Bv8 expression vector

For expression vector, after digestion of pTCII (described in JPA 2000-178297) with NdeI and BamHI (TaKaRa Shuzo Co., Ltd.) at 37°C for 2 hours, electrophoresis was performed on 1% agarose gel, the DNA fragment of 4.3 kb was extracted using QIAquick Gel Extraction Kit (Qiagen, Inc.), and the extract was dissolved in 25 μl of TE buffer. The fragment of pTCII with NdeI and BamHI was ligated to the structural gene (SEQ ID NO:71) of human type Bv8 prepared above, using TaKaRa DNA ligation kit ver.2 (TaKaRa Shuzo Co., Ltd.). Using 10 μl of the reaction solution, Escherichia coli JM109 competent cells (Toyobo) were transformed. The transformants were inoculated on LB agar medium containing 10 μg/ml of tetracycline followed by incubation at 37°C overnight. The resulting tetracycline resistant colony was selected. The transformant was cultured overnight in LB medium, and plasmid pTCh2ZAQ was prepared using QIAprep8 Miniprep Kit (Qiagen, Inc.). The base sequence of this human type Bv8 DNA was identified using Model 377 DNA Sequencer of Applied Biosystems, Inc. The plasmid pTCh2ZAQ was transfected to Escherichia coli MM294 (DE3) to acquire human type Bv8 expression strain, Escherichia coli MM294 (DE3)/pTCh2ZAQ.

(2) Preparation of human type Bv8 peptide

Escherichia coli MM294 (DE3)/pTCh2ZAQ described above was shake-cultured at 37°C for 8 hours in an 2 L volume flask charged with 1 L of LB medium (1% peptone, 0.5% yeast extract and 0.5% sodium chloride) containing 5.0 mg/L of tetracycline. The culture medium obtained was transferred to a 50 L fermentation tank charged with 19 L of main fermentation medium (1.68% sodium monohydrogenphosphate, 0.3% sodium dihydrogenphosphate, 0.1% ammonium chloride, 0.05% sodium chloride, 0.05% magnesium sulfate, 0.02% defoaming agent, 0.00025% ferrous sulfate, 0.0005% thiamine hydrochloride, 1.5% glucose, 1.5% Hicase amino) to initiate aerated agitation at 30°C. When the turbidity of the culture medium became 500 Klett units, isopropyl-β-D-thiogalactopyranoside was added thereto in a final concentration of 12 mg/L, followed by incubation for further 4 hours. After the incubation was completed, the culture medium was centrifuged to acquire about 500 g of wet cells. The cells were stored at -80°C.

(3) Activation of human type Bv8 peptide

After 200 mM Tris/HCl and 800 ml of 7 M guanidine hydrochloride (pH8.0) were added to 400 g of the cells obtained in (2) above to lyse the cells, centrifugation was performed (10000 rpm, 1 hour). To the supernatant, 20 liters of 0.4 M arginine, 50mM Tris/HCl, 0.2mM GSSG (oxidized glutathione), 1mM GSH (reduced glutathione) (pH8.0) were added and the mixture was maintained at 4°C overnight for activation.

(4) Purification of human type Bv8 peptide

The pH of regeneration buffer, which completed activation in (3) described above, was adjusted to 6.0 and adsorbed onto SP-Sepharose column (11.3cm x 15cm) equilibrated with 50 mM phosphate buffer (pH 6.0), followed by elution with 600 mM NaCl/50 mM phosphate buffer (pH6.0). The fraction containing human type Bv8 was pooled. This fraction was passed through SP-5PW (21.5 mm x 150 mmL) equilibrated with 50 mM phosphate buffer for adsorption. After washing, elution was performed with a gradual gradient (60 mins.) from 0 to 100% B (B = 50mM phosphate buffer + 1 M NaCl, pH 6.05) to obtain human type Bv8 fraction (elution time; about 40 mins.). This fraction was further passed through and adsorbed onto C4P-50 (21.5 mm ID x 300 mmL, Showa Denko K. K.) equilibrated with 0.1% trifluoroacetic acid. After washing, elution was performed with a gradual gradient (60 mins.) from 25 to 50% B (B: 80% acetonitrile/0.1% trifluoroacetic acid). The eluted human type Bv8 fraction (elution time; about 30 mins.) was pooled and lyophilized to obtain about 25 mg of lyophilized human type Bv8 powders.

(5) Characterization of human type Bv8 peptide

(a) Analysis using SDS polyacrylamide gel electrophoresis

The human type Bv8 obtained in (4) described above was suspended in a sample buffer [Laemmli, Nature, 227, 680 (1979)] supplemented with 100 mM DTT. After heating at 95°C for 1 minute, electrophoresis was performed on Multigel 15/25 (Daiichi Kagaku Yakuhin). After the electrophoresis, the gel was stained with Coomassie brilliant blue. As a result, a single protein band was observed at the position of 9 kDa. From the result, Escherichia coli-derived recombinant human type Bv8 preparation obtained in (4) described above was found to be of extremely high purity.

(b) Analysis of amino acid composition

The amino acid composition was determined using an amino acid analyzer (Hitachi L-8500A Amino Acid Analyzer). As a result, the amino acid composition coincided with the amino acid composition deduced from the base sequence of the DNA of human type Bv8 (peptide comprising the amino acid sequence represented by SEQ ID NO: 19) (TABLE 2).

TABLE 2

| Amino acid | Number of residue/mol | Value predicted from the base sequence of human type Bv8 |
|---|---|---|
| Asx | 4.0 | 4 |
| Thr[1)] | 4.6 | 5 |
| Ser[1)] | 4.3 | 5 |
| Glx | 2.3 | 2 |
| Pro | 5.2 | 5 |
| Gly | 7.8 | 8 |
| Ala | 5.0 | 5 |
| Cys[2)] | N.D. | 10 |
| Val | 3.8 | 4 |
| Met | 2.8 | 3 |
| Ile | 4.2 | 5 |
| Leu | 6 | 6 |
| Tyr | 0 | 0 |
| Phe | 3.7 | 4 |

1) Value obtained when extrapolated to 0 hour

2) Not detected

TABLE 2 (continued)

| Amino acid | Number of residue/mol | Value predicted from the base sequence of human type Bv8 |
|---|---|---|
| His | 2.9 | 3 |
| Lys | 4.9 | 5 |
| Arg | 5.8 | 6 |
| Trp | 0.9. | 1 |
| Acid hydrolysis (6N HCl-4% thioglycolic acid, mean value when hydrolyzed at 110°C for 24 and 48 hours) | | |

(c) N-Terminal amino acid sequencing

The N-terminal amino acid sequence was determined using a gas-phase protein sequencer (PE-Applied Biosystems, Model 492). The results indicate that it coincided with the N-terminal amino acid sequence of human type Bv8 deduced from the base sequence of the DNA of human type Bv8 obtained (TABLE 3).

TABLE 3

| Residue No. | PTH-Amino acid[1)] detected (pmol) | Amino acid deduced from the base sequence of human type Bv8 |
|---|---|---|
| 1 | Ala (103) | Ala |
| 2 | Val (99) | Val |
| 3 | Ile (88) | Ile |
| 4 | Thr (54) | Thr |
| 5 | Gly (66) | Gly |
| 6 | Ala (79) | Ala |
| 7 | N.D. | Cys |
| 8 | Asp (47) | Asp |
| 9 | Lys (62) | Lys |
| 10 | Asp (50) | Asp |
| 11 | Ser (36) | Ser |
| 12 | Gln (52) | Gln |
| 13 | N.D. | Cys |
| 14 | Gly (44) | Gly |
| 15 | Gly (55) | Gly |
| 16 | Gly (56) | Gly |
| 17 | Met (50) | Met |
| 18 | N.D. | Cys |
| 19 | N.D. | Cys |
| 20 | Ala (33) | Ala |

1) Detected as PTH (phenylthiohydantoin) derivative. Analysis was performed using 150 pmol of human type Bv8.
N.D. denotes that it was not detected.

(d) C-Terminal amino acid sequencing

The C-terminal amino acid sequence was determined using an amino acid analyzer (Hitachi L-8500A, Amino Acid Analyzer). The results indicate that it coincided with the C-terminal amino acid sequence of human type Bv8 deduced from the base sequence of the DNA of human type Bv8 obtained (TABLE 4).

TABLE 4

| C-Terminal amino acid | Recovery rate (%) |
|---|---|
| Lys | 18.9 |

Gaseous phase hydrazine degradation method (100°C, 3.5 hours)

(6) Assay for the activity of human type Bv8 peptide (assay for the intracellular Ca ion level increasing activity using FLIPR)

The purified Escherichia coli-derived recombinant human type Bv8 preparation, which was obtained in (4) described above, was assayed for the activity (assay for the intracellular Ca ion level increasing activity using FLIPR), in accordance with the method of EXAMPLE 2 (2-3). As a result, it had the activity equivalent to that of the CHO cell-derived recombinant preparation (purified human type Bv8: EXAMPLE 3).

INDUSTRIAL APPLICABILITY

The peptide of the invention, the DNA encoding the peptide of the invention (hereinafter sometimes referred to as the DNA of the invention) and the antibody to the peptide of the invention (hereinafter sometimes referred to as the antibody of the invention) are useful (i) as agents for the treatment/prevention of various diseases with which the peptide of the invention is associated; (ii) for screening a compound or its salt that alters the binding property between the peptide of the invention and the protein of the invention; (iii) for quantification of the peptide of the invention or a salt thereof; (iv) as a gene diagnostic agent; (v) as a pharmaceutical comprising the antisense DNA; (vi) as a pharmaceutical and diagnostic agent comprising the antibody of the invention; (vii) for preparation of non-human animals bearing the DNA of the invention; (viii) for drug design based on comparison with structurally similar ligand receptors; etc. In particular, the compound or its salt obtained by screening using the peptide of the invention and the protein of the invention is safely used as pharmaceuticals for digestive diseases, central nervous diseases, etc.

## Claims

1. A method of screening a compound or its salt that alters the binding property between a peptide or its salt containing the same or substantially the same amino acid sequence as the amino acid sequence represented by SEQ ID NO: 19 or SEQ ID NO: 39, and a protein or its salt containing the same or substantially the same amino acid sequence as the amino acid sequence represented by SEQ ID NO: 1, SEQ ID NO: 44, SEQ ID NO: 51, SEQ ID NO: 52, SEQ ID NO: 53 or SEQ ID NO: 54, which comprises using a peptide, its partial peptide or a salt thereof, containing the same or substantially the same amino acid sequence as the amino acid sequence represented by SEQ ID NO: 19 or SEQ ID NO: 39, and a protein, its partial protein or a salt thereof, containing the same or substantially the same amino acid sequence as the amino acid sequence represented by SEQ ID NO: 1, SEQ ID NO: 44, SEQ ID NO: 51, SEQ ID NO: 52, SEQ ID NO: 53 or SEQ ID NO: 54.

2. A kit for screening a compound or its salt that alters the binding property between a peptide or its salt containing the same or substantially the same amino acid sequence as the amino acid sequence represented by SEQ ID NO: 19 or SEQ ID NO: 39, and a protein or its salt containing the same or substantially the same amino acid sequence as the amino acid sequence represented by SEQ ID NO: 1, SEQ ID NO: 44, SEQ ID NO: 51, SEQ ID NO: 52, SEQ ID NO: 53 or SEQ ID NO: 54, comprising a peptide, its partial peptide or a salt thereof, containing the same or substantially the same amino acid sequence as the amino acid sequence represented by SEQ ID NO: 19 or SEQ ID NO: 39, and a protein, its partial protein or a salt thereof, containing the same or substantially the same amino acid sequence as the amino acid sequence represented by SEQ ID NO: 1, SEQ ID NO: 44, SEQ ID NO: 51, SEQ ID NO: 52, SEQ ID NO: 53 or SEQ ID NO: 54.

3. A compound or its salt that alters the binding property between a peptide or its salt containing the same or substantially the same amino acid sequence as the amino acid sequence represented by SEQ ID NO: 19 or SEQ ID NO: 39, and a protein or its salt containing the same or substantially the same amino acid sequence as the amino acid sequence represented by SEQ ID NO: 1, SEQ ID NO: 44, SEQ ID NO: 51, SEQ ID NO: 52, SEQ ID NO: 53 or SEQ ID NO: 54, which is obtainable using the screening method according to claim 1 or using the screening kit according to claim 2.

4. A pharmaceutical comprising the compound or its salt according to claim 3.

5. The pharmaceutical according to claim 4, which is an agent for the prevention/treatment of a digestive disease.

6. A peptide containing the same or substantially the same amino acid sequence as the amino acid sequence represented by SEQ ID NO: 37, or a salt thereof.

7. A DNA encoding the peptide according to claim 6.

8. The DNA according to claim 7 containing the base sequence represented by SEQ ID NO: 38.

9. A DNA containing the base sequence represented by SEQ ID NO: 40.

10. A recombinant vector containing the DNA according to claim 8.

11. A transformant transformed by the recombinant vector according to claim 10.

12. A method of manufacturing the peptide or its salt according to claim 6, which comprises culturing the transformant of claim 11 and producing/accumulating the peptide according to claim 6.

13. An antibody to a peptide or its salt containing the same or substantially the same amino acid sequence as the amino acid sequence represented by SEQ ID NO: 39.

14. A diagnostic comprising the antibody according to claim 13.

15. The diagnostic according to claim 14, which is a diagnostic for digestive diseases.

16. A non-human mammal containing the DNA according to claim 7, or its variant DNA, which is exogenous.

**17.** The mammal according to claim 16, wherein the non-human mammal is a rodent.

**18.** The mammal according to claim 17, wherein the rodent is rat.

**19.** A recombinant vector bearing the DNA according to claim 9, or its variant DNA, which is exogenous and capable of expressing in a non-human mammal.

**20.** A non-human mammalian embryonic stem cell, in which the DNA according to claim 7 or 9 is inactivated.

**21.** The embryonic stem cell according to claim 20, wherein the DNA is inactivated by introducing a reporter gene therein.

**22.** The embryonic stem cell according to claim 21, wherein the non-human mammal is a rodent.

**23.** A non-human mammal deficient in expressing the DNA according to claim 7 or 9, wherein the said DNA is inactivated.

**24.** The non-human mammal according to claim 23, wherein the DNA is inactivated by inserting a reporter gene therein and the reporter gene is capable of expressing under control of a promoter to the DNA of the invention.

**25.** The non-human mammal according to claim 23, wherein the non-human mammal is a rodent.

**26.** A method of screening a compound or its salt that promotes or inhibits the promoter activity to the DNA according to claim 7 or 9, which comprises administering a test compound to the mammal according to claim 24 and detecting expression of the reporter gene.

**27.** A method of preventing /treating digestive diseases, which comprises administering to a mammal an effective dose of the compound or its salt according to claim 3.

**28.** Use of the compound or its salt according to claim 3, for manufacturing a preventive/therapeutic agent of digestive diseases.

Fig.1

```
        10        20        30        40        50        60
ATGGAGACCACCATGGGGTTCATGGATGACAATGCCACCAACACTTCCACCAGCTTCCTT
M  E  T  T  M  G  F  M  D  D  N  A  T  N  T  S  T  S  F  L

        70        80        90       100       110       120
TCTGTGCTCAACCCTCATGGAGCCCATGCCACTTCCTTCCCATTCAACTTCAGCTACAGC
S  V  L  N  P  H  G  A  H  A  T  S  F  P  F  N  F  S  Y  S

       130       140       150       160       170       180
GACTATGATATGCCTTTGGATGAAGATGAGGATGTGACCAATTCCAGGACGTTCTTTGCT
D  Y  D  M  P  L  D  E  D  E  D  V  T  N  S  R  T  F  F  A

       190       200       210       220       230       240
GCCAAGATTGTCATTGGGATGGCCCTGGTGGGCATCATGCTGGTCTGCGGCATTGGAAAC
A  K  I  V  I  G  M  A  L  V  G  I  M  L  V  C  G  I  G  N

       250       260       270       280       290       300
TTCATCTTTATCGCTGCCCTGGTCCGCTACAAGAAACTGCGCAACCTCACCAACCTGCTC
F  I  F  I  A  A  L  V  R  Y  K  K  L  R  N  L  T  N  L  L

       310       320       330       340       350       360
ATCGCCAACCTGGCCATCTCTGACTTCCTGGTGGCCATTGTCTGCTGCCCCTTTGAGATG
I  A  N  L  A  I  S  D  F  L  V  A  I  V  C  C  P  F  E  M

       370       380       390       400       410       420
GACTACTATGTGGTGCGCCAGCTCTCCTGGGAGCACGGCCACGTCCTGTGCACCTCTGTC
D  Y  Y  V  V  R  Q  L  S  W  E  H  G  H  V  L  C  T  S  V
```

Fig.2

```
        430       440       450       460       470       480
AACTACCTGCGCACTGTCTCTCTCTATGTCTCCACCAATGCCCTGCTGGCCATCGCCATT
N  Y  L  R  T  V  S  L  Y  V  S  T  N  A  L  L  A  I  A  I

        490       500       510       520       530       540
GACAGGTATCTGGCTATTGTCCATCCGCTGAGACCACGGATGAAGTGCCAAACAGCCACT
D  R  Y  L  A  I  V  H  P  L  R  P  R  M  K  C  Q  T  A  T

        550       560       570       580       590       600
GGCCTGATTGCCTTGGTGTGGACGGTGTCCATCCTGATCGCCATCCCTTCCGCCTACTTC
G  L  I  A  L  V  W  T  V  S  I  L  I  A  I  P  S  A  Y  F

        610       620       630       640       650       660
ACCACCGAGACGGTCCTCGTCATTGTCAAGAGCCAGGAAAAGATCTTCTGCGGCCAGATC
T  T  E  T  V  L  V  I  V  K  S  Q  E  K  I  F  C  G  Q  I

        670       680       690       700       710       720
TGGCCTGTGGACCAGCAGCTCTACTACAAGTCCTACTTCCTCTTTATCTTTGGCATAGAA
W  P  V  D  Q  Q  L  Y  Y  K  S  Y  F  L  F  I  F  G  I  E

        730       740       750       760       770       780
TTCGTGGGCCCCGTGGTCACCATGACCCTGTGCTATGCCAGGATCTCCCGGGAGCTCTGG
F  V  G  P  V  V  T  M  T  L  C  Y  A  R  I  S  R  E  L  W

        790       800       810       820       830       840
TTCAAGGCGGTCCCTGGATTCCAGACAGAGCAGATCCGCAAGAGGCTGCGCTGCCGCAGG
F  K  A  V  P  G  F  Q  T  E  Q  I  R  K  R  L  R  C  R  R

        850       860       870       880       890       900
AAGACGGTCCTGGTGCTCATGTGCATCCTCACCGCCTACGTGCTATGCTGGGCGCCCTTC
K  T  V  L  V  L  M  C  I  L  T  A  Y  V  L  C  W  A  P  F
```

Fig.3

```
        910       920       930       940       950       960
TACGGCTTCACCATCGTGCGCGACTTCTTCCCCACCGTGTTCGTGAAGGAGAAGCACTAC
Y  G  F  T  I  V  R  D  F  F  P  T  V  F  V  K  E  K  H  Y

        970       980       990      1000      1010      1020
CTCACTGCCTTCTACATCGTCGAGTGCATCGCCATGAGCAACAGCATGATCAACACTCTG
L  T  A  F  Y  I  V  E  C  I  A  M  S  N  S  M  I  N  T  L

       1030      1040      1050      1060      1070      1080
TGCTTCGTGACCGTCAAGAACGACACCGTCAAGTACTTCAAAAAGATCATGTTGCTCCAC
C  F  V  T  V  K  N  D  T  V  K  Y  F  K  K  I  M  L  L  H

       1090      1100      1110      1120      1130      1140
TGGAAGGCTTCTTACAATGGCGGTAAGTCCAGTGCAGACCTGGACCTCAAGACAATTGGG
W  K  A  S  Y  N  G  G  K  S  S  A  D  L  D  L  K  T  I  G

       1150      1160      1170      1180      1190
ATGCCTGCCACCGAAGAGGTGGACTGCATCAGACTAAAATAA
M  P  A  T  E  E  V  D  C  I  R  L  K  *
```

Fig.4

```
         10        20        30        40        50        60
ATGGAGACCACCATGGGGTTCATGGATGACAATGCCACCAACACTTCCACCAGCTTCCTT
M  E  T  T  M  G  F  M  D  D  N  A  T  N  T  S  T  S  F  L

         70        80        90       100       110       120
TCTGTGCTCAACCCTCATGGAGCCCATGCCACTTCCTTCCCATTCAACTTCAGCTACAGC
S  V  L  N  P  H  G  A  H  A  T  S  F  P  F  N  F  S  Y  S

        130       140       150       160       170       180
GACTATGATATGCCTTTGGATGAAGATGAGGATGTGACCAATTCCAGGACGTTCTTTGCT
D  Y  D  M  P  L  D  E  D  E  D  V  T  N  S  R  T  F  F  A

        190       200       210       220       230       240
GCCAAGATTGTCATTGGGATGGCCCTGGTGGGCATCATGCTGGTCTGCGGCATTGGAAAC
A  K  I  V  I  G  M  A  L  V  G  I  M  L  V  C  G  I  G  N

        250       260       270       280       290       300
TTCATCTTTATCGCTGCCCTGGTCCGCTACAAGAAACTGCGCAACCTCACCAACCTGCTC
F  I  F  I  A  A  L  V  R  Y  K  K  L  R  N  L  T  N  L  L

        310       320       330       340       350       360
ATCGCCAACCTGGCCATCTCTGACTTCCTGGTGGCCATTGTCTGCTGCCCCTTTGAGATG
I  A  N  L  A  I  S  D  F  L  V  A  I  V  C  C  P  F  E  M

        370       380       390       400       410       420
GACTACTATGTGGTGCGCCAGCTCTCCTGGGAGCACGGCCACGTCCTGTGCACCTCTGTC
D  Y  Y  V  V  R  Q  L  S  W  E  H  G  H  V  L  C  T  S  V
```

Fig.5

```
        430       440       450       460       470       480
AACTACCTGCGCACTGTCTCTCTCTATGTCTCCACCAATGCCCTGCTGGCCATCGCCATT
N  Y  L  R  T  V  S  L  Y  V  S  T  N  A  L  L  A  I  A  I

        490       500       510       520       530       540
GACAGGTATCTGGCTATTGTCCATCCGCTGAGACCACGGATGAAGTGCCAAACAGCCACT
D  R  Y  L  A  I  V  H  P  L  R  P  R  M  K  C  Q  T  A  T

        550       560       570       580       590       600
GGCCTGATTGCCTTGGTGTGGACGGTGTCCATCCTGATCGCCATCCCTTCCGCCTACTTC
G  L  I  A  L  V  W  T  V  S  I  L  I  A  I  P  S  A  Y  F

        610       620       630       640       650       660
ACCACCGAGACGGTCCTCGTCATTGTCAAGAGCCAGGAAAAGATCTTCTGCGGCCAGATC
T  T  E  T  V  L  V  I  V  K  S  Q  E  K  I  F  C  G  Q  I

        670       680       690       700       710       720
TGGCCTGTGGACCAGCAGCTCTACTACAAGTCCTACTTCCTCTTTATCTTTGGCATAGAA
W  P  V  D  Q  Q  L  Y  Y  K  S  Y  F  L  F  I  F  G  I  E

        730       740       750       760       770       780
TTCGTGGGCCCCGTGGTCACCATGACCCTGTGCTATGCCAGGATCTCCCGGGAGCTCTGG
F  V  G  P  V  V  T  M  T  L  C  Y  A  R  I  S  R  E  L  W

        790       800       810       820       830       840
TTCAAGGCGGTCCCTGGATTCCAGACAGAGCAGATCCGCAAGAGGCTGCGCTGCCGCAGG
F  K  A  V  P  G  F  Q  T  E  Q  I  R  K  R  L  R  C  R  R

        850       860       870       880       890       900
AAGACGGTCCTGGTGCTCATGTGCATCCTCACCGCCTACGTGCTATGCTGGGCGCCCTTC
K  T  V  L  V  L  M  C  I  L  T  A  Y  V  L  C  W  A  P  F
```

Fig.6

```
        910       920       930       940       950       960
TACGGCTTCACCATCGTGCGCGACTTCTTCCCCACCGTGTTTGTGAAGGAGAAGCACTAC
Y  G  F  T  I  V  R  D  F  F  P  T  V  F  V  K  E  K  H  Y

        970       980       990      1000      1010      1020
CTCACTGCCTTCTACATCGTCGAGTGCATCGCCATGAGCAACAGCATGATCAACACTCTG
L  T  A  F  Y  I  V  E  C  I  A  M  S  N  S  M  I  N  T  L

       1030      1040      1050      1060      1070      1080
TGCTTCGTGACCGTCAAGAACGACACCGTCAAGTACTTCAAAAAGATCATGTTGCTCCAC
C  F  V  T  V  K  N  D  T  V  K  Y  F  K  K  I  M  L  L  H

       1090      1100      1110      1120      1130      1140
TGGAAGGCTTCTTACAATGGCGGTAAGTCCAGTGCAGACCTGGACCTCAAGACAATTGGG
W  K  A  S  Y  N  G  G  K  S  S  A  D  L  D  L  K  T  I  G

       1150      1160      1170      1180      1190
ATGCCTGCCACCGAAGAGGTGGACTGCATCAGACTAAAATAA
M  P  A  T  E  E  V  D  C  I  R  L  K  *
```

Fig.7

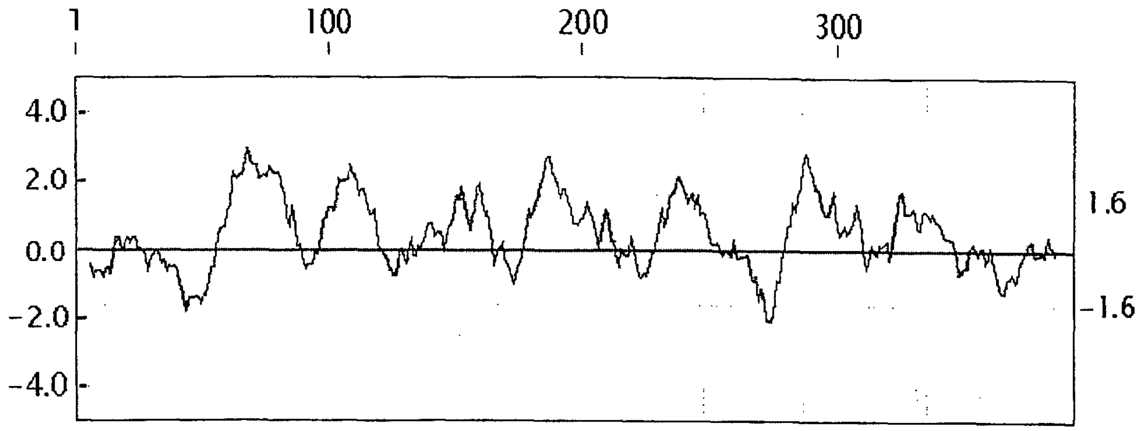

Fig.8

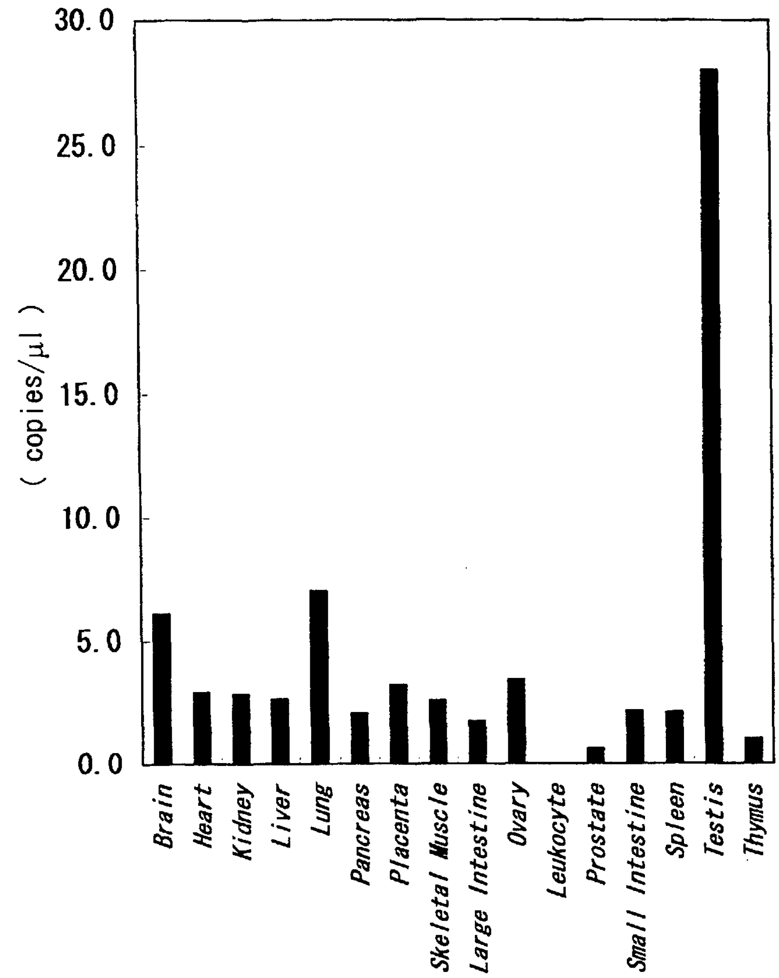
30.0
25.0
20.0
15.0
10.0
5.0
0.0
( copies/μl )
Brain
Heart
Kidney
Liver
Lung
Pancreas
Placenta
Skeletal Muscle
Large Intestine
Ovary
Leukocyte
Prostate
Small Intestine
Spleen
Testis
Thymus

Fig.9

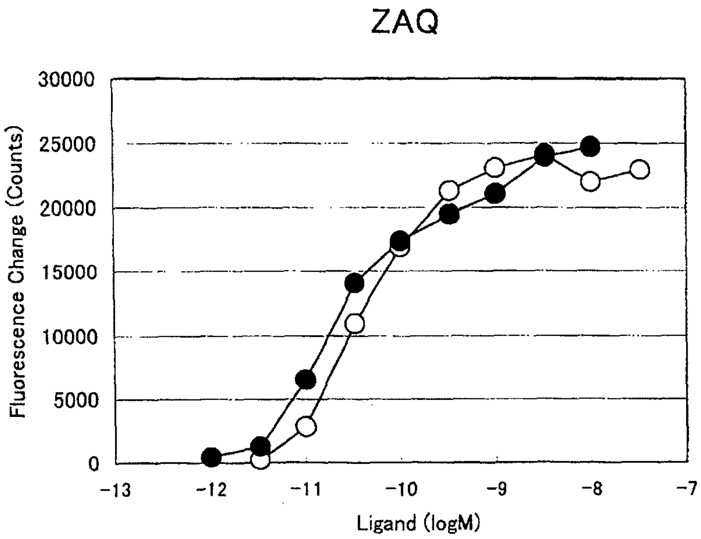
ZAQ
30000
25000
20000
15000
10000
5000
0
Fluorescence Change (Counts)
-13
-12
-11
-10
-9
-8
-7
Ligand (logM)

Fig.10

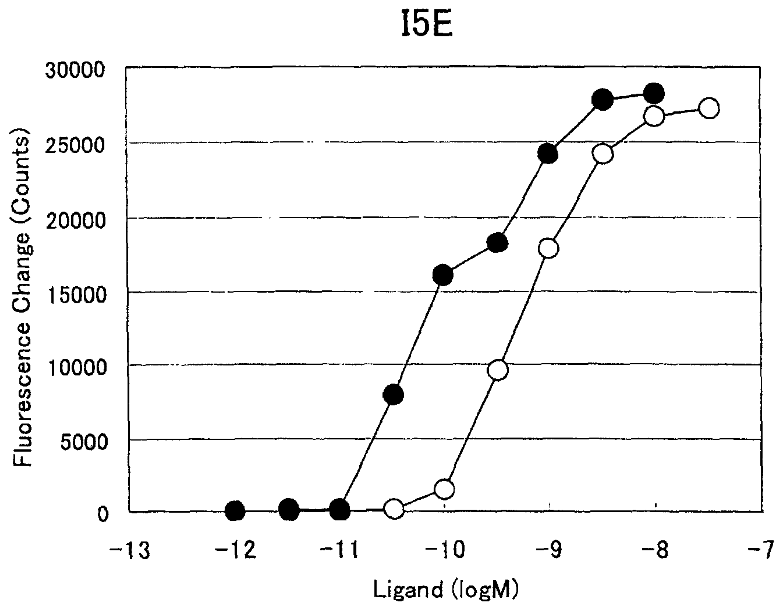
I5E
30000
25000
20000
15000
10000
5000
0
Fluorescence Change (Counts)
-13
-12
-11
-10
-9
-8
-7
Ligand (logM)

Fig.11

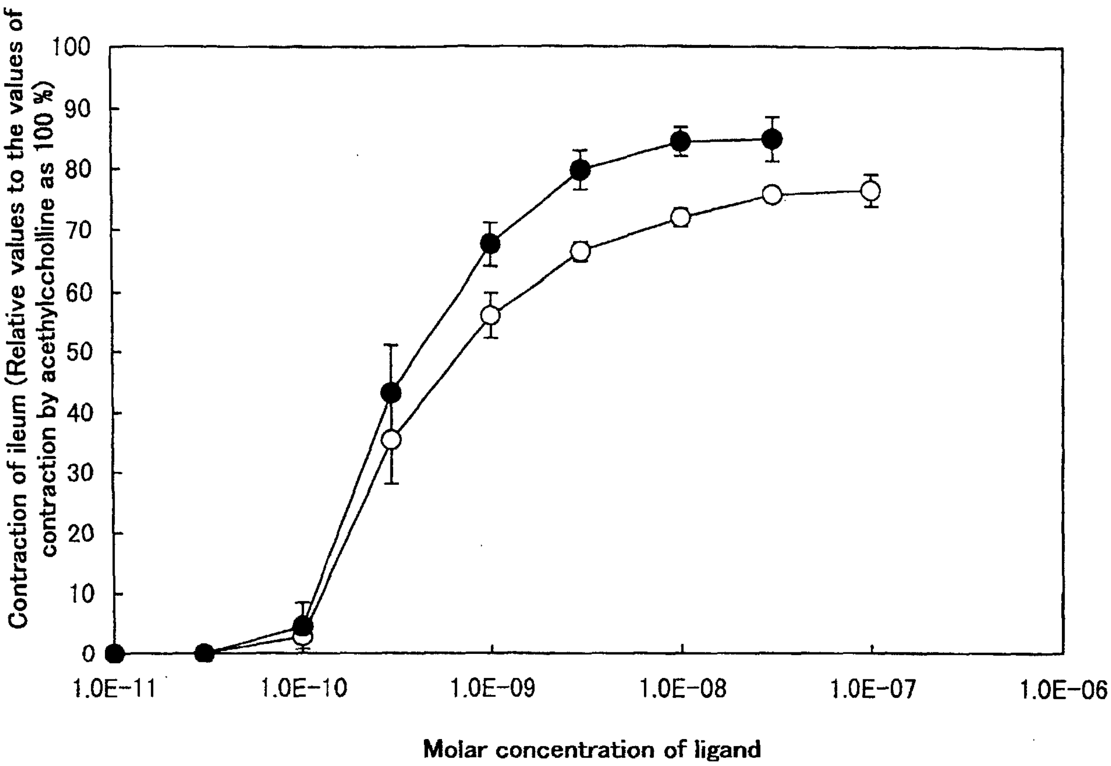
Contraction of ileum (Relative values to the values of contraction by acethylcholline as 100 %)
100
90
80
70
60
50
40
30
20
10
0
1.0E-11
1.0E-10
1.0E-09
1.0E-08
1.0E-07
1.0E-06
Molar concentration of ligand